# EUROPEAN PATENT SPECIFICATION

(11) **EP 1 064 372 B1**
(45) Date of publication and mention of the grant of the patent: **15.11.2006**
(21) Application number: 99912607.1
(22) Date of filing: 17.03.1999
(51) Int. Cl.: C12N 15/12, C07K 14/47, C07K 16/18, G01N 33/574, A61K 35/14, A61K 38/17, A61K 39/395

(54) **COMPOUNDS AND METHODS FOR THERAPY AND DIAGNOSIS OF LUNG CANCER**
VERBINDUNGEN UND VERFAHREN FÜR THERAPIE UND DIAGNOSE VON LUNGENKREBS
COMPOSES ET METHODES THERAPEUTIQUES ET DIAGNOSTIQUES DU CANCER DU POUMON

(30) Priority: 18.03.1998 US 40802; 18.03.1998 US 40984; 27.07.1998 US 123912; 27.07.1998 US 123933
(43) Date of publication of application: 03.01.2001
(73) Proprietor: CORIXA CORPORATION, Seattle, WA 98104 (US)
(72) Inventor: REED, Steven, G., Bellevue, WA 98005 (US); WANG, Tongtong, Medina, WA 98039 (US)
(74) Representative: Gowshall, Jonathan Vallance
(86) International application number: PCT/US1999/005798
(87) International publication number: WO 1999/047674

(56) References cited:
- WO-A-96/02552
- WO-A-99/44620
- CUNNINGHAM S A ET AL: "Cloning of an epithelial chloride channel from bovine trachea." THE JOURNAL OF BIOLOGICAL CHEMISTRY. UNITED STATES 29 DEC 1995, vol. 270, no. 52, 29 December 1995 (1995-12-29), pages 31016-31026, XP002280607 ISSN: 0021-9258
- ELBLE RANDOLPH C ET AL: "Cloning and characterization of lung-endothelial cell adhesion molecule-1 suggest it is an endothelial chloride channel" JOURNAL OF BIOLOGICAL CHEMISTRY, vol. 272, no. 44, 31 October 1997 (1997-10-31), pages 27853-27861, XP002280608 ISSN: 0021-9258
- LI XIURONG ET AL: "CLCA2 tumour suppressor gene in 1p31 is epigenetically regulated in breast cancer." ONCOGENE, vol. 23, no. 7, 19 February 2004 (2004-02-19), pages 1474-1480, XP002280609 ISSN: 0950-9232 (ISSN print)
- KONOPITZKY RENATE ET AL: "Identification of HLA-A*0201-restricted T cell epitopes derived from the novel overexpressed tumor antigen calcium-activated chloride channel 2" JOURNAL OF IMMUNOLOGY, vol. 169, no. 1, 1 July 2002 (2002-07-01), pages 540-547, XP002280610 ISSN: 0022-1767

## Description

### TECHNICAL FIELD

The present invention relates generally to compositions and methods for the treatment and diagnosis of lung cancer. The invention is more specifically related to nucleotide sequences that are preferentially expressed in lung tumor tissue, together with polypeptides encoded by such nucleotide sequences. The inventive nucleotide sequences and polypeptides may be used in vaccines and pharmaceutical compositions for the treatment and diagnosis of lung cancer.

### BACKGROUND OF THE INVENTION

Lung cancer is the primary cause of cancer death among both men and women in the U.S., with an estimated 172,000 new cases being reported in 1994. The five-year survival rate among all lung cancer patients, regardless of the stage of disease at diagnosis, is only 13%. This contrasts with a five-year survival rate of 46% among cases detected while the disease is still localized. However, only 16% of lung cancers are discovered before the disease has spread.

Early detection is difficult since clinical symptoms are often not seen until the disease has reached an advanced stage. Currently, diagnosis is aided by the use of chest x-rays, analysis of the type of cells contained in sputum and fiberoptic examination of the bronchial passages. Treatment regimens are determined by the type and stage of the cancer, and include surgery, radiation therapy and/or chemotherapy. In spite of considerable research into therapies for the disease, lung cancer remains difficult to treat.

Accordingly, there remains a need in the art for improved vaccines, treatment methods and diagnostic techniques for lung cancer.

WO 96/02552 discloses a lung cancer marker.

### SUMMARY OF THE INVENTION

Briefly stated, the present invention provides compounds and methods for the therapy of lung cancer. In a first aspect, isolated polynucleotide molecules encoding lung tumor polypeptides are provided, such polynucleotide molecules comprising a nucleotide sequence selected from the group consisting of: (a) sequences provided in SEQ ID NO: 160; (b) sequences complementary to a sequence provided in SEQ ID NO: 160.

In a second aspect, isolated polypeptides are provided that comprise at least an immunogenic portion of a lung tumor protein or a variant thereof. In specific embodiments, such polypeptides comprise an amino acid sequence encoded by a polynucleotide molecule comprising a nucleotide sequence selected from the group consisting of (a) sequences recited in SEQ ID NO: 160; (b) sequences complementary to a sequence provided in SEQ ID NO: 160.

In related aspects, expression vectors comprising the inventive polynucleotide molecules, together with host cells transformed or transfected with such expression vectors are provided. In preferred embodiments, the host cells are selected from the group consisting of *E. coli,* yeast and mammalian cells.

In another aspect, fusion proteins comprising a first and a second inventive polypeptide or, alternatively, an inventive polypeptide and a known lung tumor antigen, are provided.

The present invention further provides pharmaceutical compositions comprising one or more of the above polypeptides, fusion proteins or polynucleotide molecules and a physiologically acceptable carrier, together with vaccines comprising one or more such polypeptides, fusion proteins or polynucleotide molecules in combination with an immune response enhancer.

In related aspects, the present disclosure provides methods for inhibiting the development of lung cancer in a patient, comprising administering to a patient an effective amount of at least one of the above pharmaceutical compositions and/or vaccines.

Additionally, the present invention provides methods for immunodiagnosis of lung cancer, together with kits for use in such methods. Polypeptides are disclosed which comprise at least an immunogenic portion of a lung tumor protein or a variant of said protein that differs only in conservative substitutions and/or modifications, wherein the lung tumor protein comprises an amino acid sequence encoded by a polynucleotide molecule having a sequence selected from the group consisting of nucleotide sequences recited in SEQ ID NO: 160, and variants thereof. Such polypeptides may be usefully employed in the diagnosis and monitoring of lung cancer.

In one specific aspect of the present invention, methods are provided for detecting lung cancer in a patient, comprising: . (a) contacting a biological sample obtained from a patient with a binding agent that is capable of binding to one of the above polypeptides; and (b) detecting in the sample a protein or polypeptide that binds to the binding agent. In preferred embodiments, the binding agent is an antibody, most preferably a monoclonal antibody.

In related aspects, methods are provided for monitoring the progression of lung cancer in a patient, comprising: (a) contacting a biological sample obtained from a patient with a binding agent that is capable of binding to one of the above polypeptides; (b) determining in the sample an amount of a protein or polypeptide that binds to the binding agent; (c) repeating steps (a) and (b); and comparing the amounts of polypeptide detected in steps (b) and (c).

Within related aspects, the present invention provides antibodies, preferably monoclonal antibodies, that bind to the inventive polypeptides, as well as diagnostic kits comprising such antibodies, and methods of using such antibodies to inhibit the development of lung cancer.

The present invention further provides methods for detecting lung cancer comprising: (a) obtaining a biological sample from a patient; (b) contacting the sample with a first and a second oligonucleotide primer in a polymerase chain reaction, at least one of the oligonucleotide primers being specific for a polynucleotide molecule that encodes one of the above polypeptides; and (c) detecting in the sample a polynucleotide sequence that amplifies in the presence of the first and second oligonucleotide primers. In a preferred embodiment, at least one of the oligonucleotide primers comprises at least about 10 contiguous nucleotides of a polynucleotide molecule including a sequence selected from the group consisting of SEQ ID NO: 160.

In a further aspect, the present invention provides a method for detecting lung cancer in a patient comprising: (a) contacting a biological sample obtained from a patient with an oligonucleotide probe specific for a polynucleotide molecule that encodes one of the above polypeptides; and (c) detecting in the sample a polynucleotide sequence that hybridizes to the oligonucleotide probe. Preferably, the oligonucleotide probe comprises at least about 15 contiguous nucleotides of a polynucleotide molecule having a partial sequence selected from the group consisting of SEQ ID NO: 160.

In related aspects, diagnostic kits comprising the above oligonucleotide probes or primers are provided.

In yet a further aspect, methods for the treatment of lung cancer in a patient are disclosed, the methods comprising obtaining PBMC from the patient, incubating the PBMC with a polypeptide of the present invention (or a polynucleotide that encodes such a polypeptide) to provide incubated T cells and administering the incubated T cells to the patient. The present invention additionally provides methods for the treatment of lung cancer that comprise incubating antigen presenting cells with a polypeptide of the present invention (or a polynucleotide that encodes such a polypeptide) to provide incubated antigen presenting cells and administering the incubated antigen presenting cells to the patient. In certain embodiments, the antigen presenting cells are selected from the group consisting of dendritic cells and macrophages. Compositions for the treatment of lung cancer comprising T cells or antigen presenting cells that have been incubated with a polypeptide or polynucleotide of the present invention are also provided. These and other aspects of the present invention will become apparent upon reference to the following detailed description.

### DETAILED DESCRIPTION OF THE INVENTION

As noted above, the present invention is generally directed to compositions and their use in therapy and diagnosis of lung cancer and methods for the diagnosis of lung cancer. The compositions described herein include polypeptides, fusion proteins and polynucleotide molecules. Also included within the present invention are molecules (such as an antibody or fragment thereof) that bind to the inventive polypeptides. Such molecules are referred to herein as "binding agents."

In one aspect, the subject invention discloses polypeptides comprising an immunogenic portion of a human lung tumor protein, wherein the lung tumor protein includes an amino acid sequence encoded by a polynucleotide molecule including a sequence selected from the group consisting of (a) the nucleotide sequence recited in SEQ ID NO: 160, (b) the complements of said nucleotide sequences. As used herein, the term "polypeptide" encompasses amino acid chains of any length, including full length proteins, wherein the amino acid residues are linked by covalent peptide bonds. Thus, a polypeptide comprising a portion of one of the above lung tumor proteins may consist entirely of the portion, or the portion may be present within a larger polypeptide that contains additional sequences. The additional sequences may be derived from the native protein or may be heterologous, and such sequences may (but need not) be immunoreactive and/or antigenic. As detailed below, such polypeptides may be isolated from lung tumor tissue or prepared by synthetic or recombinant means.

As used herein, an "immunogenic portion" of a lung tumor protein is a portion that is capable of eliciting an immune response in a patient inflicted with lung cancer and as such binds to antibodies present within sera from a lung cancer patient. Such immunogenic portions generally comprise at least about 5 amino acid residues, more preferably at least about 10, and most preferably at least about 20 amino acid residues. Immunogenic portions of the proteins described herein may be identified in antibody binding assays. Such assays may generally be performed using any of a variety of means known to those of ordinary skill in the art, as described, for example, in Harlow and Lane, *Antibodies: A Laboratory Manual*, Cold Spring Harbor Laboratory, Cold Spring Harbor, NY, 1988. For example, a polypeptide may be immobilized on a solid support (as described below) and contacted with patient sera to allow binding of antibodies within the sera to the immobilized polypeptide. Unbound sera may then be removed and bound antibodies detected using, for example, ¹²⁵I-labeled Protein A. Alternatively, a polypeptide may be used to generate monoclonal and polyclonal antibodies for use in detection of the polypeptide in blood or other fluids of lung cancer patients. Methods for preparing and identifying immunogenic portions of antigens of known sequence are well known in the art and include those summarized in Paul, *Fundamental Immunology,* 3^{rd} ed., Raven Press, 1993, pp. 243-247.

The term "polynucleotide(s)," as used herein, means a single or double-stranded polymer of deoxyribonucleotide or ribonucleotide bases and includes DNA and corresponding RNA molecules, including HnRNA and mRNA molecules, both sense and anti-sense strands, and comprehends cDNA, genomic DNA and recombinant DNA, as well as wholly or partially synthesized polynucleotides. An HnRNA molecule contains introns and corresponds to a DNA molecule in a generally one-to-one manner. An mRNA molecule corresponds to an HnRNA and DNA molecule from which the introns have been excised. A polynucleotide may consist of an entire gene, or any portion thereof. Operable anti-sense polynucleotides may comprise a fragment of the corresponding polynucleotide, and the definition of "polynucleotide" therefore includes all such operable anti-sense fragments.

The compositions and methods of the present invention also encompass variants of the above polypeptides and polynucleotides. A polypeptide "variant," as used herein, is a polypeptide that differs from the recited polypeptide only in conservative substitutions and/or modifications, such that the therapeutic, antigenic and/or immunogenic properties of the polypeptide are retained. In a preferred embodiment, variant polypeptides differ from an identified sequence by substitution, deletion or addition of five amino acids or fewer. Such variants may generally be identified by modifying one of the above polypeptide sequences, and evaluating the antigenic properties of the modified polypeptide using, for example, the representative procedures described herein. Polypeptide variants preferably exhibit at least about 70%, more preferably at least about 90% and most preferably at least about 95% identity (determined as describe below) to the identified polypeptides.

As used herein, a "conservative substitution" is one in which an amino acid is substituted for another amino acid that has similar properties, such that one skilled in the art of peptide chemistry would expect the secondary structure and hydropathic nature of the polypeptide to be substantially unchanged. In general, the following groups of amino acids represent conservative changes: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his.

Variants may also, or alternatively, contain other modifications including the deletion or addition of amino acids that have minimal influence on the antigenic properties, secondary structure and hydropathic nature of the polypeptide. For example, a polypeptide may be conjugated to a signal (or leader) sequence at the N-terminal end of the protein which co-translationally or post-translationally directs transfer of the protein. The polypeptide may also be conjugated to a linker or other sequence for ease of synthesis, purification or identification of the polypeptide (*e.g*., poly-His), or to enhance binding of the polypeptide to a solid support. For example, a polypeptide may be conjugated to an immunoglobulin Fc region.

A nucleotide "variant" is a sequence that differs from the recited nucleotide sequence in having one or more nucleotide deletions, substitutions or additions. Such modifications may be readily introduced using standard mutagenesis techniques, such as oligonucleotide-directed site-specific mutagenesis as taught, for example, by Adelman et al. (*DNA*, *2*:183, 1983). Nucleotide variants may be naturally occurring allelic variants, or non-naturally occurring variants. Variant nucleotide sequences preferably exhibit at least about 70%, more preferably at least about 80% and most preferably at least about 90% identity (determined as described below) to the recited sequence.

The antigens provided by the present invention include variants that are encoded by polynucleotide sequences which are substantially homologous to one or more of the polynucleotide sequences specifically recited herein. "Substantial homology," as used herein, refers to polynucleotide sequences that are capable of hybridizing under moderately stringent conditions. Suitable moderately stringent conditions include prewashing in a solution of 5X SSC, 0.5% SDS, 1.0 mM EDTA (pH 8.0); hybridizing at 50°C-65°C, 5X SSC, overnight or, in the event of cross-species homology, at 45°C with 0.5X SSC; followed by washing twice at 65°C for 20 minutes with each of 2X, 0.5X and 0.2X SSC containing 0.1 % SDS. Such hybridizing polynucleotide sequences are also within the scope of this invention, as are nucleotide sequences that, due to code degeneracy, encode an immunogenic polypeptide that is encoded by a hybridizing polynucleotide sequence.

Two nucleotide or polypeptide sequences are said to be "identical" if the sequence of nucleotides or amino acid residues in the two sequences is the same when aligned for maximum correspondence as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships, in Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 *Methods in Enzymology* vol. 183, Academic Press, Inc., San Diego, CA; Higgins, D.G. and Sharp, P.M. (1989) Fast and sensitive multiple sequence alignments on a microcomputer *CABIOS 5*:151-153; Myers, E.W. and Muller W. (1988) Optimal alignments in linear space *CABIOS 4*:11-17; Robinson, E.D. (1971) *Comb. Theor 11*:105: Santou, N. Nes, M. (1987) The neighbor joining method. A new method for reconstructing phylogenetic trees *Mol. Biol. Evol. 4*:406-425; Sneath, P.H.A. and Sokal, R.R. (1973) *Numerical Taxonomy - the Principles and Practice of Numerical Taxonomy*, Freeman Press, San Francisco, CA: Wilbur, W.J. and Lipman, D.J. (1983) Rapid similarity searches of nucleic acid and protein data banks *Proc. Natl. Acad.*, *Sci. USA 80*:726-730.

Preferably, the "percentage of sequence identity" is determined by comparing two optimally aligned sequences over a window of comparison of at least 20 positions, wherein the portion of the polynucleotide sequence in the comparison window may comprise additions or deletions (i.e. gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases or amino acid residue occurs in both sequences to yield the number of matched positions, dividing the number of matched positions by the total number of positions in the reference sequence (i.e. the window size) and multiplying the results by 100 to yield the percentage of sequence identity.

Also included in the scope of the present invention are alleles of the genes encoding the nucleotide sequences recited in herein. As used herein, an "allele" or "allellic sequence" is an alternative form of the gene which may result from at least one mutation in the nucleic acid sequence. Alleles may result in altered mRNAs or polypeptides whose structure or function may or may not be altered. Any given gene may have none, one, or many allelic forms. Common mutational changes which give rise to alleles are generally ascribed to natural deletions, additions, or substitutions of nucleotides. Each of these types of changes may occur alone or in combination with the others, one or more times in a given sequence.

For lung tumor polypeptides with immunoreactive properties, variants may, alternatively, be identified by modifying the amino acid sequence of one of the above polypeptides, and evaluating the immunoreactivity of the modified polypeptide. For lung tumor polypeptides useful for the generation of diagnostic binding agents, a variant may be identified by evaluating a modified polypeptide for the ability to generate antibodies that detect the presence or absence of lung cancer. Such modified sequences may be prepared and tested using, for example, the representative procedures described herein.

The lung tumor polypeptides of the present invention, and polynucleotide molecules encoding such polypeptides, may be isolated from lung tumor tissue using any of a variety of methods well known in the art. Polynucleotide sequences corresponding to a gene (or a portion thereof) encoding one of the inventive lung tumor proteins may be isolated from a lung tumor cDNA library using a subtraction technique as described in detail below. Examples of such polynucleotide sequences are provided in SEQ ID NO: 1-109,111,113 115-151, 153, 154, 157, 158, 160, 162-164, 167, 168 and 171. Partial polynucleotide sequences thus obtained may be used to design oligonucleotide primers for the amplification of full-length polynucleotide sequences from a human genomic DNA library or from a lung tumor cDNA library in a polymerase chain reaction (PCR), using techniques well known in the art (see, for example, Mullis et al., *Cold Spring Harbor Symp. Quant. Biol. 51*:263, 1987; Erlich ed., *PCR Technology,* Stockton Press, NY, 1989). For this approach, sequence-specific primers may be designed based on the nucleotide sequences provided herein and may be purchased or synthesized.

An amplified portion may be used to isolate a full length gene from a suitable library (*e.g.,* a lung tumor cDNA library) using well known techniques. Within such techniques, a library (cDNA or genomic) is screened using one or more polynucleotide probes or primers suitable for amplification. Preferably, a library is size-selected to include larger molecules. Random primed libraries may also be preferred for identifying 5' and upstream regions of genes. Genomic libraries are preferred for obtaining introns and extending 5' sequences.

For hybridization techniques, a partial sequence may be labeled (*e.g.*, by nick-translation or end-labeling with ³²P) using well known techniques. A bacterial or bacteriophage library is then screened by hybridizing filters containing denatured bacterial colonies (or lawns containing phage plaques) with the labeled probe (*see* Sambrook et al., *Molecular Cloning: A Laboratory Manual,* Cold Spring Harbor Laboratories, Cold Spring Harbor, NY, 1989). Hybridizing colonies or plaques are selected and expanded, and the DNA is isolated for further analysis. cDNA clones may be analyzed to determine the amount of additional sequence by, for example, PCR using a primer from the partial sequence and a primer from the vector. Restriction maps and partial sequences may be generated to identify one or more overlapping clones. The complete sequence may then be determined using standard techniques, which may involve generating a series of deletion clones. The resulting overlapping sequences are then assembled into a single contiguous sequence. A full length cDNA molecule can be generated by ligating suitable fragments, using well known techniques.

Alternatively, there are numerous amplification techniques for obtaining a full length coding sequence from a partial cDNA sequence. Within such techniques, amplification is generally performed via PCR. Any of a variety of commercially available kits may be used to perform the amplification step. Primers may be designed using techniques well known in the art (*see,* for example, Mullis et al., *Cold Spring Harbor Symp. Quant Biol. 51*:263, 1987; Erlich ed., *PCR Technology,* Stockton Press, NY, 1989), and software well known in the art may also be employed. Primers are preferably 22-30 nucleotides in length, have a GC content of at least 50% and anneal to the target sequence at temperatures of about 68°C to 72°C. The amplified region may be sequenced as described above, and overlapping sequences assembled into a contiguous sequence.

One such amplification technique is inverse PCR (*see* Triglia et al., *Nucl. Acids Res. 16*:8186, 1988), which uses restriction enzymes to generate a fragment in the known region of the gene. The fragment is then circularized by intramolecular ligation and used as a template for PCR with divergent primers derived from the known region. Within an alternative approach, sequences adjacent to a partial sequence may be retrieved by amplification with a primer to a linker sequence and a primer specific to a known region. The amplified sequences are typically subjected to a second round of amplification with the same linker primer and a second primer specific to the known region. A variation on this procedure, which employs two primers that initiate extension in opposite directions from the known sequence, is described in WO 96/38591. Additional techniques include capture PCR (Lagerstrom et al., *PCR Methods Applic. 1:*111-19, 1991) and walking PCR (Parker et al., *Nucl. Acids. Res. 19*:3055-60, 1991). Transcription-Mediated Amplification, or TMA is another method that may be utilized for the amplification of DNA, rRNA. or mRNA, as described in Patent No. PCT/US91/03184. This autocatalytic and isothermic non-PCR based method utilizes two primers and two enzymes: RNA polymerase and reverse transcriptase. One primer contains a promoter sequence for RNA polymerase. In the first amplification, the promoter-primer hybridizes to the target rRNA at a defined site. Reverse transcriptase creates a DNA copy of the target rRNA by extension from the 3'end of the promoter-primer. The RNA in the resulting complex is degraded and a second primer binds to the DNA copy. A new strand of DNA is synthesized from the end of the primer by reverse transcriptase creating double stranded DNA. RNA polymerase recognizes the promoter sequence in the DNA template and initiates transcription. Each of the newly synthesized RNA amplicons re-enters the TMA process and serves as a template for a new round of replication leading to the expotential expansion of the RNA amplicon. Other methods employing amplification may also be employed to obtain a full length cDNA sequence.

In certain instances, it is possible to obtain a full length cDNA sequence by analysis of sequences provided in an expressed sequence tag (EST) database, such as that available from GenBank. Searches for overlapping ESTs may generally be performed using well known programs (*e.g.*, NCBI BLAST searches), and such ESTs may be used to generate a contiguous full length sequence.

Once a polynucleotide sequence encoding a polypeptide is obtained, the polypeptide may be produced recombinantly by inserting the polynucleotide sequence into an expression vector and expressing the polypeptide in an appropriate host. Any of a variety of expression vectors known to those of ordinary skill in the art may be employed to express recombinant polypeptides of this invention. Expression may be achieved in any appropriate host cell that has been transformed or transfected with an expression vector containing a polynucleotide molecule that encodes the recombinant polypeptide. Suitable host cells include prokaryotes, yeast, insect and higher eukaryotic cells. Preferably, the host cells employed are *E. coli,* yeast or a mammalian cell line, such as COS or CHO cells. The polynucleotide sequences expressed in this manner may encode naturally occurring polypeptides, portions of naturally occurring polypeptides, or other variants thereof. Supernatants from suitable host/vector systems which secrete the recombinant polypeptide may first be concentrated using a commercially available filter. The concentrate may then be applied to a suitable purification matrix, such as an affinity matrix or ion exchange resin. Finally, one or more reverse phase HPLC steps can be employed to further purify the recombinant polypeptide.

The lung tumor polypeptides disclosed herein may also be generated by synthetic means. In particular, synthetic polypeptides having fewer than about 100 amino acids, and generally fewer than about 50 amino acids, may be generated using techniques well known to those of ordinary skill in the art. For example, such polypeptides may be synthesized using any of the commercially available solid-phase techniques, such as the Merrifield solid-phase synthesis method, where amino acids are sequentially added to a growing amino acid chain (see, for example, Merrifield, *J. Am. Chem. Soc. 85*:2149-2146, 1963). Equipment for automated synthesis of polypeptides is commercially available from suppliers such as Perkin Elmer/Applied BioSystems Division (Foster City, CA), and may be operated according to the manufacturer's instructions.

In addition, lung tumor antigens may be identified by T cell expression cloning. One source of tumor specific T cells is from surgically excised tumors from human patients. In one method for isolating and characterizing tumor specific T cells, the excised tumor is minced and enzymatically digested for several hours to release tumor cells and infiltrating lymphocytes (tumor infiltrating T cells, or TILs). The cells are washed in HBSS buffer and passed over a Ficoll (100%/75%/HBSS) discontinuous gradient to separate tumor cells and lymphocytes from non-viable cells. Two bands are harvested from the interfaces; the upper band at the 75%/HBSS interface contains predominantly tumor cells, while the lower band at the 100%/75%/HBSS interface contains a majority of lymphocytes. The TILs are expanded in culture by techniques well known in the art, but preferably in culture media supplemented with 10 ng/ml IL-7 and 100 U/ml IL-2, or alternatively, cultured and expanded in tissue culture plates that have been pre-adsorbed with anti-CD3 monoclonal antibody (OKT3). The resulting TIL cultures are analyzed by FACS to confirm that the vast majority are CD8+ T cells (>90% of gated population).

In addition, the tumor cells are also expanded in culture using standard techniques well known in the art to establish a tumor cell line, which is later confirmed to be lung carcinoma cells by immunohistochemical analysis. The tumor cell line is transduced with a retroviral vector to express human CD80. The tumor cell line is further characterized by FACS analysis to confirm the strong expression levels of CD80, class I and II MHC molecules.

The specificity of the TIL lines to lung tumor is confirmed by INF-γ and/or TNF-α cytokine release assays. For example, TIL cells from day 21 cultures are co-cultured with either autologous or allogeneic tumor cells, EBV-immortalized LCL, or control cell lines Daudi and K562 and the culture supernatant monitored by ELISA for the presence of cytokines. The expression of these specific cytokines in the presence of tumor or negative control cells indicates whether the TIL lines are tumor specific and potentially recognizing tumor antigen presented by the autologous MHC molecules.

The characterized tumor-specific TIL lines can be expanded and cloned by methods well known in the art. For example, the TIL lines may be expanded to suitable numbers for T cell expression cloning by using soluble anti-CD3 antibody in culture with irradiated EBV transformed LCLs and PBL feeder cells in the presence of 20 U/ml IL-2. Clones from the expanded TIL lines can be generated by standard limiting dilution techniques. In particular, TIL cells are seeded at 0.5 cells/well in a 96-well U bottom plate and stimulated with CD-80-transduced autologous tumor cells, EBV transformed LCL, and PBL feeder cells in the presence of 50 U/ml IL-2. These clones may be further analyzed for tumor specificity by ⁵¹Cr microcytotoxicity and IFN-γ bioassays. Additionally, the MHC restriction element recognized by the TIL clones may be determined by antibody blocking studies well known in the art.

The CTL lines or clones described above may be employed to identify tumor specific antigens. For example, autologous fibroblasts or LCL from a patient may be transfected or transduced with polynucleotide fragments derived from a lung tumor cDNA library to generate target cells expressing tumor polypeptides. The target cells expressing tumor polypeptides in the context of MHC will be recognized by the CTL line or clone resulting in T-cell activation, which can be monitored by cytokine detection assays. The tumor gene being expressed by the target cell and recognized by the tumor-specific CTL is then isolated by techniques described above. In general, regardless of the method of preparation, the polypeptides disclosed herein are prepared in an isolated, substantially pure form (*i.e.*, the polypeptides are homogenous as determined by amino acid composition and primary sequence analysis). Preferably, the polypeptides are at least about 90% pure, more preferably at least about 95% pure and most preferably at least about 99% pure. In certain preferred embodiments, described in more detail below, the substantially pure polypeptides are incorporated into pharmaceutical compositions or vaccines for use in one or more of the methods disclosed herein.

In a related aspect, the present invention provides fusion proteins comprising a first and a second inventive polypeptide or, alternatively, a polypeptide of the present invention and a known lung tumor antigen, together with variants of such fusion proteins. The fusion proteins of the present invention may (but need not) include a linker peptide between the first and second polypeptides.

A polynucleotide sequence encoding a fusion protein of the present invention is constructed using known recombinant DNA techniques to assemble separate polynucleotide sequences encoding the first and second polypeptides into an appropriate expression vector. The 3' end of a DNA sequence encoding the first polypeptide is ligated, with or without a peptide linker, to the 5' end of a DNA sequence encoding the second polypeptide so that the reading frames of the sequences are in phase to permit mRNA translation of the two DNA sequences into a single fusion protein that retains the biological activity of both the first and the second polypeptides.

A peptide linker sequence may be employed to separate the first and the second polypeptides by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., *Gene 40*:39-46, 1985; Murphy et al., *Proc. Natl. Acad Sci. USA 83*:8258-8262, 1986; U.S. Patent No. 4,935,233 and U.S. Patent No. 4,751,180. The linker sequence may be from 1 to about 50 amino acids in length. Peptide sequences are not required when the first and second polypeptides have non-essential N-terminal amino acid regions that can be used to separate the functional domains and prevent steric interference.

The ligated polynucleotide sequences are operably linked to suitable transcriptional or translational regulatory elements. The regulatory elements responsible for expression of polynucleotide are located only 5' to the DNA sequence encoding the first polypeptides. Similarly, stop codons require to end translation and transcription termination signals are only present 3' to the DNA sequence encoding the second polypeptide.

Fusion proteins are also provided that comprise a polypeptide of the present invention together with an unrelated immunogenic protein. Preferably the immunogenic protein is capable of eliciting a recall response. Examples of such proteins include tetanus, tuberculosis and hepatitis proteins (see, for example, Stoute et al. *New Engl. J. Med., 336*:86-91 (1997)).

Polypeptides of the present invention that comprise an immunogenic portion of a lung tumor protein may generally be used for therapy of lung cancer, wherein the polypeptide stimulates the patient's own immune response to lung tumor cells. The present invention thus provides methods for using one or more of the compounds described herein (which may be polypeptides, polynucleotide molecules or fusion proteins) for immunotherapy of lung cancer in a patient. As used herein, a "patient" refers to any warm-blooded animal, preferably a human. A patient may be afflicted with disease, or may be free of detectable disease. Accordingly, the compounds disclosed herein may be used to treat lung cancer or to inhibit the development of lung cancer. The compounds are preferably administered either prior to or following surgical removal of primary tumors and/or treatment by administration of radiotherapy and conventional chemotherapeutic drugs.

In these aspects, the inventive polypeptide is generally present within a pharmaceutical composition or a vaccine. Pharmaceutical compositions may comprise one or more polypeptides, each of which may contain one or more of the above sequences (or variants thereof), and a physiologically acceptable carrier. The vaccines may comprise one or more such polypeptides and a non-specific immune-response enhancer, wherein the non-specific immune response enhancer is capable of eliciting or enhancing an immune response to an exogenous antigen. Examples of non-specific-immune response enhancers include adjuvants, biodegradable microspheres (*e.g.*, polylactic galactide) and liposomes (into which the polypeptide is incorporated). Pharmaceutical compositions and vaccines may also contain other epitopes of lung tumor antigens, either incorporated into a fusion protein as described above (*i.e.*, a single polypeptide that contains multiple epitopes) or present within a separate polypeptide.

Alternatively, a pharmaceutical composition or vaccine may contain polynucleotide encoding one or more of the above polypeptides and/or fusion proteins, such that the polypeptide is generated *in situ.* In such pharmaceutical compositions and vaccines, the polynucleotide may be present within any of a variety of delivery systems known to those of ordinary skill in the art, including nucleic acid expression systems, bacteria and viral expression systems. Appropriate nucleic acid expression systems contain the necessary polynucleotide sequences for expression in the patient (such as a suitable promoter). Bacterial delivery systems involve the administration of a bacterium (such as *Bacillus-Calmette-Guerrin*) that expresses an epitope of a lung cell antigen on its cell surface. In a preferred embodiment, the polynucleotides may be introduced using a viral expression system (*e.g.*, vaccinia or other pox virus, retrovirus, or adenovirus), which may involve the use of a non-pathogenic (defective), replication competent virus. Suitable systems are disclosed, for example, in Fisher-Hoch et al., *PNAS 86*:317-321, 1989; Flexner et al., *Ann N.Y. Acad. Sci*. *569:*86-103, 1989; Flexner et al., *Vaccine 8*:17-21, 1990; U.S. Patent Nos. 4,603,112, 4,769,330, and 5,017,487; WO 89/01973; U.S. Patent No. 4,777,127; GB 2,200,651; EP 0,345,242; WO 91/02805; Berkner, *Biotechniques 6*:616-627, 1988; Rosenfeld et al., *Science 252*:431-434, 1991; Kolls et al., *PNAS 91*:215-219, 1994; Kass-Eisler et al., *PNAS 90*:11498-11502, 1993; Guzman et al., *Circulation 88*:2838-2848, 1993; and Guzman et al., *Cir. Res. 73*:1202-1207, 1993. Techniques for incorporating polynucleotide into such expression systems are well known to those of ordinary skill in the art. The polynucleotides may also be "naked," as described, for example, in published PCT application WO 90/11092, and Ulmer et al., *Science 259*:1745-1749, 1993, reviewed by Cohen, *Science 259*:1691-1692, 1993. The uptake of naked polynucleotides may be increased by coating the polynucleotides onto biodegradable beads, which are efficiently transported into the cells.

Routes and frequency of administration, as well as dosage, will vary from individual to individual and may parallel those currently being used in immunotherapy of other diseases. In general, the pharmaceutical compositions and vaccines may be administered by injection (*e.g.,* intracutaneous, intramuscular, intravenous or subcutaneous), intranasally (*e.g.,* by aspiration) or orally. Between 1 and 10 doses may be administered over a 3-24 week period. Preferably, 4 doses are administered, at an interval of 3 months, and booster administrations may be given periodically thereafter. Alternate protocols may be appropriate for individual patients. A suitable dose is an amount of polypeptide or polynucleotide that is effective to raise an immune response (cellular and/or humoral) against lung tumor cells in a treated patient. A suitable immune response is at least 10-50% above the basal (*i.e.,* untreated) level. In general, the amount of polypeptide present in a dose (or produced *in situ* by the polynucleotide molecule(s) in a dose) ranges from about 1 pg to about 100 mg per kg of host, typically from about 10 pg to about 1 mg, and preferably from about 100 pg to about 1 µg. Suitable dose sizes will vary with the size of the patient, but will typically range from about 0.01 mL to about 5 mL.

While any suitable carrier known to those of ordinary skill in the art may be employed in the pharmaceutical compositions of this invention, the type of carrier will vary depending on the mode of administration. For parenteral administration, such subcutaneous injection, the carrier preferably comprises water, saline, alcohol, a lipid, a wax and/or a buffer. For oral administration, any of the above carriers or a solid carrier, such as mannitol, lactose, starch, magnesium stearate, sodium saccharine, talcum, cellulose, glucose, sucrose, and/or magnesium carbonate, may be employed. Biodegradable microspheres (*e.g*., polylactic glycolide) may also be employed as carriers for the pharmaceutical compositions of this invention. Suitable biodegradable microspheres are disclosed, for example, in U.S. Patent Nos. 4,897,268 and 5,075,109.

Any of a variety of immune-response enhancers may be employed in the vaccines of this invention. For example, an adjuvant may be included. Most adjuvants contain a substance designed to protect the antigen from rapid catabolism, such as aluminum hydroxide or mineral oil, and a nonspecific stimulator of immune response, such as lipid A, *Bordella pertussis* or *Mycobacterium tuberculosis.* Such adjuvants are commercially available as, for example, Freund's Incomplete Adjuvant and Complete Adjuvant (Difco Laboratories, Detroit, MI) and Merck Adjuvant 65 (Merck and Company, Inc., Rahway, NJ).Polypeptides and polynucleotides disclosed herein may also be employed in adoptive immunotherapy for the treatment of cancer. Adoptive immunotherapy may be broadly classified into either active or passive immunotherapy. In active immunotherapy, treatment relies on the *in vivo* stimulation of the endogenous host immune system to react against tumors with the administration of immune response-modifying agents (for example, tumor vaccines, bacterial adjuvants, and/or cytokines).

In passive immunotherapy, treatment involves the delivery of biologic reagents with established tumor-immune reactivity (such as effector cells or antibodies) that can directly or indirectly mediate antitumor effects and does not necessarily depend on an intact host immune system. Examples of effector cells include T lymphocytes (for example, CD8+ cytotoxic T-lymphocyte, CD4+ T-helper, gamma/delta T lymphocytes, tumor-infiltrating lymphocytes), killer cells (such as Natural Killer cells, lymphokine-activated killer cells), B cells, or antigen presenting cells (such as dendritic cells and macrophages) expressing the disclosed antigens. The polypeptides disclosed herein may also be used to generate antibodies or anti-idiotypic antibodies (as in U.S. Patent No. 4,918,164), for passive immunotherapy.

The predominant method of procuring adequate numbers of T-cells for adoptive immunotherapy is to grow immune T-cells *in vitro.* Culture conditions for expanding single antigen-specific T-cells to several billion in number with retention of antigen recognition *in vivo* are well known in the art. These *in vitro* culture conditions typically utilize intermittent stimulation with antigen, often in the presence of cytokines, such as IL-2, and non-dividing feeder cells. As noted above, the immunoreactive polypeptides described herein may be used to rapidly expand antigen-specific T cell cultures in order to generate sufficient number of cells for immunotherapy. In particular, antigen-presenting cells, such as dendritic, macrophage, monocyte, fibroblast, or B-cells, may be pulsed with immunoreactive polypeptides, or polynucleotide sequence(s) may be introduced into antigen presenting cells, using a variety of standard techniques well known in the art. For example, antigen presenting cells may be transfected or transduced with a polynucleotide sequence, wherein said sequence contains a promoter region appropriate for increasing expression, and can be expressed as part of a recombinant virus or other expression system. Several viral vectors may be used to transduce an antigen presenting cell, including pox virus, vaccinia virus, and adenovirus; also, antigen presenting cells may be transfected with polynucleotide sequences disclosed herein by a variety of means, including gene-gun technology, lipid-mediated delivery, electroporation, osmotic shock, and particlate delivery mechanisms, resulting in efficient and acceptable expression levels as determined by one of ordinary skill in the art. For cultured T-cells to be effective in therapy, the cultured T-cells must be able to grow and distribute widely and to survive long term *in vivo.* Studies have demonstrated that cultured T-cells can be induced to grow *in vivo* and to survive long term in substantial numbers by repeated stimulation with antigen supplemented with IL-2 (see, for example, Cheever, M., *et al,* "Therapy With Cultured T Cells: Principles Revisited, " *Immunological Reviews, 157:177,* 1997).

The polypeptides disclosed herein may also be employed to generate and/or isolate tumor-reactive T-cells, which can then be administered to the patient. In one technique, antigen-specific T-cell lines may be generated by *in vivo* immunization with short peptides corresponding to immunogenic portions of the disclosed polypeptides. The resulting antigen specific CD8+CTL clones may be isolated from the patient, expanded using standard tissue culture techniques, and returned to the patient.

Alternatively, peptides corresponding to immunogenic portions of the polypeptides may be employed to generate tumor reactive T cell subsets by selective *in vitro* stimulation and expansion of autologous T cells to provide antigen-specific T -cells which may be subsequently transferred to the patient as described, for example, by Chang *et al,* (*Crit. Rev. Oncol. Hematol., 22*(3),213, 1996). Cells of the immune system, such as T cells, may be isolated from the peripheral blood of a patient, using a commercially available cell separation system, such as CellPro Incorporated's (Bothell, WA) CEPRATE^{™} system (see U.S. Patent No. 5,240,856; U.S. Patent No. 5,215,926; WO 89/06280; WO 91/16116 and WO 92/07243). The separated cells are stimulated with one or more of the immunoreactive polypeptides contained within a delivery vehicle, such as a microsphere, to provide antigen-specific T cells. The population of tumor antigen-specific T cells is then expanded using standard techniques and the cells are administered back to the patient.

In other embodiments, T-cell and/or antibody receptors specific for the polypeptides disclosed herein can be cloned, expanded, and transferred into other vectors or effector cells for use in adoptive immunotherapy. In particular, T cells may be transfected with the appropriate genes to express the variable domains from tumor specific monoclonal antibodies as the extracellular recognition elements and joined to the T cell receptor signaling chains, resulting in T cell activation, specific lysis, and cytokine release. This enables the T cell to redirect its specificity in an MHC-independent manner. See for example, Eshhar, Z., *Cancer Immunol Immunother,* 45(3-4):131-6, 1997 and Hwu, P., et al, *Cancer Res,* 55(15):3369-73, 1995. Another embodiment may include the transfection of tumor antigen specific alpha and beta T cell receptor chains into alternate T cells, as in Cole, DJ, et al, *Cancer Res,* 55(4):748-52, 1995.

In a further embodiment, syngeneic or autologous dendritic cells may be pulsed with peptides corresponding to at least an immunogenic portion of a polypeptide disclosed herein. The resulting antigen-specific dendritic cells may either be transferred into a patient, or employed to stimulate T cells to provide antigen-specific T cells which may, in turn, be administered to a patient. The use of peptide-pulsed dendritic cells to generate antigen-specific T cells and the subsequent use of such antigen-specific T cells to eradicate tumors in a murine model has been demonstrated by Cheever et al, *Immunological Reviews 157*:177, 1997).

Furthermore, vectors expressing the disclosed polynucleotides may be introduced into stem cells taken from the patient and clonally propagated *in vitro* for autologous transplant back into the same patient.

Additionally, vectors expressing the disclosed polynucleotides may be introduced into stem cells taken from the patient and clonally propagated *in vitro* for autologous transplant back into the same patient. Polypeptides and fusion proteins of the present invention may also, or alternatively, be used to generate binding agents, such as antibodies or fragments thereof, that are capable of detecting metastatic human lung tumors. Binding agents of the present invention may generally be prepared using methods known to those of ordinary skill in the art, including the representative procedures described herein. Binding agents are capable of differentiating between patients with and without lung cancer, using the representative assays described herein. In other words, antibodies or other binding agents raised against a lung tumor protein, or a suitable portion thereof, will generate a signal indicating the presence of primary or metastatic lung cancer in at least about 20% of patients afflicted with the disease, and will generate a negative signal indicating the absence of the disease in at least about 90% of individuals without primary or metastatic lung cancer. Suitable portions of such lung tumor proteins are portions that are able to generate a binding agent that indicates the presence of primary or metastatic lung cancer in substantially all (*i.e.,* at least about 80%, and preferably at least about 90%) of the patients for which lung cancer would be indicated using the full length protein, and that indicate the absence of lung cancer in substantially all of those samples that would be negative when tested with full length protein. The representative assays described below, such as the two-antibody sandwich assay, may generally be employed for evaluating the ability of a binding agent to detect metastatic human lung tumors.

The ability of a polypeptide prepared as described herein to generate antibodies capable of detecting primary or metastatic human lung tumors may generally be evaluated by raising one or more antibodies against the polypeptide (using, for example, a representative method described herein) and determining the ability of such antibodies to detect such tumors in patients. This determination may be made by assaying biological samples from patients with and without primary or metastatic lung cancer for the presence of a polypeptide that binds to the generated antibodies. Such test assays may be performed, for example, using a representative procedure described below. Polypeptides that generate antibodies capable of detecting at least 20% of primary or metastatic lung tumors by such procedures are considered to be useful in assays for detecting primary or metastatic human lung tumors. Polypeptide specific antibodies may be used alone or in combination to improve sensitivity.

Polypeptides capable of detecting primary or metastatic human lung tumors may be used as markers for diagnosing lung cancer or for monitoring disease progression in patients. In one embodiment, lung cancer in a patient may be diagnosed by evaluating a biological sample obtained from the patient for the level of one or more of the above polypeptides, relative to a predetermined cut-off value. As used herein, suitable "biological samples" include blood, sera, urine and/or lung secretions.

The level of one or more of the above polypeptides may be evaluated using any binding agent specific for the polypeptide(s). A "binding agent," in the context of this invention, is any agent (such as a compound or a cell) that binds to a polypeptide as described above. As used herein, "binding" refers to a noncovalent association between two separate molecules (each of which may be free (*i.e.,* in solution) or present on the surface of a cell or a solid support), such that a "complex" is formed. Such a complex may be free or immobilized (either covalently or noncovalently) on a support material. The ability to bind may generally be evaluated by determining a binding constant for the formation of the complex. The binding constant is the value obtained when the concentration of the complex is divided by the product of the component concentrations. In general, two compounds are said to "bind" in the context of the present invention when the binding constant for complex formation exceeds about 10³ L/mol. The binding constant may be determined using methods well known to those of ordinary skill in the art.

Any agent that satisfies the above requirements may be a binding agent. For example, a binding agent may be a ribosome with or without a peptide component, an RNA molecule or a peptide. In a preferred embodiment, the binding partner is an antibody, or a fragment thereof. Such antibodies may be polyclonal, or monoclonal. In addition, the antibodies may be single chain, chimeric, CDR-grafted or humanized. Antibodies may be prepared by the methods described herein and by other methods well known to those of skill in the art.

There are a variety of assay formats known to those of ordinary skill in the art for using a binding partner to detect polypeptide markers in a sample. *See, e.g.*, Harlow and Lane, *Antibodies: A Laboratory Manual*, Cold Spring Harbor Laboratory, 1988. In a preferred embodiment, the assay involves the use of binding partner immobilized on a solid support to bind to and remove the polypeptide from the remainder of the sample. The bound polypeptide may then be detected using a second binding partner that contains a reporter group. Suitable second binding partners include antibodies that bind to the binding partner/polypeptide complex. Alternatively, a competitive assay may be utilized, in which a polypeptide is labeled with a reporter group and allowed to bind to the immobilized binding partner after incubation of the binding partner with the sample. The extent to which components of the sample inhibit the binding of the labeled polypeptide to the binding partner is indicative of the reactivity of the sample with the immobilized binding partner.

The solid support may be any material known to those of ordinary skill in the art to which the antigen may be attached. For example, the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681. The binding agent may be immobilized on the solid support using a variety of techniques known to those of skill in the art, which are amply described in the patent and scientific literature. In the context of the present invention, the term "immobilization" refers to both noncovalent association, such as adsorption, and covalent attachment (which may be a direct linkage between the antigen and functional groups on the support or may be a linkage by way of a cross-linking agent). Immobilization by adsorption to a well in a microtiter plate or to a membrane is preferred. In such cases, adsorption may be achieved by contacting the binding agent, in a suitable buffer, with the solid support for a suitable amount of time. The contact time varies with temperature, but is typically between about 1 hour and about 1 day. In general, connecting a well of a plastic microtiter plate (such as polystyrene or polyvinylchloride) with an amount of binding agent ranging from about 10 ng to about 10 µg, and preferably about 100 ng to about 1 µg, is sufficient to immobilize an adequate amount of binding agent.

Covalent attachment of binding agent to a solid support may generally be achieved by first reacting the support with a bifunctional reagent that will react with both the support and a functional group, such as a hydroxyl or amino group, on the binding agent. For example, the binding agent may be covalently attached to supports having an appropriate polymer coating using benzoquinone or by condensation of an aldehyde group on the support with an amine and an active hydrogen on the binding partner (*see, e.g.*, Pierce Immunotechnology Catalog and Handbook, 1991, at A12-A13).

In certain embodiments, the assay is a two-antibody sandwich assay. This assay may be performed by first contacting an antibody that has been immobilized on a solid support, commonly the well of a microtiter plate, with the sample, such that polypeptides within the sample are allowed to bind to the immobilized antibody. Unbound sample is then removed from the immobilized polypeptide-antibody complexes and a second antibody (containing a reporter group) capable of binding to a different site on the polypeptide is added. The amount of second antibody that remains bound to the solid support is then determined using a method appropriate for the specific reporter group.

More specifically, once the antibody is immobilized on the support as described above, the remaining protein binding sites on the support are typically blocked. Any suitable blocking agent known to those of ordinary skill in the art, such as bovine serum albumin or Tween 20^{™} (Sigma Chemical Co., St. Louis, MO). The immobilized antibody is then incubated with the sample, and polypeptide is allowed to bind to the antibody. The sample may be diluted with a suitable diluent, such as phosphate-buffered saline (PBS) prior to incubation. In general, an appropriate contact time (*i.e.,* incubation time) is that period of time that is sufficient to detect the presence of polypeptide within a sample obtained from an individual with lung cancer. Preferably, the contact time is sufficient to achieve a level of binding that is at least about 95% of that achieved at equilibrium between bound and unbound polypeptide. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1% Tween 20^{™}. The second antibody, which contains a reporter group, may then be added to the solid support. Preferred reporter groups include enzymes (such as horseradish peroxidase), substrates, cofactors, inhibitors, dyes, radionuclides, luminescent groups, fluorescent groups and biotin. The conjugation of antibody to reporter group may be achieved using standard methods known to those of ordinary skill in the art.

The second antibody is then incubated with the immobilized antibody-polypeptide complex for an amount of time sufficient to detect the bound polypeptide. An appropriate amount of time may generally be determined by assaying the level of binding that occurs over a period of time. Unbound second antibody is then removed and bound second antibody is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of lung cancer, the signal detected from the reporter group that remains bound to the solid support is generally compared to a signal that corresponds to a predetermined cut-off value. In one preferred embodiment, the cut-off value is the average mean signal obtained when the immobilized antibody is incubated with samples from patients without lung cancer. In general, a sample generating a signal that is three standard deviations above the predetermined cut-off value is considered positive for lung cancer. In an alternate preferred embodiment, the cut-off value is determined using a Receiver Operator Curve, according to the method of Sackett et al., *Clinical Epidemiology: A Basic Science for Clinical Medicine,* Little Brown and Co., 1985, p. 106-7. Briefly, in this embodiment, the cut-off value may be determined from a plot of pairs of true positive rates (*i.e.*, sensitivity) and false positive rates (100%-specificity) that correspond to each possible cut-off value for the diagnostic test result. The cut-off value on the plot that is the closest to the upper left-hand corner (*i.e.*, the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for lung cancer.

In a related embodiment, the assay is performed in a flow-through or strip test format, wherein the antibody is immobilized on a membrane, such as nitrocellulose. In the flow-through test, polypeptides within the sample bind to the immobilized antibody as the sample passes through the membrane. A second, labeled antibody then binds to the antibody-polypeptide complex as a solution containing the second antibody flows through the membrane. The detection of bound second antibody may then be performed as described above. In the strip test format, one end of the membrane to which antibody is bound is immersed in a solution containing the sample. The sample migrates along the membrane through a region containing second antibody and to the area of immobilized antibody. Concentration of second antibody at the area of immobilized antibody indicates the presence of lung cancer. Typically, the concentration of second antibody at that site generates a pattern, such as a line, that can be read visually. The absence of such a pattern indicates a negative result. In general, the amount of antibody immobilized on the membrane is selected to generate a visually discernible pattern when the biological sample contains a level of polypeptide that would be sufficient to generate a positive signal in the two-antibody sandwich assay, in the format discussed above. Preferably, the amount of antibody immobilized on the membrane ranges from about 25 ng to about 1 µg, and more preferably from about 50 ng to about 500 ng. Such tests can typically be performed with a very small amount of biological sample.

Of course, numerous other assay protocols exist that are suitable for use with the antigens or antibodies of the present invention. The above descriptions are intended to be exemplary only.

In another embodiment, the above polypeptides may be used as markers for the progression of lung cancer. In this embodiment, assays as described above for the diagnosis of lung cancer may be performed over time, and the change in the level of reactive polypeptide(s) evaluated. For example, the assays may be performed every 24-72 hours for a period of 6 months to 1 year, and thereafter performed as needed. In general, lung cancer is progressing in those patients in whom the level of polypeptide detected by the binding agent increases over time. In contrast, lung cancer is not progressing when the level of reactive polypeptide either remains constant or decreases with time.

Antibodies for use in the above methods may be prepared by any of a variety of techniques known to those of ordinary skill in the art. *See, e.g.,* Harlow and Lane, *Antibodies: A Laboratory Manual,* Cold Spring Harbor Laboratory, 1988. In one such technique, an immunogen comprising the antigenic polypeptide is initially injected into any of a wide variety of mammals (*e.g.*, mice, rats, rabbits, sheep and goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for the antigenic polypeptide of interest may be prepared, for example, using the technique of Kohler and Milstein, *Eur. J. Immunol. 6*:511-519, 1976, and improvements thereto. Briefly, these methods involve the preparation of immortal cell lines capable of producing antibodies having the desired specificity (*i.e*., reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides of this invention may be used in the purification process in, for example, an affinity chromatography step.

Monoclonal antibodies of the present invention may also be used as therapeutic reagents, to diminish or eliminate lung tumors. The antibodies may be used on their own (for instance, to inhibit metastases) or coupled to one or more therapeutic agents. Suitable agents in this regard include radionuclides, differentiation inducers, drugs, toxins, and derivatives thereof. Preferred radionuclides include ⁹⁰Y, ¹²³I, ¹²⁵I, ¹³¹I, ¹⁸⁶Re, ¹⁸⁸Re, ²¹¹At, and ²¹²Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin. Shigella toxin, and pokeweed antiviral protein.

A therapeutic agent may be coupled (*e.g.*, covalently bonded) to a suitable monoclonal antibody either directly or indirectly (*e.g.*, via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (*e.g*., a halide) on the other.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

It will be evident to those skilled in the art that a variety of bifunctional or polyfunctional reagents, both homo- and hetero-functional (such as those described in the catalog of the Pierce Chemical Co., Rockford, IL), may be employed as the linker group. Coupling may be effected, for example, through amino groups, carboxyl groups, sulfhydryl groups or oxidized carbohydrate residues. There are numerous references describing such methodology, *e.g.,* U.S. Patent No. 4,671,958, to Rodwell et al.

Where a therapeutic agent is more potent when free from the antibody portion of the immunoconjugates of the present invention, it may be desirable to use a linker group which is cleavable during or upon internalization into a cell. A number of different cleavable linker groups have been described. The mechanisms for the intracellular release of an agent from these linker groups include cleavage by reduction of a disulfide bond *(e.g.,* U.S. Patent No. 4,489,710, to Spitler), by irradiation of a photolabile bond (*e.g*., U.S. Patent No. 4,625,014, to Senter et al.), by hydrolysis of derivatized amino acid side chains (*e.g.*, U.S. Patent No. 4,638,045, to Kohn et al.), by serum complement-mediated hydrolysis (*e.g*., U.S. Patent No. 4,671,958, to Rodwell et al.), and acid-catalyzed hydrolysis *(e.g.,* U.S. Patent No. 4,569,789, to Blattler et al.).

It may be desirable to couple more than one agent to an antibody. In one embodiment, multiple molecules of an agent are coupled to one antibody molecule. In another embodiment, more than one type of agent may be coupled to one antibody. Regardless of the particular embodiment, immunoconjugates with more than one agent may be prepared in a variety of ways. For example, more than one agent may be coupled directly to an antibody molecule, or linkers which provide multiple sites for attachment can be used. Alternatively, a carrier can be used.

A carrier may bear the agents in a variety of ways, including covalent bonding either directly or via a linker group. Suitable carriers include proteins such as albumins (*e.g.*, U.S. Patent No. 4,507,234, to Kato et al.), peptides and polysaccharides such as aminodextran (*e.g.*, U.S. Patent No. 4,699,784, to Shih et al.). A carrier may also bear an agent by noncovalent bonding or by encapsulation, such as within a liposome vesicle (*e.g*., U.S. Patent Nos. 4,429,008 and 4,873,088). Carriers specific for radionuclide agents include radiohalogenated small molecules and chelating compounds. For example, U.S. Patent No. 4, 735, 792 discloses representative radiohalogenated small molecules and their synthesis. A radionuclide chelate may be formed from chelating compounds that include those containing nitrogen and sulfur atoms as the donor atoms for binding the metal, or metal oxide, radionuclide. For example, U.S. Patent No. 4,673,562, to Davison et al. discloses representative chelating compounds and their synthesis.

A variety of routes of administration for the antibodies and immunoconjugates may be used. Typically, administration will be intravenous, intramuscular, subcutaneous or in the bed of a resected tumor. It will be evident that the precise dose of the antibody/immunoconjugate will vary depending upon the antibody used, the antigen density on the tumor, and the rate of clearance of the antibody.

Diagnostic reagents of the present invention may also comprise polynucleotide sequences encoding one or more of the above polypeptides, or one or more portions thereof. For example, at least two oligonucleotide primers may be employed in a polymerase chain reaction (PCR) based assay to amplify lung tumor-specific cDNA derived from a biological sample, wherein at least one of the oligonucleotide primers is specific for a polynucleotide molecule encoding a lung tumor protein of the present invention. The presence of the amplified cDNA is then detected using techniques well known in the art, such as gel electrophoresis. Similarly, oligonucleotide probes specific for a polynucleotide molecule encoding a lung tumor protein of the present invention may be used in a hybridization assay to detect the presence of an inventive polypeptide in a biological sample.

As used herein, the term "oligonucleotide primer/probe specific for a polynucleotide molecule" means an oligonucleotide sequence that has at least about 60%, preferably at least about 75% and more preferably at least about 90%, identity to the polynucleotide molecule in question. Oligonucleotide primers and/or probes which may be usefully employed in the inventive diagnostic methods preferably have at least about 10-40 nucleotides. In a preferred embodiment, the oligonucleotide primers comprise at least about 10 contiguous nucleotides of a polynucleotide molecule comprising sequence selected from SEQ ID NO: 1-109, 111, 113 115-151, 153, 154, 157, 158, 160, 162-164, 167, 168 and 171. Preferably, oligonucleotide probes for use in the inventive diagnostic methods comprise at least about 15 contiguous oligonucleotides of a polynucleotide molecule comprising a sequence provided in SEQ ID NO: 1-109,111, 113 115-151, 153, 154, 157, 158, 160, 162-164, 167, 168 and 171. Techniques for both PCR based assays and hybridization assays are well known in the art (see, for example, Mullis *et al. Ibid;* Ehrlich, *Ibid*). Primers or probes may thus be used to detect lung tumor-specific sequences in biological samples, including blood, semen, lung tissue and/or lung tumor tissue.

The following Examples are offered by way of illustration and not by way of limitation.

### EXAMPLES

### Example 1

### ISOLATION AND CHARACTERIZATION OF cDNA SEQUENCES ENCODING LUNG TUMOR POLYPEPTIDES

This example illustrates the isolation of cDNA molecules encoding lung tumor-specific polypeptides from lung tumor cDNA libraries.

### A. Isolation of cDNA Sequences from a Lung Squamous Cell Carcinoma Library

A human lung squamous cell carcinoma cDNA expression library was constructed from poly A⁺ RNA from a pool of two patient tissues using a Superscript Plasmid System for cDNA Synthesis and Plasmid Cloning kit (BRL Life Technologies, Gaithersburg, MD) following the manufacturer's protocol. Specifically, lung carcinoma tissues were homogenized with polytron (Kinematica, Switzerland) and total RNA was extracted using Trizol reagent (BRL Life Technologies) as directed by the manufacturer. The poly A⁺ RNA was then purified using an oligo dT cellulose column as described in Sambrook et al., *Molecular Cloning: A Laboratory Manual,* Cold Spring Harbor Laboratories, Cold Spring Harbor, NY, 1989. First-strand cDNA was synthesized using the NotI/Oligo-dT18 primer. Double-stranded cDNA was synthesized, ligated with BstXI/EcoRI adaptors (Invitrogen, San Diego, CA) and digested with NotI. Following size fractionation with cDNA size fractionation columns (BRL Life Technologies), the cDNA was ligated into the BstXI/NotI site of pcDNA3.1 (Invitrogen) and transformed into ElectroMax *E. coli* DH10B cells (BRL Life Technologies) by electroporation.

Using the same procedure, a normal human lung cDNA expression library was prepared from a pool of four tissue specimens. The cDNA libraries were characterized by determining the number of independent colonies, the percentage of clones that carried insert, the average insert size and by sequence analysis. The lung squamous cell carcinoma library contained 2.7 x 10⁶ independent colonies, with 100% of clones having an insert and the average insert size being 2100 base pairs. The normal lung cDNA library contained 1.4 x 10⁶ independent colonies, with 90% of clones having inserts and the average insert size being 1800 base pairs. For both libraries, sequence analysis showed that the majority of clones had a full length cDNA sequence and were synthesized from mRNA

cDNA library subtraction was performed using the above lung squamous cell carcinoma and normal lung cDNA libraries, as described by Hara *et al.* (*Blood, 84*:189-199, 1994) with some modifications. Specifically, a lung squamous cell carcinoma-specific subtracted cDNA library was generated as follows. Normal tissue cDNA library (80 µg) was digested with BamHI and XhoI, followed by a filling-in reaction with DNA polymerase Klenow fragment. After phenol-chloroform extraction and ethanol precipitation, the DNA was dissolved in 133 µl of H₂O, heat-denatured and mixed 133 µl (133 µg) of Photoprobe biotin (Vector Laboratories, Burlingame, CA). As recommended by the manufacturer, the resulting mixture was irradiated with a 270 W sunlamp on ice for 20 minutes. Additional Photoprobe biotin (67 µl) was added and the biotinylation reaction was repeated. After extraction with butanol five times, the DNA was ethanol-precipitated and dissolved in 23 µl H₂O to form the driver DNA.

To form the tracer DNA, 10 µg lung squamous cell carcinoma cDNA library was digested with NotI and Spel, phenol chloroform extracted and passed through Chroma spin-400 columns (Clontech, Palo Alto, CA). Typically, 5 pg of cDNA was recovered after the sizing column. Following ethanol precipitation, the tracer DNA was dissolved in 5 µl H₂O. Tracer DNA was mixed with 15 µl driver DNA and 20 µl of 2 x hybridization buffer (1.5 M NaCl/10 mM EDTA/50 mM HEPES pH 7.5/0.2% sodium dodecyl sulfate), overlaid with mineral oil, and heat-denatured completely. The sample was immediately transferred into a 68 °C water bath and incubated for 20 hours (long hybridization [LH]). The reaction mixture was then subjected to a streptavidin treatment followed by phenol/chloroform extraction. This process was repeated three more times. Subtracted DNA was precipitated, dissolved in 12 µl H₂O, mixed with 8 µl driver DNA and 20 µl of 2 x hybridization buffer, and subjected to a hybridization at 68 °C for 2 hours (short hybridization [SH]). After removal of biotinylated double-stranded DNA, subtracted cDNA was ligated into NotI/SpeI site of chloramphenicol resistant pBCSK⁺ (Stratagene, La Jolla, CA) and transformed into ElectroMax *E. coli* DH10B cells by electroporation to generate a lung squamous cell carcinoma specific subtracted cDNA library (herein after referred to as "lung subtraction I").

A second lung squamous cell carcinoma specific subtracted cDNA library (referred to as "lung subtraction II'') was generated in a similar way to the lung subtraction library I. except that eight frequently recovered genes from lung subtraction I were included in the driver DNA, and 24,000 independent clones were recovered.

To analyze the subtracted cDNA libraries, plasmid DNA was prepared from 320 independent clones, randomly picked from the subtracted lung squamous cell carcinoma specific libraries. Representative cDNA clones were further characterized by DNA sequencing with a Perkin Elmer/Applied Biosystems Division Automated Sequencer Model 373A and/or Model 377 (Foster City, CA). The cDNA sequences for sixty isolated clones are provided in SEQ ID NO: 1-60. These sequences were compared to known sequences in the gene bank using the EMBL and GenBank, databases (release 96). No significant homologies were found to the sequences provided in SEQ ID NO: 2, 3, 19, 38 and 46. The sequences of SEQ ID NO: 1, 6-8, 10-13, 15, 17, 18, 20-27, 29, 30, 32, 34-37, 39-45, 47-49, 51, 52, 54, 55 and 57-59 were found to show some homology to previously identified expressed sequence tags (ESTs). The sequences of SEQ ID NO: 9, 28, 31 and 33 were found to show some homology to previously identified non-human gene sequences and the sequences of SEQ ID NO: 4, 5, 14, 50, 53, 56 and 60 were found to show some homology to gene sequences previously identified in humans.

The subtraction procedure described above was repeated using the above lung squamous cell carcinoma cDNA library as the tracer DNA, and the above normal lung tissue cDNA library and a cDNA library from normal liver and heart (constructed from a pool of one sample of each tissue as described above), plus twenty other cDNA clones that were frequently recovered in lung subtractions I and II, as the driver DNA (lung subtraction III). The normal liver and heart cDNA library contained 1.76 x 10⁶ independent colonies, with 100% of clones having inserts and the average insert size being 1600 base pairs. Ten additional clones were isolated (SEQ ID NO: 61-70). Comparison of these cDNA sequences with those in the gene bank as described above, revealed no significant homologies to the sequences provided in SEQ ID NO: 62 and 67. The sequences of SEQ ID NO: 61, 63-66, 68 and 69 were found to show some homology to previously isolated ESTs and the sequence provided in SEQ ID NO: 70 was found to show some homology to a previously identified rat gene.

### B. Isolation of cDNA Sequences from a Lung Adenocarcinoma Library

A human lung adenocarcinoma cDNA expression library was constructed as described above. The library contained 3.2 x 10⁶ independent colonies, with 100% of clones having an insert and the average insert size being 1500 base pairs. Library subtraction was performed as described above using the normal lung and normal liver and heart cDNA expression libraries described above as the driver DNA. Twenty-six hundred independent clones were recovered.

Initial cDNA sequence analysis from 100 independent clones revealed many ribosomal protein genes. The cDNA sequences for fifteen clones isolated in this subtraction are provided in SEQ ID NO: 71-86. Comparison of these sequences with those in the gene bank as described above revealed no significant homologies to the sequence provided in SEQ ID NO: 84. The sequences of SEQ ID NO: 71, 73, 74, 77, 78 and 80-82 were found to show some homology to previously isolated ESTs, and the sequences of SEQ ID NO: 72, 75, 76, 79, 83 and 85 were found to show some homology to previously identified human genes.

### Example 2

### DETERMINATION OF TISSUE SPECIFICITY OF LUNG TUMOR POLYPEPTIDES

Using gene specific primers, mRNA expression levels for seven representative lung tumor polypeptides described in Example 1 were examined in a variety of normal and tumor tissues using RT-PCR.

Briefly, total RNA was extracted from a variety of normal and tumor tissues using Trizol reagent as described above. First strand synthesis was carried out using 2 µg of total RNA with SuperScript II reverse transcriptase (BRL Life Technologies) at 42 °C for one hour. The cDNA was then amplified by PCR with gene-specific primers. To ensure the semi-quantitative nature of the RT-PCR, β-actin was used as an internal control for each of the tissues examined. 1 µl of 1:30 dilution of cDNA was employed to enable the linear range amplification of the β-actin template and was sensitive enough to reflect the differences in the initial copy numbers. Using these conditions, the β-actin levels were determined for each reverse transcription reaction from each tissue. DNA contamination was minimized by DNase treatment and by assuring a negative PCR result when using first strand cDNA that was prepared without adding reverse transcriptase.

mRNA Expression levels were examined in five different types of tumor tissue (lung squamous cell carcinoma from 3 patients, lung adenocarcinoma, colon tumor from 2 patients, breast tumor and prostate tumor), and thirteen different normal tissues (lung from 4 donors, prostate, brain, kidney, liver, ovary, skeletal muscle, skin, small intestine, stomach, myocardium, retina and testes). Using a 10-fold amount of cDNA, the antigen LST-S1-90 (SEQ ID NO: 3) was found to be expressed at high levels in lung squamous cell carcinoma and in breast tumor, and at low to undetectable levels in the other tissues examined.

The antigen LST-S2-68 (SEQ ID NO: 15) appears to be specific to lung and breast tumor, however, expression was also detected in normal kidney. Antigens LST-S1-169 (SEQ ID NO: 6) and LST-S1-133 (SEQ ID NO: 5) appear to be very abundant in lung tissues (both normal and tumor), with the expression of these two genes being decreased in most of the normal tissues tested. Both LST-S1-169 and LST-S1-133 were also expressed in breast and colon tumors. Antigens. LST-S1-6 (SEQ ID NO: 7) and LST-S2-I2-5F (SEQ ID NO: 47) did not show tumor or tissue specific expression, with the expression of LST-S 1-28 being rare and only detectable in a few tissues. The antigen LST-S3-7 (SEQ ID NO: 63) showed lung and breast tumor specific expression, with its message only being detected in normal testes when the PCR was performed for 30 cycles. Lower level expression was detected in some normal tissues when the cycle number was increased to 35. Antigen LST-S3-13 (SEQ ID NO: 66) was found to be expressed in 3 out of 4 lung tumors, one breast tumor and both colon tumor samples. Its expression in normal tissues was lower compared to tumors, and was only detected in 1 out of 4 normal lung tissues and in normal tissues from kidney, ovary and retina. Expression of antigens LST-S3-4 (SEQ ID NO: 62) and LST-S3-14 (SEQ ID NO: 67) was rare and did not show any tissue or tumor specificity. Consistent with Northern blot analyses, the RT-PCT results on antigen LAT-S1-A-10A (SEQ ID NO: 78) suggested that its expression is high in lung, colon, stomach and small intestine tissues, including lung and colon tumors, whereas its expression was low or undetectable in other tissues.

A total of 2002 cDNA fragments isolated in lung subtractions I, II and III, described above, were colony PCR amplified and their mRNA expression levels in lung tumor, normal lung, and various other normal and tumor tissues were determined using microarray technology (Synteni, Palo Alto, CA). Briefly, the PCR amplification products were dotted onto slides in an array format, with each product occupying a unique location in the array. mRNA was extracted from the tissue sample to be tested, reverse transcribed, and fluorescent-labeled cDNA probes were generated. The microarrays were probed with the labeled cDNA probes, the slides scanned and fluorescence intensity was measured. This intensity correlates with the hybridization intensity. Seventeen non-redundant cDNA clones showed over-expression in lung squamous tumors, with expression in normal tissues tested (lung, skin, lymph node, colon, liver, pancreas, breast, heart, bone marrow, large intestine, kidney, stomach, brain, small intestine, bladder and salivary gland) being either undetectable, or 10-fold less compared to lung squamous tumors. The determined partial cDNA sequences for the clone L513S are provided in SEQ ID NO: 87 and 88; those for L514S are provided in SEQ ID NO: 89 and 90; those for L516S in SEQ ID NO: 91 and 92; that for L517S in SEQ ID NO: 93; that for L519S in SEQ ID NO: 94; those for L520S in SEQ ID NO: 95 and 96; those for L521S in SEQ ID NO: 97 and 98; that for L522S in SEQ ID NO: 99; that for L523S in SEQ ID NO: 100: that for L524S in SEQ ID NO: 101; that for L52SS in SEQ ID NO: 102; that for L526S in SEQ ID NO: 103: that for L527S in SEQ ID NO: 104; that for L528S in SEQ ID NO: 105; that for L529S in SEQ ID NO: 106; and those for L530S in SEQ ID NO: 107 and 108. Additionally, the full-length cDNA sequences for L503S and L514S (variants 1 and 2), are provided in SEQ ID NO: 151, 153 and 154, respectively, with the corresponding predicted amino acid sequence being provided in SEQ ID NO: 152. 155 and 156. Due to polymorphisms, the clone L531S appears to have two forms. A first determined full-length cDNA sequence for L531S is provided in SEQ ID NO: 109, with the corresponding predicted amino acid sequence being provided in SEQ ID NO: 110. A second determined full-length cDNA sequence for L531S is provided in SEQ ID NO: 111, with the corresponding predicted amino acid sequence being provided in SEQ ID NO: 112. The sequence of SEQ ID NO: 111 is identical to that of SEQ ID NO: 109, except that it contains a 27 bp insertion. Similarly, L514S also has two alternatively spliced forms; the first variant cDNA is listed as SEQ ID NO: 153, with the corresponding amino acid sequence as SEQ ID NO: 155. The second variant form of L514S full-length cDNA is referred to as SEQ ID NO: 154, with its corresponding amino acid sequence as SEQ ID NO: 156.

Full length cloning for L524S (SEQ ID NO: 101) yielded two variants (SEQ ID NO: 163 and 164) with the corresponding predicted amino acid sequences (SEQ ID NO: 165 and 166), respectively. Both variants have been shown to encode parathyroid hormone-related peptide.

Comparison of the sequences of L514S and L531S (SEQ ID NO: 87 and 88, 89 and 90, and 109, respectively) with those in the gene bank, as described above, revealed no significant homologies to known sequences. The sequences of L513S, L516S, L517S, L519S, L520S and L530S (SEQ ID NO: 87 and 88, 91 and 92, 93, 94, 95 and 96, 107 and 108, respectively) were found to show some homology to previously identified ESTs. The sequences of L521 S, L522S, L523S, L524S, L525S, L526S, L527S, L528S and L529S (SEQ ID NO: 97 and 98, 99, 99, 101, 102, 103, 104, 105, and 106, respectively) were found to represent known genes. The determined full-length cDNA sequences for L520S is provided in SEQ ID NO: 113, with the corresponding predicted amino acid sequence being provided in SEQ ID NO: 114. Subsequent microarray analysis has shown L520S to be overexpressed in breast tumors in addition to lung squamous tumors.

Further analysis has demonstrated L529S (SEQ ID NO: 106 and 115), L525S (SEQ ID NO: 102 and 120) and L527S (SEQ ID NO: 104) are cytosleletal components and potentially squamous cell specific proteins. L529S is connexin 26, a gap junction protein. It is highly expressed in lung squamous tumor 9688T, and moderately over-expressed in two others. However, lower level expression of connexin 26 is also detectable in normal skin, colon, liver and stomach. The over-expression of connexin 26 in some breast tumors has been reported and a mutated form of L529S may result in over-expression in lung tumors. L525S is plakophilin 1, a desmosomal protein found in plaque-bearing adhering junctions of the skin. Expression levels for L525S mRNA is highly elevated in three out of four lung squamous tumors tested, and in normal skin. L527S has been identified as keratin 6 isoform, type II 58 Kd keratin, and cytokeratin 13 and shows over-expression in squamous tumors and low expression in normal skin, breast and colon tissues. Notably, keratin and keratin-related genes have been extensively documented as potential markers for lung cancer including CYFRA2.1 (Pastor, A., et al, *Eur. Respir. J.,* 10:603-609, 1997). L513S (SEQ ID NO: 87 and 88) shows moderate over-expression in several tumor tissues tested, and encodes a protein that was first isolated as a pemphigus vulgaris antigen.

L520S (SEQ ID NO: 95 and 96) and L521S (SEQ ID NO: 97 and 98) are highly expressed in lung squamous tumors, and L520S is up-regulated in normal salivary gland and L521S is over-expressed in normal skin. Both belong to a family of small proline rich proteins and represent markers for fully differentiated squamous cells.. L521S has been described as a specific marker for lung squamous tumor (Hu, R, et al, *Lung Cancer,* 20:25-30, 1998). L515S (SEQ ID NO: 162) encodes IGF-β2 and L516S is an aldose reductase homologue and both are moderately expressed in lung squamous tumors and in normal colon. Notably, L516S (SEQ ID NO: 91 and 92) is up-regulated in metastatic tumors but not primary lung adenocarcinoma., an indication of its potential role in metatasis and a potential prognostic marker. L522S (SEQ ID NO: 99) is moderately over-expressed in lung squamous tumors with minimum expression in normal tissues. L522S has been shown to belong to a class IV alcohol dehydrogenase, ADH7, and its expression profile suggests it is a squamous cell specific antigen. L523S (SEQ ID NO: 100) is moderately over-expressed in lung squamous tumor, human pancreatic cancer cell lines and pancreatic cancer tissues, suggesting this gene may be a shared antigen between pancreatic and lung squamous cell cancer.

L524S (SEQ ID NO: 101) is over-expressed in the majority of squamous tumors tested and is homolgous with parathyroid hormone-related peptide (PTHrP), which is best known to cause humoral hypercalcaemia associated with malignant tumors such as leukemia, prostate and breast cancer. It is also believed that PTHrP is most commonly associated with squamous carcinoma of lung and rarely with lung adenocarcinoma (Davidson, L.A., et al, *J. Pathol.,* 178: 398-401, 1996). L528S (SEQ ID NO: 105) is highly over-expressed in two lung squamous tumors with moderate expression in two other squamous tumors, one lung adenocarcinoma and some normal tissues, including skin, lymph nodes, heart, stomach and lung. It encodes the NMB gene that is similar to the precursor of melanocyte specific gene Pmel17, wfhich is reported to be preferentially expressed in low-metastatic potential melanoma cell lines. This suggests that L528S may be a shared antigen in both melanoma and lung aquamous cell carcinoma. L526S (SEQ ID NO: 103) is overexpressed in all lung squamous cell tumor tissues tested and has been shown to share homology with a gene (ATM) in which a mutation causes ataxia telangiectasia, a genetic disorder in humans causing a predisposition to cancer, among other symptoms. ATM encodes a protein that activates p53 mediated cell-cycle checkpoint through direct binding and phosphorylation of the p53 molecule. Approximately 40% of lung cancer is associated with p53 mutations, and it is speculated that over-expression of ATM is a result of compensation for loss of p53 function, but it is unknown whether over-expression is the cause of result of lung squamous cell carcinoma. Additionally, expression of L526S (ATM) is also detected in a metastatic but not lung adenocarcinoma, suggesting a role in metastasis.

### Example 3

### ISOLATION AND CHARACTERIZATION OF LUNG TUMOR POLYPEPTIDES BY PCR-BASED SUBTRACTION

Eight hundred and fifty seven clones from a cDNA subtraction library, containing cDNA from a pool of two human lung squamous tumors subtracted against eight normal human tissue cDNAs including lung, PBMC, brain, heart, kidney, liver, pancreas, and skin, (Clontech, Palo Alto, CA) were derived and submitted to a first round of PCR amplification. This library was subjected to a second round of PCR amplification, following the manufacturer's protocol. The resulting cDNA fragments were subcloned into the vector P7- Adv vector (Clontech, Palo Alto, CA) and transformed into DH5α *E. coli* (Gibco, BRL). DNA was isolated from independent clones and sequenced using a Perkin Elmer/Applied Biosystems Division Automated Sequencer Model 373A.

One hundred and sixty two positive clones were sequenced. Comparison of the DNA sequences of these clones with those in the gene bank using the EMBL and GenBank databases, as described above, revealed no significant homologies to 13 of these clones, hereinafter referred to as Contig 13, 16, 17, 19, 22, 24, 29, 47, 49, 56-59 . The determined cDNA sequences for these clones are provided in SEQ ID NO: 125, 127-129, 131-133, 142, 144, 148-150, and 157, respectively. Contigs 1, 3-5, 7-10, 12, 11, 15, 20, 31, 33, 38, 39, 41, 43, 44, 45, 48, 50, 53, 54 (SEQ ID NO: 115-124, 126, 130, 134-141, 143, 145-147, respectively) were found to show some degree of homology to previously identified DNA sequences. Contig 57 (SEQ ID NO: 149) was found to represent the clone L519S (SEQ ID NO: 94) disclosed in US. Patent Application No. 09/123,912, filed July 27, 1998. To the best of the inventors' knowledge, none of these sequences have been previously shown to be differentially over-expressed in lung tumors.

mRNA expression levels for representative clones in lung tumor tissues, normal lung tissues (n=4), resting PBMC, salivary gland, heart, stomach, lymph nodes, skeletal muscle, soft palate, small intestine, large intestine, bronchial, bladder, tonsil, kidney, esophagus, bone marrow, colon, adrenal gland, pancreas, and skin, (all derived from human) were determined by RT-PCR as described above. Expression levels using microarray technology, as described above, were examined in one sample of each tissue type unless otherwise indicated.

Contig 3 (SEQ ID NO: 116) was found to be highly expressed in all head and neck squamous cell tumors tested (17/17), and expressed in the majority (8/12) of lung squamous tumors, (high expression in 7/12, moderate in 2/12, and low in 2/12), while showing negative expression for 2/4 normal lung tissues and low expression in the remaining two samples. Contig 3 showed moderate expression in skin and soft palate, and lowered expression levels in resting PBMC, large intestine, salivary gland, tonsil, pancreas, esophagus, and colon. Contig 11 (SEQ ID NO: 124) was found to be expressed in all head and neck squamous cell tumors tested (17/17): highly expressed in 14/17, and moderately expressed in 3/17. Additionally, expression in lung squamous tumors showed high expression in 3/12 and moderate in 4/12. Contig 11 was negative for 3/4 normal lung samples, with the remaining sample having only low expression. Contig 11 showed low to moderate reactivity to salivary gland, soft palate, bladder, tonsil, skin, esophagus, and large intestine. Contig 13 (SEQ ID NO: 125) was found to be expressed in all head and neck squamous cell tumors tested (17/17): highly expressed in 12/17, and moderately expressed in 5/17. Contig 13 was expressed in 7/12 lung squamous tumors, with high expression in 4/12 and moderate expression in three samples. Analysis of normal lung samples showed negative expression for 2/4 and low to moderate expression in the remaining two samples. Contig 13 did show low to moderate reactivity to resting PBMC, salivary gland, bladder, pancreas, tonsil, skin, esophagus, and large intestine, as well as high expression in soft palate. Contig 16 (SEQ ID NO: 127) was found to be moderately expressed in some head and neck squamous cell tumors (6/17) and one lung squamous tumor; while showing no expression in any normal lung, samples tested. Contig 16 did show low reactivity to resting PBMC, large intestine, skin, salivary gland, and soft palate. Contig 17 (SEQ ID NO: 128) was shown to be expressed in all head and neck squamous cell tumors tested (17/17): highly expressed in 5/17, and moderately expressed in 12/17. Expression levels in lung squamous tumors showed one tumor sample with high expression and 3/12 with moderate levels. Contig 17 was negative for 2/4 normal lung samples, with the remaining samples having only low expression. Additionally, low level expression was found in esophagus and soft palate. Contig 19 (SEQ ID NO: 129) was found to be expressed in most head and neck squamous cell tumors tested (11/17): with two samples having high levels, 6/17 showing moderate expression, and low expression being found in 3/17. Testing in lung squamous tumors revealed only moderate expression in 3/12 samples. Expression levels in 2/4 of normal lung samples were negative, the two other samples having only low expression. Contig 19 did show low expression levels in esophagus, resting PBMC, salivary gland, bladder, soft palate, and pancreas.

Contig 22, (SEQ ID NO: 131) was shown to be expressed in most head and neck squamous cell tumors tested (13/17) with high expression in four of these samples, moderate expression in 6/17, and low expression in 3/17. Expression levels in lung squamous tumors were found to be moderate to high for 3/12 tissues tested, with negative expression in two normal lung samples and low expression in two other samples (n=4). Contig 22 did show low expression in skin, salivary gland and soft palate. Similarly, Contig 24 (SEQ ID NO: 132) was found to be expressed in most head and neck squamous cell tumors tested (13/17) with high expression in three of these samples, moderate expression in 6/17, and low expression in 4/17. Expression levels in lung squamous tumors were found to be moderate to high for 3/12 tissues tested, with negative expression for three normal lung samples and low expression in one sample (n=4). Contig 24 did show low expression in skin, salivary gland and soft palate. Contig 29 (SEQ ID NO: 133) was expressed in nearly all head and neck squamous cell tumors tested (16/17): highly expressed in 4/17, moderately expressed in 11/17, with low expression in one sample. Also, it was moderately expressed in 3/12 lung squamous tumors, while being negative for 2/4 normal lung samples. Contig 29 showed low to moderate expression in large intestine, skin, salivary gland, pancreas, tonsil, heart and soft palate. Contig 47 (SEQ ID NO: 142) was expressed in most head and neck squamous cell tumors tested (12/17): moderate expression in 10/17, and low expression two samples. In lung squamous tumors, it was highly expressed in one sample and moderately expressed in two others (n=13). Contig 47 was negative for 2/4 normal lung samples, with the remaining two samples having moderate expression. Also, Contig 47 showed moderate expression in large intestine, and pancreas, and low expression in skin, salivary gland, soft palate, stomach, bladder, resting PBMC, and tonsil.

Contig 48 (SEQ ID NO: 143) was expressed in all head and neck squamous cell tumors tested (17/17): highly expressed in 8/17 and moderately expressed in 7/17, with low expression in two samples. Expression levels in lung squamous tumors were high to moderate in three samples (n=13). Contig 48 was negative for one out of four normal lung samples, the remaining showing low or moderate expression. Contig 48 showed moderate expression in soft palate, large intestine, pancreas, and bladder, and low expression in esophagus, salivary gland, resting PBMC, and heart. Contig 49 (SEQ ID NO: 144) was expressed at low to moderate levels in 6/17 head and neck squamous cell tumors tested. Expression levels in lung squamous tumors were moderate in three samples (n=13). Contig 49 was negative for 2/4 normal lung samples, the remaining samples showing low expression. Moderate expression levels in skin, salivary gland, large intestine, pancreas, bladder and resting PBMC were shown, as well as low expression in soft palate, lymph nodes, and tonsil. Contig 56 (SEQ ID NO: 148) was expressed in low to moderate levels in 3/17 head and neck squamous cell tumors tested, and in lung squamous tumors, showing low to moderate levels in three out of thirteen samples. Notably, low expression levels were detected in one adenocarcinoma lung tumor sample (n=2). Contig 56 was negative for 3/4 normal lung samples, and showed moderate expression levels in only large intestine, and low expression in salivary gland, soft palate, pancreas, bladder, and resting PBMC. Contig 58, also known as L769P, (SEQ ID NO: 150) was expressed at moderate levels in 11/17 head and neck squamous cell tumors tested and low expression in one additional sample. Expression in lung squamous tumors showed low to moderate levels in three out of thirteen samples. Contig 58 was negative for 3/4 normal lung samples, with one sample having low expression. Moderate expression levels in skin, large intestine, and resting PBMC were demonstrated, as well as low expression in salivary gland, soft palate, pancreas, and bladder. Contig 59 (SEQ ID NO: 157) was expressed in some head, neck, and lung squamous tumors. Low level expression of Contig 59 was also detected in salivary gland and large intestine.

Additionally, the full-length cDNA sequence for Contigs 22, referred to as L763P, is provided in SEQ ID NO: 158, with the corresponding predicted amino acid sequence being provided in SEQ ID NO: 159. Also, the full-length cDNA sequence incorporating Contigs 17, 19, and 24, referred to as L762P, is provided in SEQ ID NO: 160, with the corresponding predicted amino acid sequence being provided in SEQ ID NO: 161. Further analysis of L762P has determined it to be a type 1 membrane protein and two additional variants have been sequenced. Variant 1 (SEQ ID NO: 167 and the corresponding amino acid sequence in SEQ ID NO: 169) is an alternatively spliced form of SEQ ID NO: 160 resulting in deletion of 503 nucleotides, as well as deletion of a short segment of the expressed protein. Variant 2 (SEQ ID NO: 168 and the corresponding amino acid sequence in SEQ ID NO: 170) has a two nucleotide deletion at the 3' coding region in comparison to SEQ ID NO: 160, resulting in a secreted form of the expressed protein.

The full-length cDNA sequence for contig 56 (SEQ ID NO: 148), referred to as L773P, is provided in SEQ ID NO: 171, with the predicted amino acid sequence in SEQ ID NO: 172. Subsequent Northern blot analysis of L773P demonstrates this transcript is differentially over-expressed in squamous tumors and detected at approximately 1.6 Kb in primary lung tumor tissue and approximately 1.3 Kb in primary head and neck tumor tissue.

Subsequent microarray analysis has shown Contig 58, also referred to as L769S (SEQ ID NO: 150), to be overexpressed in breast tumors in addition to lung squamous tumors.

### Example 4

### SYNTHESIS OF POLYPEPTIDES

Polypeptides may be synthesized on a Perkin Elmer/Applied Biosystems Division 430A peptide synthesizer using FMOC chemistry with HPTU (O-Benzotriazole-N,N,N',N'-tetramethyluronium hexafluorophosphate) activation. A Gly-Cys-Gly sequence may be attached to the amino terminus of the peptide to provide a method of conjugation, binding to an immobilized surface, or labeling of the peptide. Cleavage of the peptides from the solid support may be carried out using the following cleavage mixture: trifluoroacetic acid:ethanedithiol:thioanisole:water:phenol (40:1:2:2:3). After cleaving for 2 hours, the peptides may be precipitated in cold methyl-t-butyl-ether. The peptide pellets may then be dissolved in water containing 0.1% trifluoroacetic acid (TFA) and lyophilized prior to purification by C18 reverse phase HPLC. A gradient of 0%-60% acetonitrile (containing 0.1% TFA) in water (containing 0.1% TFA) may be used to elute the peptides. Following lyophilization of the pure fractions, the peptides may be characterized using electrospray or other types of mass spectrometry and by amino acid analysis.

## Claims

1. An isolated polynucleotide molecule comprising the nucleotide sequence provided in SEQ ID NO: 160 or the complement thereof.

2. An isolated polypeptide comprising an amino acid sequence encoded by the polynucleotide molecule of claim 1.

3. An isolated polypeptide comprising the amino acid sequence provided in SEQ ID NO:161.

4. An expression vector comprising the isolated polynucleotide molecule of claim 1.

5. A host cell transformed with the expression vector of claim 4.

6. The host cell of claim 5 wherein the host cell is selected from the group consisting of *E*. *coli,* yeast and mammalian cell lines.

7. A pharmaceutical composition comprising the polypeptide of claim 2 or claim 3 and a physiologically acceptable carrier.

8. A vaccine comprising an isolated polynucleotide molecule of claim 1 or the polypeptide of claims 2 or 3 and a non-specific immune response enhancer.

9. A pharmaceutical composition for the treatment of lung cancer comprising a polypeptide according to claim 2 or claim 3 and a physiologically acceptable carrier.

10. A vaccine for the treatment of lung cancer comprising a polypeptide according to claim 2 or claim 3 and a non-specific immune response enhancer.

11. A vaccine for the treatment of lung cancer comprising an isolated polynucleotide molecule according to claim 1 and a non-specific immune response enhancer..

12. A fusion protein comprising at least one polypeptide according to claim 2, preferably further comprising a known lung tumor antigen.

13. A pharmaceutical composition comprising a fusion protein according to claim 12 and a physiologically acceptable carrier.

14. A vaccine comprising a fusion protein according to claim 12 and a non-specific immune response enhancer.

15. The vaccine of claim 8 or 14 wherein the non-specific immune response enhancer is an adjuvant.

16. The pharmaceutical composition of any one of claims 7, 9 or 13 for use in a method for inhibiting the development of lung cancer in a patient.

17. The vaccine of any one of claims 8, 10, 11, 14 or 15 for use in a method for inhibiting the development of lung cancer in a patient.

18. A method for detecting lung cancer in a patient, comprising:
(a) contacting a biological sample obtained from the patient with a binding agent which is capable of binding to a polypeptide, the polypeptide comprising at least an immunogenic portion of a lung protein or a variant thereof, wherein said protein comprises an amino acid sequence encoded by a polynucleotide molecule comprising the polynucleotide provided in SEQ ID NO:160;; and
(b) detecting in the sample a protein or polypeptide that binds to the binding agent, thereby detecting lung cancer in the patient.

19. The method of claim 18 wherein the binding agent is a monoclonal antibody, or a polyclona1 antibody.

20. A method for monitoring the progression of lung cancer in a patient, comprising:
(a) contacting a biological sample obtained from the patient with a binding agent that is capable of binding to a polypeptide, said polypeptide comprising at least an immunogenic portion of a lung protein or a variant thereof, wherein said protein comprises an amino acid sequence encoded by the polynucleotide provided in SEQ ID NO:160; and;
(b) determining in the sample an amount of a protein or polypeptide that binds to the binding agent;
(c) repeating steps (a) and (b); and
(d) comparing the amount of polypeptide detected in steps (b) and (c) to monitor the progression of lung cancer in the patient.

21. A monoclonal antibody that is specific for the polypeptide comprising an amino acid sequence encoded by the polynucleotide provided in SEQ ID NO:160.

22. A monoclonal antibody according to claim 21 for use in a method for inhibiting the development of lung cancer in a patient.

23. The antibody of claim 22 wherein the monoclonal antibody is conjugated to a therapeutic agent.

24. A method for detecting lung cancer in a patient comprising:
(a) contacting a biological sample obtained from the patient with at least two oligonucleotide primers in a polymerase chain reaction, wherein at least one of the oligonucleotides is specific for the polynucleotide provided in SEQ ID NO:160 or the complement thereof; and
(b) detecting in the sample a polynucleotide sequence that amplifies in the presence of the oligonucleotide primers, thereby detecting lung cancer.

25. A diagnostic kit comprising:
(a) one or more monoclonal antibodies of claim 21; and
(b) a detection reagent.

26. The kit of claims 25 wherein the monoclonal antibodies are immobilized on a solid support, preferably comprising nitrocellulose, latex or a plastic materiel.

27. The kit of claims 25 or 26 wherein the detection reagent comprises a reporter group, preferably selected from the group consisting of radioisotopes, fluorescent groups, luminescent groups, enzymes, biotin and dye particles, conjugated to a binding agent, preferably selected from the group consisting of anti-immunoglobulins, Protein G, Protein A and lectins.

28. A diagnostic kit comprising at least two oligonucleotide primers, at least one of the oligonucleotide primers being specific for the polynucleotide molecule provided in SEQ ID NO:160 or the complement thereof.

29. A method according to claim 24 or a diagnostic kit of claim 28 wherein at least one of the oligonucleotide primers comprises at least 10 contiguous nucleotides of a polynucleotide molecule comprising the sequence provided in SEQ ID NO:160.

30. A method for detecting lung cancer in a patient, comprising:
(b) contacting a biological sample obtained from the patient with an oligonucleotide probe specific for the polynucleotide molecule provided in SEQ ID NO: 160, or the complement thereof; and
(c) detecting in the sample a polynucleotide sequence that hybridizes to the oligonucleotide probe, thereby detecting lung cancer in the patient.

31. A diagnostic kit comprising an oligonucleotide probe specific for the polynucleotide molecule provided in SEQ ID NO:160 or the complement thereof.

32. The method of claim 30 or the diagnostic kit of claim 31, wherein the oligonucleotide probe comprises at least 15 contiguous nucleotides of the polynucleotide molecule provided in SEQ ID NO:160 or the complement thereof.

33. A method for preparing a blood product, comprising the step of: incubating peripheral blood cells, obtained from a patient, in the presence of at least one polypeptide of claim 2, or at least one polynucleotide of claim 1, such that T cells proliferate.

34. The method of claim 33 wherein the step of incubating the T cells is repeated one or more times.

35. The method of claim 33 or 34, further comprising the step of separating T cells from the peripheral blood cells, such that the cells incubated are the T cells.

36. The method of claim 33 or 34 further comprising separating CD4+ cells or CD8+ cells from the peripheral blood cells such that the cells proliferated are CD4+ or CD8+ T cells.

37. The method of any one of claims 33 to 36 further comprising cloning one or more T cells that proliferated in the presence of the polypeptide or polynucleotide.

38. A blood product prepared according to any one of claims 33 to 37 for use in a method for treating lung cancer.

39. A composition for the treatment of lung cancer in a patient, comprising T cells proliferated in the presence of a polypeptide of claim 2 or a polynucleotide of claim 1, in combination with a pharmaceutically acceptable carrier.

40. Antigen presenting cells incubated in the presence of at least one polypeptide of claim 2 or at least one polynucleotide of claim 1 for use in a method of treating lung cancer in a patient.

41. The antigen presenting cells of claim 40 wherein the antigen presenting cells are selected from the group consisting of dendritic cells and macrophage cells.

42. A composition for the treatment of lung cancer in a patient, comprising antigen presenting cells incubated in the presence of a polypeptide of claim

43. The use of a monoclonal antibody that binds to a polypeptide comprising an amino acid sequence encoded by the polynucleotide provided in SEQ ID No: 160 for the manufacture of a medicament for inhibiting the development of lung cancer in a patient.

44. The use of claim 43, wherein the monoclonal antibody is conjugated to a therapeutic agent.

## Patentansprüche

1. Isoliertes Polynucleotid-Molekül, umfassend die Nucleotidsequenz gemäß SEQ ID No. 160 oder deren Komplement.

2. Isoliertes Polypeptid, umfassend eine Aminosäuresequenz kodiert durch das Polynucleotid-Molekül gemäß Anspruch 1.

3. Isoliertes Polypeptid, umfassend die Aminosäuresequenz gemäß SEQ ID No. 161.

4. Expressionsvektor, umfassend das Isolierte Polynucleotid-Molekül gemäß Anspruch 1.

5. Wirtszelle, transformiert mit dem Expressionsvektor gemäß Anspruch 4.

6. Wirtszelle nach Anspruch 6, wobei die Wirtszelle ausgewählt ist aus der Gruppe bestehend aus E. coli, Hefe und Säuger-Zellinien.

7. Pharmazeutische Zusammensetzung, umfassend das Polypeptid gemäß Anspruch 2 oder 3 und einen physiologisch akzeptablen Träger.

8. Vakzin, umfassend ein isoliertes Polynucleotid-Molekül gemäß Anspruch 1 oder das Polypeptid gemäß Anspruch 2 oder 3, und einen unspezifischen Immunantwort-Verstärker.

9. Pharmazeutische Zusammensetzung zur Behandlung von Lungenkrebs, umfassend ein Polypeptid gemäß Anspruch 2 oder 3 und einen physiologisch akzeptablen Träger.

10. Vakzin für die Behandlung von Lungenkrebs, umfassend ein Polypeptid gemäß Anspruch 2 oder 3 und einen unspezifischen Immunantwort-Verstärker.

11. Vakzin zur Behandlung von Lungenkrebs, umfassend ein Isoliertes Polynucleotld-Molekül gemäß Anspruch 1 und einen unspezifischen Immunantwort-Verstärker.

12. Fusionsprotein, umfassend zumindest ein Polypeptid gemäß Anspruch 2, vorzugsweise ferner umfassend ein bekanntes Lungentumor-Antigen.

13. Pharmazeutische Zusammensetzung, umfassend ein Fusionsprotein gemäß Anspruch 12 und einen physiologisch akzeptablen Träger.

14. Vakzin, umfassend ein Fusionsprotein gemäß Anspruch 12 und einen unspezifischen Immunantwort-Verstärker.

15. Vakzin gemäß Anspruch 8 oder 14, wobei der unspezifische immunantwort-Verstärker ein Adjuvans ist.

16. Pharmazeutische Zusammensetzung gemäß einem der Ansprüche 7, 9 oder 13 zur Verwendung in einem Verfahren zum Inhibieren der Entwicklung von Lungenkrebs in einem Patienten.

17. Vakzin gemäß einem der Ansprüche 8, 10, 11, 14 oder 15 zur Verwendung in einem Verfahren zum Inhibieren der Entwicklung von Lungenkrebs in einem Patienten.

18. Verfahren zum Nachweisen von Lungenkrebs in einem Patienten, umfassend:
(a) In Kontakt bringen einer biologischen Probe, die vom Patienten erhalten wurde, mit einem Bindungsmittel, das geeignet ist zum Binden an ein Polypeptid, wobei das Polypeptid zumindest einen Immunogenen Abschnitt eines Lungenproteins oder einer Variante eines solchen umfasst, wobei das Protein einer Aminosäuresequenz umfasst, die kodiert ist durch ein Polynucleotid-Molekül umfassend das Polynucleotid gemäß SEQ ID No. 160; und
(b) in der Probe Nachweisen eines Proteins oder Polypeptids, das an das Bindungsmittel bindet, um Lungenkrebs im Patienten nachzuweisen.

19. Verfahren nach Anspruch 18, wobei das Bindungsmittel ein monoklonaler Antikörper oder ein polyklonaler Antikörper ist.

20. Verfahren zum Beobachten des Fortschreitens von Lungenkrebs in einem Patienten, umfassend
(a) in Kontakt bringen einer biologischen Probe, die vom Patienten erhalten wurde, mit einem Bindungsmittel, das geeignet ist zum Binden an ein Polypeptid, wobei das Polypeptid zumindest einen immunogenen Abschnitt eines Lungenproteins oder einer Variante eines solchen umfasst, wobei das Protein einer Aminosäuresequenz umfasst, die kodiert ist durch ein Polynucleotid-Molekül umfassend das Polynucleotid gemäß SEQ ID No. 160; und
(b) in der Probe Bestimmen einer Menge an Protein oder Polypeptid, die an das Bindungsmittel bindet;
(c) Wiederholen der Schritte (a) und (b); und
(d) Vergleichen der Menge an nachgewiesenem Polypeptid in den Schritten (b) und (c), zum Überwachen des Fortschreitens von Lungenkrebs im Patienten.

21. Monoklonaler Antikörper, der spezifich ist für das Polypeptid umfassend eine Aminosäuresequenz kodiert durch das Polynucleotid gemäß SEQ ID No. 160.

22. Monoklonaler Antikörper gemäß Anspruch 21 zur Verwendung in einem Verfahren zum Inhibieren der Entwicklung von Lungenkrebs in einem Patienten.

23. Antikörper gemäß Anspruch 22, wobei der monoklonale Antikörper konjugiert ist mit einem therapeutischen Mittel.

24. Verfahren zum Nachweisen von Lungenkrebs in einem Patienten, umfassend:
(a) in Kontakt bringen einer biologischen Probe, die vom Patienten erhalten wurde, mit zumindest zwei Oligonucleotid-Primern in einer Polymerase-Kettenreaktion, wobei zumindest eines der Oligonucleotide spezifisch für das Polynucleotid gemäß SEQ ID No. 160 oder dessen Komplement ist, und
(b) in der Probe Nachweisen einer Polynucleotidsequenz, die in Gegenwart der Oligonucleotid-Primer vervielfältigt wird, wodurch Lungenkrebs nachgewiesen wird.

25. Diagnosezusammenstellung, umfassend
(a) einen oder mehrere monoklonale Antikörper gemäß Anspruch 21; und
(b) ein Nachweismittel.

26. Zusammenstellung gemäß Anspruch 25, wobei die monoklonalen Antikörper auf einem festen Träger Immobilisiert sind, der vorzugsweise Nitrozellulose, Latex oder ein Plastikmaterial umfasst.

27. Zusammenstellung gemäß Anspruch 25 oder 26, wobei das Nachweismittel eine Reportergruppe umfasst, die vorzugsweise ausgewählt ist aus der Gruppe bestehend Radioisotopen, Fluoreszontengruppen, Lumineszentengruppen, Enzymen, Biotin und Farbstoffpartikeln, konjugiert mit einem Bindungsmittel, das vorzugsweise ausgewählt ist aus der Gruppe bestehend aus Anti-Immunglobulinen, Protein G, Protein A und Lectinen.

28. Diagnosezusammenstellung, umfassend zumindest zwei Oligonucleotid-Primer, und wobei zumindest einer der Oligonucleotid-Primer spezifisch für das Polynucleotid-Molekül gemäß SEQ ID No. 160 oder dessen Komplement ist.

29. Verfahren gemäß Anspruch 24 oder Diagnosezusammenstellung gemäß Anspruch 28, wobei zumindest einer der Oligonucleotid Primer zumindest zehn zusammenhängende Nucleotide eines Polynucleotid-Moleküls umfassend die Sequenz gemäß SEQ ID No. 160 umfasst.

30. Verfahren zum Nachweisen von Lungenkrebs in einem Patienten, umfassend:
(b) in Kontakt bringen einer biologischen Probe, die vom Patienten erhalten wurde, mit einer Oligonucleotid-Sonde, die spezifisch ist für das Polynucleotid-Molekül gemäß SEQ ID No. 160 oder dessen Komplement; und
(c) in der Probe nachweisen einer Polynucleotidsequenz, die mit der Oligonucleotid-Sonde hybridisiert, um Lungenkrebs im Patienten nachzuweisen.

31. Diagnosezusammenstellung, umfassend eine Oligonucleotid-Sonde, die spezifisch ist für das Polynucleotid-Molekül gemäß SEQ ID No. 160 oder dessen Komplement.

32. Verfahren gemäß Anspruch 30 oder Diagnosezusammenstellung gemäß Anspruch 31, wobei die Oligonucleotid-Sonde zumindest 15 zusammenhängende Oligonucleotide des Polynucleotid-Moleküls gemäß SEQ ID No. 160 oder dessen Komplement umfasst.

33. Verfahren zum Herstellen eines Blutproduktes, umfassend den Schritt: inkubieren peripherer Blutzellen, die von einem Patienten erhalten wurde, in Anwesenheit von zumindest einem Polypeptid gemäß Anspruch 2 oder zumindest einem Polynucleotid gemäß Anspruch 1, so dass T-Zellen proliferieren.

34. Verfahren gemäß Anspruch 33, wobei der Schritt des inkubierens der T-Zellen einmal oder mehrmals wiederholt wird.

35. Verfahren gemäß Anspruch 33 oder 34, ferner umfassend den Schritt des Abtrennens von T-Zellen von den peripheren Blutzellen, so dass die inkubierten Zellen die T-Zellen sind.

36. Verfahren gemäß Anspruch 33 oder 34, ferner umfassend: Abtrennen von CD4+-Zellen oder CDB+-Zellen von den peripheren Blutzellen, so dass die proliferierten Zellen CD4+ oder CD8+ T-Zellen sind.

37. Verfahren gemäß einem der Ansprüche 33 bis 36, ferner umfassend: Klonieren von einer oder mehreren T-Zellen, die in Anwesenheit des Polypeptids oder Polynucleolids proliferiert sind.

38. Blutprodukt, hergestellt gemäß einem der Ansprüche 33 bis 37, zur Verwendung in einem Verfahren zur Behandlung von Lungenkrebs,

39. Zusammensetzung zur Behandlung von Lungenkrebs in einem Patienten, umfassend T-Zellen, die proliferiert sind in Gegenwart eines Polypeptids gemäß Anspruch 2 oder eines Polynucleotids gemäß Anspruch 1, in Kombination mit einem pharmazeutisch akzeptablen Träger.

40. Antigen-präsentierende Zellen, die in Anwesenheit von zumindest einem Polypeptid gemäß Anspruch 2 oder zumindest einem Polynucleotid gemäß Anspruch 1 inkubiert sind, zur Verwendung in einem Verfahren zum Behandeln von Lungenkrebs in einem Patienten.

41. Antigen-präsentierende Zellen gemäß Anspruch 40, wobei die Antigenpräsentierenden Zeilen ausgewählt sind aus der Gruppe bestehend aus dendritischen Zellen und Mekrophagenzellen.

42. Zusammensetzung zur Behandlung von Lungenkrebs in einem Patienten, umfassend Anfigen-präsentierende Zellen, die in Anwesenheit eines Polypeptids gemäß Anspruch 2 inkubiert sind oder ein Polynucleotid gemäß Anspruch 1 in Kombination mit einem pharmazeutisch akzeptablen Träger.

43. Verwendung eines monoklonalen Antikörpers, der an ein Polypeptid bindet, das eine Aminosäuresequenz umfasst, die durch das Polynucleotid gemäß SEQ ID No. 160 kodiert wird, zum Herstellen eines Arzneimittels zum Inhibieren der Entwicklung von Lungenkrebs in einem Patienten.

44. Verwendung nach Anspruch 43, wobei der monoklonale Antikörper konjugiert ist an ein therapeutisches Mittel.

## Revendications

1. Molécule polynucléotidique isolée comprenant la séquence nucléotidique proposée dans la SEQ ID NO : 160 ou le complément de celle-ci.

2. Polypeptide isolé comprenant une séquence d'acides aminés codée par la molécule polynucléotidique selon la revendication 1.

3. Polypeptide isolé comprenant la séquence d'acides aminés proposée dans la SEQ ID NO : 161.

4. Vecteur d'expression comprenant la molécule polynucléotidique isolée selon la revendication 1.

5. Cellule hôte transformée par le vecteur d'expression selon la revendication 4.

6. Cellule hôte selon la revendication 5, la cellule hôte étant choisie dans le groupe composé de lignées cellulaires de *E. coli,* de levures et de mammifères.

7. Composition pharmaceutique comprenant le polypeptide selon la revendication 2 ou la revendication 3 et un véhicule physiologiquement acceptable.

8. Vaccin comprenant une molécule polynucléotidique isolée selon la revendication 1 ou le polypeptide selon les revendications 2 ou 3 et un stimulateur de réponse immunitaire non spécifique.

9. Composition pharmaceutique pour le traitement du cancer du poumon comprenant un polypeptide selon la revendication 2 ou la revendication 3 et un véhicule physiologiquement acceptable.

10. Vaccin pour le traitement du cancer du poumon comprenant un polypeptide selon la revendication 2 ou la revendication 3 et un stimulateur de réponse immunitaire non spécifique.

11. Vaccin pour le traitement du cancer du poumon comprenant une molécule polynucléotidique isolée selon la revendication 1 et un stimulateur de réponse immunitaire non spécifique.

12. Protéine de fusion comprenant au moins un polypeptide selon la revendication 2, de préférence comprenant en outre un antigène de cancer du poumon connu.

13. Composition pharmaceutique comprenant une protéine de fusion selon la revendication 12 et un véhicule physiologiquement acceptable.

14. Vaccin comprenant une protéine de fusion selon la revendication 12 et un stimulateur de réponse immunitaire non spécifique.

15. Vaccin selon la revendication 8 ou 14, dans lequel le stimulateur de réponse immunitaire non spécifique est un adjuvant.

16. Composition pharmaceutique selon l'une quelconque des revendications 7, 9 ou 13 à utiliser dans un procédé destiné à inhiber le développement d'un cancer du poumon chez un patient.

17. Vaccin selon l'une quelconque des revendications 8, 10, 11, 14 ou 15 à utiliser dans un procédé destiné à inhiber le développement d'un cancer du poumon chez un patient.

18. Procédé de détection du cancer du poumon chez un patient comprenant :
(a) mettre en contact un échantillon biologique obtenu chez le patient avec un agent de liaison qui est capable de se lier à un polypeptide, le polypeptide comprenant au moins une partie immunogène d'une protéine du poumon ou une variante de celle-ci, dans lequel ladite protéine comprend une séquence d'acides aminés codée par une molécule polynucléotidique comprenant le polynucléotide proposé dans la SEQ ID NO : 160 ; et
(b) détecter dans l'échantillon une protéine ou un polypeptide qui se lie à l'agent de liaison, en détectant par ce moyen un cancer du poumon chez le patient.

19. Procédé selon la revendication 18, dans lequel l'agent de liaison est un anticorps monoclonal ou un anticorps polyclonal.

20. Procédé de surveillance de la progression du cancer du poumon chez un patient comprenant :
(a) mettre en contact un échantillon biologique obtenu chez le patient avec un agent de liaison qui est capable de se lier à un polypeptide, ledit polypeptide comprenant au moins une partie immunogène d'une protéine du poumon ou une variante de celle-ci, dans lequel ladite protéine comprend une séquence d'acides aminés codée par le polynucléotide proposé dans la SEQ ID NO : 160 ; et
(b) déterminer dans l'échantillon biologique la quantité d'une protéine ou d'un polypeptide qui se lie à l'agent de liaison ;
(c) répéter les étapes (a) et (b) ; et
(d) comparer la quantité de polypeptide détectée aux étapes (b) et (c) pour surveiller la progression du cancer du poumon chez le patient.

21. Anticorps monoclonal qui est spécifique du polypeptide comprenant une séquence d'acides aminés codée par le polynucléotide proposé dans la SEQ ID NO : 160.

22. Anticorps monoclonal selon la revendication 21 à utiliser dans un procédé d'inhibition du développement du cancer du poumon chez un patient.

23. Anticorps selon la revendication 22, dans lequel l'anticorps monoclonal est conjugué à un agent thérapeutique.

24. Procédé de détection du cancer du poumon chez un patient comprenant :
(a) mettre en contact un échantillon biologique obtenu chez le patient avec au moins deux amorces oligonucléotidiques dans une amplification en chaîne par polymérase, dans lequel au moins un des deux oligonucléotides est spécifique du polynucléotide proposé dans la SEQ ID NO : 160 ou du complément de celui-ci ; et
(b) détecter dans l'échantillon une séquence polynucléotidique qui s'amplifie en présence des amorces oligonucléotidiques, en détectant par ce moyen le cancer du poumon.

25. Kit de diagnostic comprenant :
(a) un ou plusieurs anticorps monoclonaux selon la revendication 21 ; et
(b) un réactif de détection.

26. Kit selon la revendication 25, dans lequel les anticorps monoclonaux sont immobilisés sur un support solide, de préférence comprenant de la nitrocellulose, un latex ou une matière plastique.

27. Kit selon les revendications 25 ou 26, dans lequel le réactif de détection comprend un groupe rapporteur, de préférence choisi dans le groupe consistant en des radio-isotopes, des groupes fluorescents, des groupes luminescents, des enzymes, de la biotine et des particules de colorant, conjugué à un agent de liaison, de préférence choisi dans le groupe consistant en des anti-immunoglobulines, de la protéine G, de la protéine A et des lectines.

28. Kit de diagnostic comprenant au moins deux amorces oligonucléotidiques, au moins une des amorces oligonucléotidiques étant spécifique de la molécule polynucléotidique proposée dans la SEQ ID NO : 160 ou du complément de celle-ci.

29. Procédé selon la revendication 24 ou kit de diagnostic selon la revendication 28, dans lequel au moins une des amorces oligonucléotidiques comprend au moins 10 nucléotides contigus d'une molécule polynucléotidique comprenant la séquence proposée dans la SEQ ID NO : 160.

30. Procédé de détection du cancer du poumon chez un patient comprenant :
(a) mettre en contact un échantillon biologique obtenu chez le patient avec une sonde oligonucléotidique spécifique de la molécule polynucléotidique proposée dans la SEQ ID NO : 160 ou du complément de celle-ci ; et
(b) détecter dans l'échantillon une séquence polynucléotidique qui s'hybride à la sonde oligonucléotidique, en détectant par ce moyen le cancer du poumon chez le patient.

31. Kit de diagnostic comprenant une sonde oligonucléotidique spécifique de la molécule polynucléotidique proposée dans la SEQ ID NO : 160 ou du complément de celle-ci.

32. Procédé selon la revendication 30 ou kit de diagnostic selon la revendication 31, dans lequel la sonde oligonucléotidique comprend au moins 15 nucléotides contigus de la molécule polynucléotidique proposée dans la SEQ ID NO : 160 ou du complément de celle-ci.

33. Procédé de préparation d'un produit sanguin, comprenant l'étape suivante :
incuber des cellules du sang périphérique obtenues chez un patient en présence d'au moins un polypeptide selon la revendication 2 ou d'au moins un polynucléotide selon la revendication 1, de sorte que les cellules T prolifèrent.

34. Procédé selon la revendication 33, dans lequel l'étape d'incubation des cellules T est répétée une ou plusieurs fois.

35. Procédé selon la revendication 33 ou 34, comprenant en outre une étape de séparation entre les cellules T et les cellules du sang périphérique, de sorte que les cellules incubées soient les cellules T.

36. Procédé selon la revendication 33 ou 34, comprenant en outre une séparation entre les cellules CD4+ ou les cellules CD8+ et les cellules du sang périphérique, de sorte que les cellules que l'on fait proliférer soient des cellules CD4+ ou des cellules CD8+.

37. Procédé selon l'une quelconque des revendications 33 à 36, comprenant en outre le clonage d'une ou plusieurs des cellules T qui ont proliféré en présence du polypeptide ou du polynucléotide.

38. Produit sanguin préparé selon l'une quelconque des revendications 33 à 37 à utiliser dans un procédé de traitement du cancer du poumon.

39. Composition destinée au traitement du cancer du poumon chez un patient, comprenant des cellules T que l'on a fait proliférer en présence d'un polypeptide selon la revendication 2 ou d'un polynucléotide selon la revendication 1 en combinaison avec un véhicule pharmaceutiquement acceptable.

40. Cellules présentant l'antigène incubées en présence d'au moins un polypeptide selon la revendication 2 ou d'au moins un polynucléotide selon la revendication 1 à utiliser dans un procédé de traitement du cancer du poumon chez un patient.

41. Cellules présentant l'antigène selon la revendication 40, dans lesquelles les cellules présentant l'antigène sont sélectionnées dans le groupe consistant en des cellules dendritiques et des cellules macrophages.

42. Composition de traitement du cancer du poumon chez un patient, comprenant des cellules présentant l'antigène incubées en présence d'un polypeptide selon la revendication 2 ou d'un polynucléotide selon la revendication 1 en combinaison avec un véhicule pharmaceutiquement acceptable.

43. Utilisation d'un anticorps monoclonal qui se lie à un polypeptide comprenant une séquence d'acides aminés codée par le polynucléotide proposé dans la SEQ ID NO : 160 pour la fabrication d'un médicament destiné à inhiber le développement du cancer du poumon chez un patient.

44. Utilisation selon la revendication 43, dans laquelle l'anticorps monoclonal est conjugué à un agent thérapeutique.


### SEQUENCE LISTING

<110> Wang, Tongtong
<120> COMPOUNDS AND METHODS FOR THERAPY OF LUNG CANCER
<130> 210121.455PC
<140> PCT
   <141> 1999-03-16
<160> 172
<170> FastSEQ for Windows Version 3.0
<210> 1
   <211> 315
   <212> DNA
   <213> Homo sapien
<220>
   <221> misc_feature
   <222> (1)...(315)
   <223> n = A,T,C cr G
<400> 1
   gcagagacag actggtggtt gaacctggag gtgccaaaaa agccagctgc gggcccagcg      60
   cagctgccgt gagactcccg atgtcacagg cagtctgtgt ggttacagcg cccctcagcc   120
   ttcatctcca gcagagacaa cggaggaggc tcccaccagg acggttctca ttacttatat  180
   gttaatatgt ttgtaaactc atgtacagtt ttttttgggg gggaagcaat gggaanggta  240
   naaattacaa atagaatcat ttgctgtaat ccttaaatgg caaacggtca ggccacgtga  300
   aaaaaaaaaa aaaaa                                                   315
<210> 2
   <211> 380
   <212> DNA
   <213> Homo sapien
<400> 2
   atttaggctt aagattttgt ttacccttgt tactaaggag caaattagta ttaaagtata   60
   atatatataa acaaatacaa aaagttttga gtggttcagc ttttttattt tttttaatgg   120
   cataactttt aacaacactg ctctgtaatg ggttgaactg tggtactcag actgagataa  180
   ctgaaatgag tggatgtata gtgttattgc ataattatcc cactatgaag caaaggcact    240
   ggataaattc ccagtctaga ttattagcct ttgttaacca tcaagcacct agaagaagaa  300
   ttattggaaa ttttgtcctc tgtaactggc actttggggt gtgacttatc ttttgccttt  360
   gtaaaaaaaa aaaaaaaaaa                                              380
<210> 3
   <211> 346
   <212> DNA
   <213> Homo sapien
<220>
   <221> misc_feature
   <222> (1)...(346)
   <223> n = A,T,C or G
<400> 3
<210> 4
   <211> 372
   <212> DNA
   <213> Homo sapien
<220>
   <221> misc_feature
   <222> (1)...(372)
   <223> n = A,T,C or G
<400> 4
<210> 5
   <211> 698
   <212> DNA
   <213> Homo sapien
<220>
   <221> misc_feature
   <222> (1)...(698)
   <223> n = A,T,C or G
<400> 5
<210> 6
   <211> 740
   <212> DNA
   <213> Homo sapien
<220>
   <221> misc_feature
   <222> (1)...(740)
   <223> n = A,T,C or G
<400> 6
   actagtcaaa aatgctaaaa taatttggga gaaaatattt tttaagtagt gttatagttt 60
   catgtttatc ttttattatg tnttgtgaag ttgtgtcttt tcactaatta cctatactat 120
   gccaatattt ccttatatct atccataaca tttatactac atttgtaaga gaatatgcac 180
   gtgaaactta acactttata aggtaaaaat gaggtttcca agatttaata atctgatcaa 240
   gttcttgtta tttccaaata gaatggactt ggtctgttaa ggggctaagg gagaagaaga 300
   agataaggtt aaaagttgtt aatgaccaaa cattctaaaa gaaatgcaaa aaaaaattta 360
   ttttcaagcc ttcgaactat ttaaggaaag caaaatcatt tcctanatgc atatcatttg 420
   tgagantttc tcantaatat cctgaatcat tcatttcagc tnaggcttca tgttgacttg 480
   atatgtcatc tagggaaagt ctatttcatg gtccaaacct gttgccatag ttggtnaggc 540
   tttcctttaa ntgtgaanta ttnacangaa attttctctt tnanagttct tnatagggtt 600
   aggggtgtgg gaaaagcttc taacaatctg tagtgttncg tgttatctgt ncagaaccan 660
   aatnacggat cgnangaagg actgggtcta tttacangaa cgaatnatct ngttnnntgt 720
   gtnnncaact ccngggagcc 740
<210> 7
   <211> 670
   <212> DNA
   <213> Homo sapien
<220>
   <221> misc_feature
   <222> (1)...(670)
   <223> n = A,T,C or G
<400> 7
   gctggggagc tcggcatggc ggtccccgct gcagccatgg ggccctcggc gttgggccag 60
   agcggccccg gctcgatggc cccgtggtgc tcagtgagca gcggcccgtc gcgctacgtg 120
   cttgggatgc aggagctgtt ccggggccac agcaagaccg cgagttcctg gcgcacagcg 180
   ccaaggtgca ctcggtggcc tggagttgcg acgggcgtcg cctacctcgg ggtcttcgac 240
   aagacgccac gtcttcttgc tgganaanga ccgttggtca aagaaaacaa ttatcgggga 300
   catggggata gtgtggacca ctttgttggc atccaagtaa tcctgaccta tttgttacgg 360
   cgtctggaga taaaaccatt cgcatctggg atgtgaggac tacaaaatgc attgccactg 420
   tgaacactaa aggggagaac attaatatct gctggantcc tgatgggcan accattgctg 480
   tagcnacaag gatgatgtgg tgactttatt gatgccaaga aaccccgttc caaagcaaaa 540
   aaacanttcc aanttcgaag tcaccnaaat ctcctggaac aatgaacatn aatatntcct 600
   tcctgacaat ggnccttggg tgtntcacat cctcagctnc cccaaaactg aancctgtnc 660
   natccacccc 670
<210> 8
   <211> 689
   <212> DNA
   <213> Homo sapien
<220>
   <221> misc_feature
   <222> (1)...(689)
   <223> n = A,T,C or G
<400> 8
   actagtatct aggaatgaac agtaaaagag gagcagttgg ctacttgatt acaacagagt      60
   aaatgaagta ctggatttgg gaaaacctgg ttttattaga acatatggaa tgaaagccta     120
   cacctagcat tgcctactta gccccctgaa ttaacagagc ccaattgaga caaacccctg     180
   gcaacaggaa attcaaggga gaaaaagtaa gcaacttggg ctaggatgag ctgactccct     240
   tagagcaaag ganagacagc ccccattacc aaataccatt tttgcctggg gcttgtgcag     300
   ctggcagtgt tcctgcccca gcatggcacc ttatngtttt gatagcaact tcgttgaatt     360
   ttcaccaact tattacttga aattataata tagcctgtcc gtttgctgtn tccaggctgt     420
   gatatatntt cctagtggtt tgactttnaa aataaatnag gtttantttt ctccccccnn     480
   cnntnctncc nntcnctcnn cnntcccccc cnctcngtcc tccnnnnttn gggggggccn     540
   cccccncggn ggacccccct ttggtccctt agtggaggtt natggcccct ggnnttatcc     600
   nggccntann tttccccgtn nnaaatgntt ccccctccca ntcccnccac ctcaanccgg     660
   aagcctaagt ttntaccctg ggggtcccc                                       689
<210> 9
   <211> 674
   <212> DNA
   <213> Homo sapien
<220>
   <221> misc_feature
   <222> (1)...(674)
   <223> n = A,T,C or G
<400> 9
   gtccactctc ctttgagtgt actgtcttac tgtgcactct gtttttcaac tttctagata      60
   taaaaaatgc ttgttctata gtggagtaag agctcacaca cccaaggcag caagataact     120
   gaaaaaagcg aggctttttt gccaccttgg taaaggccag ttcactgcta tagaactgct     180
   ataagcctga agggaagtag ctatgagact ttccattttt cttagttctc ccaataggct     240
   ccttcatgga aaaaggcttc ctgtaataat tttcacctaa tgaattagca gtgtgattat     300
   ttctgaaata agagacaaat tgggccgcag agtcttcctg tgatttaaaa taaacaaccc     360
   aaagttttgt ttggtcttca ccaaaggaca tactctaggg ggtatgttgt tgaagacatt     420
   caaaaacatt agctgttctg tctttcaatt tcaagttatt ttggagactg cctccatgtg     480
   agttaattac tttgctctgg aactagcatt attgtcatta tcatcacatt ctgtcatcat     540
   catctgaata atattgtgga tttccccctc tgcttgcatc ttcttttgac tcctctggga     600
   anaaatgtca aaaaaaaagg tcgatctact cngcaaggnc catctaatca ctgcgctgga     660
   aggacccnct gccc                                                       674
<210> 10
   <211> 346
   <212> DNA
   <213> Homo sapien
<220>
   <221> misc_feature
   <222> (1)...(346)
   <223> n = A,T,C or G
<400> 10
   actagtctgc tgatagaaag cactatacat cctattgttt ctttctttcc aaaatcagcc      60
   ttctgtctgt aacaaaaatg tactttatag agatggagga aaaggtctaa tactacatag     120
   ccttaagtgt ttctgtcatt gttcaagtgt attttctgta acagaaacat atttggaatg     180
   tttttctttt ccccttataa attgtaattc ctgaaatact gctgctttaa aaagtcccac     240
   tgtcagatta tattatctaa caattgaata ttgtaaatat acttgtctta cctctcaata     300
   aaagggtact tttctattan nnagnngnnn gnnnnataaa anaaaa                   346
<210> 11
   <211> 602
   <212> DNA
   <213> Homo sapien
<400> 11
   actagtaaaa agcagcattg ccaaataatc cctaattttc cactaaaaat ataatgaaat      60
   gatgttaagc tttttgaaaa gtttaggtta aacctactgt tgttagatta atgtatttgt     120
   tgcttccctt tatctggaat gtggcattag cttttttatt ttaaccctct ttaattctta     180
   ttcaattcca tgacttaagg ttggagagct aaacactggg atttttggat aacagactga     240
   cagttttgca taattataat cggcattgta catagaaagg atatggctac cttttgttaa     300
   atctgcactt tctaaatatc aaaaaaggga aatgaagtta taaatcaatt tttgtataat     360
   ctgtttgaaa catgagtttt atttgcttaa tattagggct ttgccccttt tctgtaagtc     420
   tcttgggatc ctgtgtagaa ctgttctcat taaacaccaa acagttaagt ccattctctg     480
   gtactagcta caaattcggt ttcatattct acttaacaat ttaaataaac tgaaatattt     540
   ctagatggtc tacttctgtt catataaaaa caaaacttga tttccaaaaa aaaaaaaaaa     600
   aa                                                                    602
<210> 12
   <211> 685
   <212> DNA
   <213> Homo sapien
<220>
   <221> misc_feature
   <222> (1)...(685)
   <223> n = A, T, C or G
<400> 12
   actagtcctg tgaaagtaca actgaaggca gaaagtgtta ggattttgca tctaatgttc      60
   attatcatgg tattgatgga cctaagaaaa taaaaattag actaagcccc caaataagct     120
   gcatgcattt gtaacatgat tagtagattt gaatatatag atgtagtatn ttgggtatct     180
   aggtgtttta tcattatgta aaggaattaa agtaaaggac tttgtagttg tttttattaa     240
   atatgcatat agtagagtgc aaaaatatag caaaaatana aactaaaggt agaaaagcat     300
   tttagatatg ccttaatnta nnaactgtgc caggtggccc tcggaataga tgccaggcag     360
   agaccagtgc ctgggtggtg cctccccttg tctgcccccc tgaagaactt ccctcacgtg     420
   angtagtgcc ctcgtaggtg tcacgtggan tantggganc aggccgnncn gtnanaagaa     480
   ancanngtga nagtttcncc gtngangcng aactgtccct gngccnnnac gctcccanaa     540
   cntntccaat ngacaatcga gtttccnnnc tccngnaacc tngccgnnnn cnngcccnnc     600
   cantntgnta accccgcgcc cggatcgctc tcnnntcgtt ctcncncnaa ngggntttcn     660
   cnnccgccgt cncnnccccg cnncc                                           685
<210> 13
   <211> 694
   <212> DNA
   <213> Homo sapien
<220>
   <221> misc_feature
   <222> (1)...(694)
   <223> n = A,T,C or G
<400> 13
   cactagtcac tcattagcgt tttcaatagg gctcttaagt ccagtagatt acgggtagtc 60
   agttgacgaa gatctggttt acaagaacta attaaatgtt tcattgcatt tttgtaagaa 120
   cagaataatt ttataaaatg tttgtagttt ataattgccg aaaataattt aaagacactt 180
   tttctctgtg tgtgcaaatg tgtgtttgtg atccattttt tttttttttt taggacacct 240
   gtttactagc tagctttaca atatgccaaa aaaggatttc tccctgaccc catccgtggt 300
   tcaccctctt ttccccccat gctttttgcc ctagtttata acaaaggaat gatgatgatt 360
   taaaaagtag ttctgtatct tcagtatctt ggtcttccag aaccctctgg ttgggaaggg 420
   gatcattttt tactggtcat ttccctttgg agtgtactac tttaacagat ggaaagaact 480
   cattggccat ggaaacagcc gangtgttgg gagccagcag tgcatggcac cgtccggcat 540
   ctggcntgat tggtctggct gccgtcattg tcagcacagt gccatgggac atggggaana 600
   ctgactgcac ngccaatggt tttcatgaag aatacngcat ncncngtgat cacgtnancc 660
   angacgctat gggggncana gggccanttg cttc 694
<210> 14
   <211> 679
   <212> DNA
   <213> Homo sapien
<220>
   <221> misc_feature
   <222> (1)...(679)
   <223> n = A,T,C or G
<400> 14
   cagccgcctg catctgtatc cagcgccang tcccgccagt cccagctgcg cgcgcccccc 60
   agtcccgnac ccgttcggcc cangctnagt tagncctcac catnccggtc aaaggangca 120
   ccaagtgcat caaatacctg cngtncggat ntaaattcat cttctggctt gccgggattg 180
   ctgtccntgc cattggacta nggctccgat ncgactctca gaccanganc atcttcganc 240
   naganactaa tnatnattnt tccagcttct acacaggagt ctatattctg atcggatccg 300
   gcnccctcnt gatgctggtg ggcttcctga gctgctgcgg ggctgtgcaa gagtcccant 360
   gcatgctggg actgttcttc ggcttcntct tggtgatatn cgccattgaa atacctgcgg 420
   ccatctgggg atattccact ncgatnatgt gattaaggaa ntccacggag ttttacaagg 480
   acacgtacaa cnacctgaaa accnnggatg anccccaccg ggaancnctg aangccatcc 540
   actatgcgtt gaactgcaat ggtttggctg gggnccttga acaatttaat cncatacatc 600
   tggccccann aaaggacntn ctcganncct tcnccgtgna attcngttct gatnccatca 660
   cagaagtctc gaacaatcc 679
<210> 15
   <211> 695
   <212> DNA
   <213> Homo sapien
<220>
   <221> misc_feature
   <222> (1)...(695)
   <223> n = A,T,C or G
<400> 15
   actagtggat aaaggccagg gatgctgctc aacctcctac catgtacagg gacgtctccc 60
   cattacaact acccaatccg aagtgtcaac tgtgtcagga ctaanaaacc ctggttttga 120
   ttaaaaaagg gcctgaaaaa aggggagcca caaatctgtc tgcttcctca cnttantcnt 180
   tggcaaatna gcattctgtc tcnttggctg cngcctcanc ncaaaaaanc ngaactcnat 240
   cnggcccagg aatacatctc ncaatnaacn aaattganca aggcnntggg aaatgccnga      300
   tgggattatc ntccgcttgt tgancttcta agtttcnttc ccttcattcn accctgccag      360
   ccnagttctg ttagaaaaat gccngaattc naacnccggt tttcntactc ngaatttaga 420
   tctncanaaa cttcctggcc acnattcnaa ttnanggnca cgnacanatn ccttccatna 480
   ancncacccc acntttgana gccangacaa tgactgcntn aantgaaggc ntgaaggaan 540
   aactttgaaa ggaaaaaaaa ctttgtttcc ggccccttcc aacncttctg tgttnancac 600
   tgccttctng naaccctgga agcccngnga cagtgttaca tgttgttcta nnaaacngac 660
   ncttnaatnt cnatcttccc nanaacgatt ncncc 695
<210> 16
   <211> 669
   <212> DNA
   <213> Homo sapien
<220>
   <221> misc_feature
   <222> (1) ... (669)
   <223> n = A,T,C or G
<400> 16
   cgccgaagca gcagcgcagg ttgtccccgt ttcccctccc ccttcccttc tccggttgcc 60
   ttcccgggcc ccttacactc cacagtcccg gtcccgccat gtcccagaaa caagaagaag 120
   agaaccctgc ggaggagacc ggcgaggaga agcaggacac gcaggagaaa gaaggtattc 180
   tgcctgagag agctgaagag gcaaagctaa aggccaaata cccaagccta ggacaaaagc 240
   ctggaggctc cgacttcctc atgaagagac tccagaaagg gcaaaagtac tttgactcng 300
   gagactacaa catggccaaa gccaacatga agaataagca gctgccaagt gcangaccag 360
   acaagaacct ggtgactggt gatcacatcc ccaccccaca ggatctgccc agagaaagtc 420
   ctcgctcgtc accagcaagc ttgcgggtgg ccaagttgaa tgatgctgcc ggggctctgc 480
   canatctgag acgcttccct ccctgcccca cccgggtcct gtgctggctc ctgcccttcc 540
   tgcttttgca gccangggtc aggaagtggc ncnggtngtg gctggaaagc aaaacccttt 600
   cctgttggtg tcccacccat ggagcccctg gggcgagccc angaacttga ncctttttgt 660
   tntcttncc 669
<210> 17
   <211> 697
   <212> DNA
   <213> Homo sapien
<220>
   <221> misc_feature
   <222> (1) ... (697)
   <223> n = A,T,C or G
<400> 17
   gcaagatatg gacaactaag tgagaaggta atnctctact gctctagntn ctccnggcnn 60
   gacgcgctga ggagannnac gctggcccan ctgccggcca cacacgggga tcntggtnat 120
   gcctgcccan gggancccca ncnctcggan cccatntcac acccgnnccn tncgcccacn 180
   ncctggctcn cncngcccng nccagctcnc gnccccctcc gccnnnctcn ttnncntctc 240
   cncnccctcc ncnacnacct cctacccncg gctccctccc cagccccccc ccgcaancct 300
   ccacnacncc ntcnncncga ancnccnctc gcnctcngcc ccngccccct gccccccgcc 360
   cncnacnncg cgntcccccg cgcncgcngc ctcnccccct cccacnacag ncncacccgc 420
   agncacgcnc tccgcccnct gacgccccnn cccgccgcgc tcaccttcat ggnccnacng 480
   ccccgctcnc nccnctgcnc gccgncnngg cgccccgccc cnnccgngtn ccncncgnng      540
   ccccngcngn angcngtgcg cnncangncc gngccgnncn ncaccctccg nccnccgccc       600
   cgcccgctgg gggctcccgc cncgcggntc antccccncc cntncgccca ctntccgntc 660
   cnncnctcnc gctcngcgcn cgcccnccnc ccccccc 697
<210> 18
   <211> 670
   <212> DNA
   <213> Homo sapien
<220>
   <221> misc_feature
   <222> (1)...(670)
   <223> n = A,T,C or G
<400> 18
   ctcgtgtgaa gggtgcagta cctaagccgg agcggggtag aggcgggccg gcaccccctt 60
   ctgacctcca gtgccgccgg cctcaagatc agacatggcc cagaacttga acgacttggc 120
   gggacggctg cccgccgggc cccggggcat gggcacggcc ctgaagctgt tgctgggggc 180
   cggcgccgtg gcctacggtg tgcgcgaatc tgtgttcacc gtggaaggcg ggcncagagc 240
   catcttcttc aatcggatcg gtggagtgca caggacacta tcctgggccg anggccttca 300
   cttcaggatc cttggttcca gtaccccanc atctatgaca ttcgggccag acctcgaaaa 360
   aatctcctcc ctacaggctc caaagaccta cagatggtga atatctccct gcgagtgttg 420
   tctcgaccaa tgctcangaa cttcctaaca tgttccancg cctaagggct ggactacnaa 480
   gaacgantgt tgccgtccat tgtcacgaag tgctcaagaa tttnggtggc caagttcaat 540
   gncctcacnn ctgatcnccc agcggggcca agttanccct ggttgatccc cgggganctg 600
   acnnaaaagg gccaaggact tcccctcatc ctggataatg tggccntcac aaagctcaac 660
   tttanccacc 670
<210> 19
   <211> 606
   <212> DNA
   <213> Homo sapien
<220>
   <221> misc_feature
   <222> (1)...(606)
   <223> n = A,T,C or G
<400> 19
   actagtgcca acctcagctc ccaggccagt tctctgaatg tcgaggagtt ccaggatctc 60
   tggcctcagt tgtccttggt tattgatggg ggacaaattg gggatggcca gagccccgag 120
   tgtcgccttg gctcaactgt ggttgatttg tctgtgcccg gaaagtttgg catcattcgt 180
   ccaggctgtg ccctggaaag tactacagcc atcctccaac agaagtacgg actgctcccc 240
   tcacatgcgt cctacctgtg aaactctggg aagcaggaag gcccaagacc tggtgctgga 300
   tactatgtgt ctgtccactg acgactgtca aggcctcatt tgcagaggcc accggagcta 360
   gggcactagc ctgactttta aggcagtgtg tctttctgag cactgtagac caagcccttg 420
   gagctgctgg tttagccttg cacctgggga aaggatgtat ttatttgtat tttcatatat 480
   cagccaaaag ctgaatggaa aagttnagaa cattcctagg tggccttatt ctaataagtt 540
   tcttctgtct gttttgtttt tcaattgaaa agttattaaa taacagattt agaatctagt 600
   gagacc 606
<210> 20
   <211> 449
   <212> DNA
   <213> Homo sapien
<400> 20
   actagtaaac aacagcagca gaaacatcag tatcagcagc gtcgccagca ggagaatatg 60
   cagcgccaga gccgaggaga acccccgctc cctgaggagg acctgtccaa actcttcaaa 120
   ccaccacagc cgcctgccag gatggactcg ctgctcattg caggccagat aaacacttac 180
   tgccagaaca tcaaggagtt cactgcccaa aacttaggca agctcttcat ggcccaggct 240
   cttcaagaat acaacaacta agaaaaggaa gtttccagaa aagaagttaa catgaactct 300
   tgaagtcaca ccagggcaac tcttggaaga aatatatttg catattgaaa agcacagagg 360
   atttctttag tgtcattgcc gattttggct ataacagtgt ctttctagcc ataataaaat 420
   aaaacaaaat cttgactgct tgctcaaaa 449
<210> 21
   <211> 409
   <212> DNA
   <213> Homo sapien
<400> 21
   tatcaatcaa ctggtgaata attaaacaat gtgtggtgtg atcatacaaa gggtaccact 60
   caatgataaa aggaacaagc tgcctatatg tggaacaaca tggatgcatt tcagaaactt 120
   tatgttgagt gaaagaacaa acacggagaa catactatgt ggttctcttt atgtaacatt 180
   acagaaataa aaacagaggc aaccaccttt gaggcagtat ggagtgagat agactggaaa 240
   aaggaaggaa ggaaactcta cgctgatgga aatgtctgtg tcttcattgg gtggtagtta 300
   tgtggggata tacatttgtc aaaatttatt gaactatata ctaaagaact ctgcatttta 360
   ttgggatgta aataatacct caattaaaaa gacaaaaaaa aaaaaaaaa 409
<210> 22
   <211> 649
   <212> DNA
   <213> Homo sapien
<220>
   <221> misc_feature
   <222> (1)...(649)
   <223> n = A,T,C or G
<400> 22
   acaattttca ttatcttaag cacattgtac atttctacag aacctgtgat tattctcgca 60
   tgataaggat ggtacttgca tatggtgaat tactactgtt gacagtttcc gcagaaatcc 120
   tatttcagtg gaccaacatt gtggcatggc agcaaatgcc aacattttgt ggaatagcag 180
   caaatctaca agagaccctg gttggttttt cgttttgttt tctttgtttt ttcccccttc 240
   tcctgaatca gcagggatgg aangagggta gggaagttat gaattactcc ttccagtagt 300
   agctctgaag tgtcacattt aatatcagtt ttttttaaac atgattctag ttnaatgtag 360
   aagagagaag aaagaggaag tgttcacttt tttaatacac tgatttagaa atttgatgtc 420
   ttatatcagt agttctgagg tattgatagc ttgctttatt tctgccttta cgttgacagt 480
   gttgaagcag ggtgaataac taggggcata tatatttttt ttttttgtaa gctgtttcat 540
   gatgttttct ttggaatttc cggataagtt caggaaaaca tctgcatgtt gttatctagt    600
   ctgaagttcn tatccatctc attacaacaa aaacncccag aacggnttg                649
<210> 23
   <211> 669
   <212> DNA
   <213> Homo sapien
<220>
   <221> misc_feature
   <222> (1)...(669)
   <223> n = A,T,C or G
<400> 23
   actagtgccg tactggctga aatccctgca ggaccaggaa gagaaccagt tcagactttg      60
   tactctcagt caccagctct ggaattagat aaattccttg aagatgtcag gaatgggatc     120
   tatcctctga cagcctttgg gctgcctcgg ccccagcagc cacagcagga ggaggtgaca     180
   tcacctgtcg tgcccccctc tgtcaagact ccgacacctg aaccagctga ggtggagact     240
   cgcaaggtgg tgctgatgca gtgcaacatt gagtcggtgg aggagggagt caaacaccac     300
   ctgacacttc tgctgaagtt ggaggacaaa ctgaaccggc acctgagctg tgacctgatg     360
   ccaaatgaga atatccccga gttggcggct gagctggtgc agctgggctt cattagtgag     420
   gctgaccaga gccggttgac ttctctgcta gaagagactt gaacaagttc aattttgcca     480
   ggaacagtac cctcaactca gccgctgtca ccgtctcctc ttagagctca ctcgggccag     540
   gccctgatct gcgctgtggc tgtcctggac gtgctgcacc ctctgtcctt ccccccagtc     600
   agtattacct gtgaagccct tccctccttt attattcagg anggctgggg gggctccttg     660
   nttctaacc                                                            669
<210> 24
   <211> 442
   <212> DNA
   <213> Homo sapien
<400> 24 <210> 25
   <211> 656
   <212> DNA
   <213> Homo sapien
<220>
   <221> misc_feature
   <222> (1)...(656)
   <223> n = A,T,C or G
<400> 25
<210> 26
   <211> 434
   <212> DNA
   <213> Homo sapien
<220>
   <221> misc_feature
   <222> (1)...(434)
   <223> n = A,T,C or G
<400> 26
   actagttcag actgccacgc caaccccaga aaatacccca catgccagaa aagtgaagtc      60
   ctaggtgttt ccatctatgt ttcaatctgt ccatctacca ggcctcgcga taaaaacaaa     120
   acaaaaaaac gctgccaggt tttagaagca gttctggtct caaaaccatc aggatcctgc     180
   caccagggtt cttttgaaat agtaccacat gtaaaaggga atttggcttt cacttcatct     240
   aataactgaa ttgtcaggct ttgattgata attgtagaaa taagtagcct tctgttgtgg     300
   gaataagtta taatcagtat tcatctcttt gttttttgtc actcttttct ctctaattgt      360
   gtcatttgta ctgtttgaaa aatatttctt ctatnaaatt aaactaacct gccttaaaaa     420
   aaaaaaaaaa aaaa                                                       434
<210> 27
   <211> 654
   <212> DNA
   <213> Homo sapien
<220>
   <221> misc_feature
   <222> (1)...(654)
   <223> n = A,T,C or G
<400> 27
   actagtccaa cacagtcaga aacattgttt tgaatcctct gtaaaccaag gcattaatct      60
   taataaacca ggatccattt aggtaccact tgatataaaa aggatatcca taatgaatat     120
   tttatactgc atcctttaca ttagccacta aatacgttat tgcttgatga agacctttca     180
   cagaatccta tggattgcag catttcactt ggctacttca tacccatgcc ttaaagaggg     240
   gcagtttctc aaaagcagaa acatgccgcc agttctcaag ttttcctcct aactccattt     300
   gaatgtaagg gcagctggcc cccaatgtgg ggaggtccga acattttctg aattcccatt     360
   ttcttgttcg cggctaaatg acagtttctg tcattactta gattccgatc tttcccaaag     420
   gtgttgattt acaaagaggc cagctaatag cagaaatcat gaccctgaaa gagagatgaa     480
   attcaagctg tgagccaggc agganctcag tatggcaaag gtcttgagaa tcngccattt     540
   ggtacaaaaa aaattttaaa gcntttatgt tataccatgg aaccatagaa anggcaaggg     600
   aattgttaag aanaatttta agtgtccaga cccanaanga aaaaaaaaaa aaaa           654
<210> 28
   <211> 670
   <212> DNA
   <213> Homo sapien
<220>
   <221> misc_feature
   <222> (1) ... (670)
   <223> n = A,T,C or G
<400> 28
   cgtgtgcaca tactgggagg atttccacag ctgcacggtc acagccctta cggattgcca      60
   ggaaggggcg aaagatatgt gggataaact gagaaaagaa nccaaaaacc tcaacatcca     120
   aggcagctta ttcgaactct gcggcagcgg caacggggcg gcggggtccc tgctcccggc     180
   gttcccggtg ctcctggtgt ctctctcggc agctttagcg acctgncttt ccttctgagc     240
   gtggggccag ctccccccgc ggcgcccacc cacnctcact ccatgctccc ggaaatcgag     300
   aggaagatca ttagttcttt ggggacgttn gtgattctct gtgatgctga aaaacactca     360
   tatagggaat gtgggaaatc ctganctctt tnttatntcg tntgatttct tgtgttttat     420
   ttgccaaaat gttaccaatc agtgaccaac cnagcacagc caaaaatcgg acntcngctt     480
   tagtccgtct tcacacacag aataagaaaa cggcaaaccc accccacttt tnantttnat     540
   tattactaan ttttttctgt tgggcaaaag aatctcagga acngccctgg ggccnccgta     600
   ctanagttaa ccnagctagt tncatgaaaa atgatgggct ccncctcaat gggaaagcca     660
   agaaaaagnc                                                            670
<210> 29
   <211> 551
   <212> DNA
   <213> Homo sapien
<220>
   <221> misc_feature
   <222> (1)...(551)
   <223> n = A,T,C or G
<400> 29
   actagtcctc cacagcctgt gaatccccct agacctttca agcatagtga gcggagaaga      60
   agatctcagc gtttagccac cttacccatg cctgatgatt ctgtagaaaa ggtttcttct     120
   ccctctccag ccactgatgg gaaagtattc tccatcagtt ctcaaaatca gcaagaatct     180
   tcagtaccag aggtgcctga tgttgcacac ttgccacttg agaagctggg accctgtctc     240
   cctcttgact taagtcgtgg ttcagaagtt acagcaccgg tagcctcaga ttcctcttac     300
   cgtaatgaat gtcccagggc agaaaaagag gatacncaga tgcttccaaa tccttcttcc     360
   aaagcaatag ctgatgggaa gaggagctcc agcagcagca ggaatatcga aaacagaaaa     420
   aaaagtgaaa ttgggaagac aaaagctcaa cagcatttgg taaggagaaa aganaagatg     480
   aggaaggaag agagaagaga gacnaagatc nctacggacc gnnncggaag aagaagaagn     540
   aaaaaanaaa a                                                          551
<210> 30
   <211> 684
   <212> DNA
   <213> Homo sapien
<220>
   <221> misc_feature
   <222> (1)...(684)
   <223> n = A,T,C or G
<400> 30
   actagttcta tctggaaaaa gcccgggttg gaagaagctg tggagagtgc gtgtgcaatg      60
   cgagactcat ttcttggaag catccctggc aaaaatgcag ctgagtacaa ggttatcact     120
   gtgatagaac ctggactgct ttttgagata atagagatgc tgcagtctga agagacttcc     180
   agcacctctc agttgaatga attaatgatg gcttctgagt caactttact ggctcaggaa     240
   ccacgagaga tgactgcaga tgtaatcgag cttaaaggga aattcctcat caacttagaa     300
   ggtggtgata ttcgtgaaga gtcttcctat aaagtaattg tcatgccgac tacgaaagaa     360
   aaatgccccc gttgttggaa gtatacagcg ggagtcttca gatacactgt gtcctcgatg     420
   tgcagaagtt gtcagtggga aaatagtatt aacagctcac tcgagcaaga accctcctga     480
   cagtactggg ctagaagttt ggatggatta tttacaatat aggaaagaaa gccaagaatt     540
   aggtnatgag tggatgagta aatggtggan gatggggaat tcaaatcaga attatggaag     600
   aagttnttcc tgttactata gaaaggaatt atgtttattt acatgcagaa aatatanatg     660
   tgtggtgtgt accgtggatg gaan                                            684
<210> 31
   <211> 654
   <212> DNA
   <213> Homo sapien
<220>
   <221> misc_feature
   <222> (1)...(654)
   <223> n = A,T,C or G
<400> 31
   gcgcagaaaa ggaaccaata tttcagaaac aagcttaata ggaacagctg cctgtacatc      60
   aacatcttct cagaatgacc cagaagttat catcgtggga gctggcgtgc ttggctctgc     120
   tttggcagct gtgctttcca gagatggaag aaaggtgaca gtcattgaga gagacttaaa     180
   agagcctgac agaatagttg gagaattcct gcagccgggt ggttatcatg ttctcaaaga     240
   ccttggtctt ggagatacag tggaaggtct tgatgcccag gttgtaaatg gttacatgat     300
   tcatgatcag ggaaagcaaa tcagangttc agattcctta ccctctgtca gaaaacaatc     360
   aagtgcagag tggaagagct ttccatcacg gaagattcat catgagtctc cggaaagcag     420
   ctatggcaga gcccaatgca aagtttattg aaggtgttgt gttacagtta ttagaggaag     480
   atgatgttgt gatgggagtt cagtacaagg ataaagagac tgggagatat caaggaactc     540
   catgctccac tgactgttgt tgcagatggg cttttctcca anttcaggaa aagcctggtc     600
   tcaataaagt ttctgtatca ctcatttggt tggcttctta tgaagaatgc nccc           654
<210> 32
   <211> 673
   <212> DNA
   <213> Homo sapien
<220>
   <221> misc_feature
   <222> (1)...(673)
   <223> n = A,T,C or G
<400> 32
   actagtgaag aaaaagaaat tctgatacgg gacaaaaatg ctcttcaaaa catcattctt      60
   tatcacctga caccaggagt tttcattgga aaaggatttg aacctggtgt tactaacatt     120
   ttaaagacca cacaaggaag caaaatcttt ctgaaagaag taaatgatac acttctggtg     180
   aatgaattga aatcaaaaga atctgacatc atgacaacaa atggtgtaat tcatgttgta     240
   gataaactcc tctatccagc agacacacct gttggaaatg atcaactgct ggaaatactt     300
   aataaattaa tcaaatacat ccaaattaag tttgttcgtg gtagcacctt caaagaaatc     360
   cccgtgactg tctatnagcc aattattaaa aaatacacca aaatcattga tgggagtgcc     420
   tgtgggaaat aactgaaaaa gagaccgaga agaacgaatc attacaggtc ctgaaataaa     480
   atacctagga tttctactgg aggtggagaa acagaagaac tctgaagaaa ttgttacaag     540
   aagangtccc aaggtcacca aattcattga aggtggtgat ggtctttatt tgaagatgaa     600
   gaaattaaaa gacgcttcag ggagacnccc catgaaggaa ttgccagcca caaaaaaatt     660
   cagggattag aaa                                                        673
<210> 33
   <211> 673
   <212> DNA
   <213> Homo sapien
<220>
   <221> misc_feature
   <222> (1)...(673)
   <223> n = A,T,C or G
<400> 33
   actagttatt tactttcctc cgcttcagaa ggtttttcag actgagagcc taagcatact      60
   ggatctgttg tttcttttgg gtctcacctc atcagtgtgc atagtggcag aaattataaa     120
   gaaggttgaa aggagcaggg aaaagatcca gaagcatgtt agttcgacat catcatcttt     180
   tcttgaagta tgatgcatat tgcattattt tatttgcaaa ctaggaattg cagtctgagg     240
   atcatttaga agggcaagtt caagaggata tgaagatttg agaacttttt aactattcat     300
   tgactaaaaa tgaacattaa tgttnaagac ttaagacttt aacctgctgg cagtcccaaa     360
   tgaaattatg caactttgat atcatattcc ttgatttaaa ttgggctttt gtgattgant     420
   gaaactttat aaagcatatg gtcagttatt tnattaaaaa ggcaaaacct gaaccacctt     480
   ctgcacttaa agaagtctaa cagtacaaat acctatctat cttagatgga tntatttntt     540
   tntattttta aatattgtac tatttatggt nggtggggct ttcttactaa tacacaaatn     600
   aatttatcat ttcaanggca ttctatttgg gtttagaagt tgattccaag nantgcatat     660
   ttcgctactg tnt                                                        673
<210> 34
   <211> 684
   <212> DNA
   <213> Homo sapien
<220>
   <221> misc_feature
   <222> (1)...(684)
   <223> n = A,T,C or G
<400> 34
   actagtttat tcaagaaaag aacttactga ttcctctgtt cctaaagcaa gagtggcagg 60
   tgatcagggc tggtgtagca tccggttcct ttagtgcagc taactgcatt tgtcactgat 120
   gaccaaggag gaaatcacta agacatttga gaagcagtgg tatgaacgtt cttggacaag 180
   ccacagttct gagccttaac cctgtagttt gcacacaaga acgagctcca cctccccttc 240
   ttcaggagga atctgtgcgg atagattggc tggacttttc aatggttctg ggttgcaagt 300
   gggcactgtt atggctgggt atggagcgga cagccccagg aatcagagcc tcagcccggc 360
   tgcctggttg gaaggtacag gtgttcagca ccttcggaaa aagggcataa agtngtgggg 420
   gacaattctc agtccaagaa gaatgcattg accattgctg gctatttgct tncctagtan 480
   gaattggatn catttttgac cangatnntt ctnctatgct ttnttgcaat gaaatcaaat 540
   cccgcattat ctacaagtgg tatgaagtcc tgcnnccccc agagaggctg ttcaggcnat 600
   gtcttccaag ggcagggtgg gttacaccat tttacctccc ctctcccccc agattatgna 660
   cncagaagga atttntttcc tccc 684
<210> 35
   <211> 614
   <212> DNA
   <213> Homo sapien
<220>
   <221> misc_feature
   <222> (1)...(614)
   <223> n = A,T,C or G
<400> 35
   actagtccaa cgcgttngcn aatattcccc tggtagccta cttccttacc cccgaatatt 60
   ggtaagatcg agcaatggct tcaggacatg ggttctcttc tcctgtgatc attcaagtgc 120
   tcactgcatg aagactggct tgtctcagtg tntcaacctc accagggctg tctcttggtc 180
   cacacctcgc tccctgttag tgccgtatga cagcccccat canatgacct tggccaagtc 240
   acggtttctc tgtggtcaat gttggtnggc tgattggtgg aaagtanggt ggaccaaagg 300
   aagncncgtg agcagncanc nccagttctg caccagcagc gcctccgtcc tactngggtg 360
   ttccngtttc tcctggccct gngtgggcta nggcctgatt cgggaanatg cctttgcang 420
   gaaggganga taantgggat ctaccaattg attctggcaa aacnatntct aagattnttn 480
   tgctttatgt ggganacana tctanctctc atttnntgct gnanatnaca ccctactcgt 540
   gntcgancnc gtcttcgatt ttcgganaca cnccantnaa tactggcgtt ctgttgttaa 600
   aaaaaaaaaa aaaa 614
<210> 36
   <211> 686
   <212> DNA
   <213> Homo sapien
<220>
   <221> misc_feature
   <222> (1)...(686)
   <223> n = A,T,C or G
<400> 36
   gtggctggcc cggttctccg cttctcccca tcccctactt tcctccctcc ctccctttcc      60
   ctccctcgtc gactgttgct tgctggtcgc agactccctg acccctccct cacccctccc     120
   taacctcggt gccaccggat tgcccttctt ttcctgttgc ccagcccagc cctagtgtca      180
   gggcgggggc ctggagcagc ccgaggcact gcagcagaag ananaaaaga cacgacnaac      240
   ctcagctcgc cagtccggtc gctngcttcc cgccgcatgg caatnagaca gacgccgctc      300
   acctgctctg ggcacacgcg acccgtggtt gatttggcct tcagtggcat cacccttatg     360
   ggtatttctt aatcagcgct tgcaaagatg gttaacctat gctacgccag ggagatacag      420
   gagactggat tggaacattt ttggggtcta aaggtctgtt tggggtgcaa cactgaataa      480
   ggatgccacc aaagcagcta cagcagctgc agatttcaca gcccaagtgt gggatgctgt      540
   ctcagganat naattgataa cctggctcat aacacattgt caagaatgtg gatttcccca      600
   ggatattatt atttgtttac cggggganag gataactgtt tcncntattt taattgaaca      660
   aactnaaaca aaanctaagg aaatcc                                           686
<210> 37
   <211> 681
   <212> DNA
   <213> Homo sapien
<220>
   <221> misc_feature
   <222> (1)...(681)
   <223> n = A,T,C or G
<400> 37
   gagacanacn naacgtcang agaanaaaag angcatggaa cacaanccag gcncgatggc       60
   caccttccca ccagcancca gcgcccccca gcngccccca ngnccggang accangactc      120
   cancctgnat caatctganc tctattcctg gcccatncct acctcggagg tggangccgn      180
   aaaggtcgca cnnncagaga agctgctgcc ancaccancc gccccnnccc tgncgggctn      240
   nataggaaac tggtgaccnn gctgcanaat tcatacagga gcacgcgang ggcacnnnct      300
   cacactgagt tnnngatgan gcctnaccan ggacctnccc cagcnnattg annacnggac      360
   tgcggaggaa ggaagacccc gnacnggatc ctggccggcn tgccaccccc ccacccctag      420
   gattatnccc cttgactgag tctctgaggg gctacccgaa cccgcctcca ttccctacca      480
   natnntgctc natcgggact gacangctgg ggatnggagg ggctatcccc cancatcccc      540
   tnanaccaac agcnacngan natnggggct ccccngggtc ggngcaacnc tcctncaccc      600
   cggcgcnggc cttcggtgnt gtcctccntc aacnaattcc naaanggcgg gccccccngt       660
   ggactcctcn ttgttccctc c                                                681
<210> 38
   <211> 687
   <212> DNA
   <213> Homo sapien
<220>
   <221> misc_feature
   <222> (1)...(687)
   <223> n = A,T,C or G
<400> 38
   canaaaaaaa aaaacatggc cgaaaccagn aagctgcgcg atggcgccac ggcccctctt 60
   ctcccggcct gtgtccggaa ggtttccctc cgaggcgccc cggctcccgc aagcggagga 120
   gagggcggga cntgccgggg ccggagctca naggccctgg ggccgctctg ctctcccgcc 180
   atcgcaaggg cggcgctaac ctnaggcctc cccgcaaagg tccccnangc ggnggcggcg 240
   gggggctgtg anaaccgcaa aaanaacgct gggcgcgcng cgaacccgtc cacccccgcg 300
   aaggananac ttccacagan gcagcgtttc cacagcccan agccacnttt ctagggtgat 360
   gcaccccagt aagttcctgn cggggaagct caccgctgtc aaaaaanctc ttcgctccac 420
   cggcgcacna aggggangan ggcangangc tgccgcccgc acaggtcatc tgatcacgtc 480
   gcccgcccta ntctgctttt gtgaatctcc actttgttca accccacccg ccgttctctc 540
   ctccttgcgc cttcctctna ccttaanaac cagcttcctc tacccnatng tanttnctct 600
   gcncnngtng aaattaattc ggtccnccgg aacctcttnc ctgtggcaac tgctnaaaga 660
   aactgctgtt ctgnttactg cngtccc 687
<210> 39
   <211> 695
   <212> DNA
   <213> Homo sapien
<220>
   <221> misc_feature
   <222> (1)...(695)
   <223> n = A,T,C or G
<400> 39
   actagtctgg cctacaatag tgtgattcat gtaggacttc tttcatcaat tcaaaacccc 60
   tagaaaaacg tatacagatt atataagtag ggataagatt tctaacattt ctgggctctc 120
   tgacccctgc gctagactgt ggaaagggag tattattata gtatacaaca ctgctgttgc 180
   cttattagtt ataacatgat aggtgctgaa ttgtgattca caatttaaaa acactgtaat 240
   ccaaactttt ttttttaact gtagatcatg catgtgaatg ttaatgttaa tttgttcaan 300
   gttgttatgg gtagaaaaaa ccacatgcct taaaatttta aaaagcaggg cccaaactta 360
   ttagtttaaa attaggggta tgtttccagt ttgttattaa ntggttatag ctctgtttag 420
   aanaaatcna ngaacangat ttngaaantt aagntgacat tatttnccag tgacttgtta 480
   atttgaaatc anacacggca ccttccgttt tggtnctatt ggnntttgaa tccaancngg 540
   ntccaaatct tnttggaaac ngtccnttta acttttttac nanatcttat ttttttattt 600
   tggaatggcc ctatttaang ttaaaagggg ggggnnccac naccattcnt gaataaaact 660
   naatatatat ccttggtccc ccaaaattta aggng 695
<210> 40
   <211> 674
   <212> DNA
   <213> Homo sapien
<220>
   <221> misc_feature
   <222> (1)...(674)
   <223> n = A,T,C or G
<400> 40
   actagtagtc agttgggagt ggttgctata ccttgacttc atttatatga atttccactt 60
   tattaaataa tagaaaagaa aatcccggtg cttgcagtag agttatagga cattctatgc 120
   ttacagaaaa tatagccatg attgaaatca aatagtaaag gctgttctgg ctttttatct 180
   tcttagctca tcttaaataa gtagtacact tgggatgcag tgcgtctgaa gtgctaatca 240
   gttgtaacaa tagcacaaat cgaacttagg atgtgtttct tctcttctgt gtttcgattt 300
   tgatcaattc tttaattttg ggaacctata atacagtttt cctattcttg gagataaaaa 360
   ttaaatggat cactgatatt taagtcattc tgcttctcat ctnaatattc catattctgt 420
   attagganaa antacctccc agcacagccc cctctcaaac cccacccaaa accaagcatt 480
   tggaatgagt ctcctttatt tccgaantgt ggatggtata acccatatcn ctccaatttc 540
   tgnttgggtt gggtattaat ttgaactgtg catgaaaagn ggnaatcttt nctttgggtc 600
   aaantttncc ggttaatttg nctngncaaa tccaatttnc tttaagggtg tctttataaa 660
   atttgctatt cngg 674
<210> 41
   <211> 657
   <212> DNA
   <213> Homo sapien
<220>
   <221> misc_feature
   <222> (1)...(657)
   <223> n = A,T,C or G
<400> 41
   gaaacatgca agtaccacac actgtttgaa ttttgcacaa aaagtgactg tagggatcag 60
   gtgatagccc cggaatgtac agtgtcttgg tgcaccaaga tgccttctaa aggctgacat 120
   accttgggac cctaatgggg cagagagtat agccctagcc cagtggtgac atgaccactc 180
   cctttgggag gctgaagtta aagggaatgg tatgtgtttt ctcatggaag cagcacatga 240
   atnggtnaca ngatgttaaa ntaaggntct antttgggtg tcttgtcatt tgaaaaantg 300
   acacactcct ancanctggt aaaggggtgc tggaagccat ggaagaactc taaaaacatt 360
   agcatgggct gatctgatta cttcctggca tcccgctcac ttttatggga agtcttatta 420
   naaggatggg ananttttcc atatccttgc tgttggaact ctggaacact ctctaaattt 480
   ccctctatta aaaatcactg nccttactac acttcctcct tganggaata gaaatggacc 540
   tttctctgac ttagttcttg gcatggganc cagcccaaat taaaatctga cttntccggt 600
   ttctccngaa ctcacctact tgaattggta aaacctcctt tggaattagn aaaaacc 657
<210> 42
   <211> 389
   <212> DNA
   <213> Homo sapien
<220>
   <221> misc_feature
   <222> (1)...(389)
   <223> n = A,T,C or G
<400> 42
   actagtgctg aggaatgtaa acaagtttgc tgggccttgc gagacttcac caggttgttt 60
   cgatagctca cactcctgca ctgtgcctgt cacccaggaa tgtctttttt aattagaaga 120
   caggaagaaa acaaaaacca gactgtgtcc cacaatcaga aacctccgtt gtggcagang 180
   ggccttcacc gccaccaggg tgtcccgcca gacagggaga gactccagcc ttctgaggcc 240
   atcctgaaga attcctgttt gggggttgtg aaggaaaatc acccggattt aaaaagatgc 300
   tgttgcctgc ccgcgtngtn gggaagggac tggtttcctg gtgaatttct taaaagaaaa 360
   atattttaag ttaagaaaaa aaaaaaaaa 389
<210> 43
   <211> 279
   <212> DNA
   <213> Homo sapien
<400> 43
   actagtgaca agctcctggt cttgagatgt cttctcgtta aggagatggg ccttttggag      60
   gtaaaggata aaatgaatga gttctgtcat gattcactat tctagaactt gcatgacctt     120
   tactgtgtta gctctttgaa tgttcttgaa attttagact ttctttgtaa acaaataata     180
   tgtccttatc attgtataaa agctgttatg tgcaacagtg tggagatcct tgtctgattt     240
   aataaaatac ttaaacactg aaaaaaaaaa aaaaaaaaa                            279
<210> 44
   <211> 449
   <212> DNA
   <213> Homo sapien
<220>
   <221> misc_feature
   <222> (1)...(449)
   <223> n = A,T,C or G
<400> 4
<210> 45
   <211> 559
   <212> DNA
   <213> Homo sapien
<220>
   <221> misc_feature
   <222> (1)...(559)
   <223> n = A,T,C or G
<400> 45
   actagtgtgg gggaatcacg gacacttaaa gtcaatctgc gaaataattc ttttattaca      60
   cactcactga agtttttgag tcccagagag ccattctatg tcaaacattc caagtactct     120
   ttgagagccc agcattacat caacatgccc gtgcagttca aaccgaagtc cgcaggcaaa     180
   tttgaagctt tgcttgtcat tcaaacagat gaaggcaaga gtattgctat tcgactaatt     240
   ggtgaagctc ttggaaaaaa ttnactagaa tactttttgt gttaagttaa ttacataagt     300
   tgtattttgt taactttatc tttctacact acaattatgc ttttgtatat atattttgta     360
   tgatggatat ctataattgt agattttgtt tttacaagct aatactgaag actcgactga     420
   aatattatgt atctagccca tagtattgta cttaactttt acagggtgaa aaaaaaattc     480
   tgtgtttgca ttgattatga tattctgaat aaatatggga atatatttta atgtgggtaa     540
   aaaaaaaaaa aaaaaggaa                                                  559
<210> 46
   <211> 731
   <212> DNA
   <213> Homo sapien
<220>
   <221> misc_feature
   <222> (1)...(731)
   <223> n = A,T, C or G
<400> 46
   actagttcta gtaccatggc tgtcatagat gcaaccatta tattccattt agtttcttcc   60
   tcaggttccc taacaattgt ttgaaactga atatatatgt ttatgtatgt gtgtgtgttt   120
   actgtcatgt atatggtgta tatgggatgt gtgcagtttt cagttatata tatattcata   180
   tatacatatg catatatatg tataatatac atatatacat gcatacactt gtataatata   240
   catatatata cacatatatg cacacatatn atcactgagt tccaaagtga gtctttattt   300
   ggggcaattg tattctctcc ctctgtctgc tcactgggcc tttgcaagac atagcaattg   360
   cttgatttcc tttggataag agtcttatct tcggcactct tgactctagc cttaacttta   420
   gatttctatt ccagaatacc tctcatatct atcttaaaac ctaaganggg taaagangtc   480
   ataagattgt agtatgaaag antttgctta gttaaattat atctcaggaa actcattcat   540
   ctacaaatta aattgtaaaa tgatggtttg ttgtatctga aaaaatgttt agaacaagaa   600
   atgtaactgg gtacctgtta tatcaaagaa cctcnattta ttaagtctcc tcatagccan   660
   atccttatat ngccctctct gacctgantt aatananact tgaataatga atagttaatt   720
   taggnttgggc                                                          731
<210> 47
   <211> 640
   <212> DNA
   <213> Homo sapien
<220>
   <221> misc_feature
   <222> (1)...(640)
   <223> n = A,T,C or G
<400> 47
   tgcgngccgg tttggccctt ctttgtanga cactttcatc cgccctgaaa tcttcccgat   60
   cgttaataac tcctcaggtc cctgcctgca cagggttttt tcttantttg ttgcctaaca   120
   gtacaccaaa tgtgacatcc tttcaccaat atngattnct tcataccaca tcntcnatgg   180
   anacgactnc aacaattttt tgatnacccn aaanactggg ggctnnaana agtacantct   240
   ggagcagcat ggacctgtcn gcnactaang gaacaanagt nntgaacatt tacacaacct   300
   ttggtatgtc ttactgaaag anagaaacat gcttctnncc ctagaccacg aggncaaccg   360
   caganattgc caatgccaag tccgagcggt tagatcaggt aatacattcc atggatgcat   420
   tacatacntt gtccccgaaa nanaagatgc cctaanggct tcttcanact ggtccngaaa   480
   acanctacac ctggtgcttg ganaacanac tctttggaag atcatctggc acaagttccc   540
   cccagtgggt tttnccttgg cacctanctt accanatcna ttcggaancc attctttgcc   600
   ntggcnttnt nttgggacca ntcttctcac aactgnaccc                         640
<210> 48
   <211> 257
   <212> DNA
   <213> Homo sapien
<400> 48
   actagtatat gaaaatgtaa atatcacttg tgtactcaaa caaaagttgg tcttaagctt      60
   ccaccttgag cagccttgga aacctaacct gcctctttta gcataatcac attttctaaa     120
   tgattttctt tgttcctgaa aaagtgattt gtattagttt tacatttgtt ttttggaaga     180
   ttatatttgt atatgtatca tcataaaata tttaaataaa aagtatcttt agagtgaaaa     240
   aaaaaaaaaa aaaaaaa                                                    257
<210> 49
   <211> 652
   <212> DNA
   <213> Homo sapien
<220>
   <221> misc_feature
   <222> (1)...(652)
   <223> n = A,T,C or G
<400> 49
   actagttcag atgagtggct gctgaagggg cccccttgtc attttcatta taacccaatt      60
   tccacttatt tgaactctta agtcataaat gtataatgac ttatgaatta gcacagttaa     120
   gttgacacta gaaactgccc atttctgtat tacactatca aataggaaac attggaaaga     180
   tggggaaaaa aatcttattt taaaatggct tagaaagttt tcagattact ttgaaaattc     240
   taaacttctt tctgtttcca aaacttgaaa atatgtagat ggactcatgc attaagactg     300
   ttttcaaagc tttcctcaca tttttaaagt gtgattttcc ttttaatata catatttatt     360
   ttctttaaag cagctatatc ccaacccatg actttggaga tatacctatn aaaccaatat     420
   aacagcangg ttattgaagc agctttctca aatgttgctt cagatgtgca agttgcaaat     480
   tttattgtat ttgtanaata caatttttgt tttaaactgt atttcaatct atttctccaa     540
   gatgcttttc atatagagtg aaatatccca ngataactgc ttctgtgtcg tcgcatttga     600
   cgcataactg cacaaatgaa cagtgtatac ctcttggttg tgcattnacc cc             652
<210> 50
   <211> 650
   <212> DNA
   <213> Homo sapien
<220>
   <221> misc_feature
   <222> (1)...(650)
   <223> n = A,T,C or G
<400> 50
   ttgcgctttg atttttttag ggcttgtgcc ctgtttcact tatagggtct agaatgcttg      60
   tgttgagtaa aaaggagatg cccaatattc aaagctgcta aatgttctct ttgccataaa     120
   gactccgtgt aactgtgtga acacttggga tttttctcct ctgtcccgag gtcgtcgtct     180
   gctttctttt ttgggttctt tctagaagat tgagaaatgc atatgacagg ctgagancac     240
   ctccccaaac acacaagctc tcagccacan gcagcttctc cacagcccca gcttcgcaca     300
   ggctcctgga nggctgcctg ggggaggcag acatgggagt gccaaggtgg ccagatggtt     360
   ccaggactac aatgtcttta tttttaactg tttgccactg ctgccctcac ccctgcccgg     420
   ctctggagta ccgtctgccc canacaagtg ggantgaaat gggggtgggg gggaacactg     480
   attcccantt agggggtgcc taactgaaca gtagggatan aaggtgtgaa cctgngaant     540
   gcttttataa attatnttcc ttgttanatt tattttttaa tttaatctct gttnaactgc     600
   ccngggaaaa ggggaaaaaa aaaaaaaaat tctntttaaa cacatgaaca                650
<210> 51
   <211> 545
   <212> DNA
   <213> Homo sapien
<220>
   <221> misc_feature
   <222> (1)...(545)
   <223> n = A,T,C or G
<400> 51
   tggcgtgcaa ccagggtagc tgaagtttgg gtctgggact ggagattggc cattaggcct     60
   cctganattc cagctccctt ccaccaagcc cagtcttgct acgtggcaca gggcaaacct    120
   gactcccttt gggcctcagt ttcccctccc cttcatgana tgaaaagaat actacttttt    180
   cttgttggtc taacnttgct ggacncaaag tgtngtcatt attgttgtat tgggtgatgt    240
   gtncaaaact gcagaagctc actgcctatg agaggaanta agagagatag tggatganag    300
   ggacanaagg agtcattatt tggtatagat ccacccntcc caacctttct ctcctcagtc    360
   cctgcncctc atgtntctgg tntggtgagt cctttgtgcc accanccatc atgctttgca    420
   ttgctgccat cctgggaagg gggtgnatcg tctcacaact tgttgtcatc gtttganatg    480
   catgctttct tnatnaaaca aanaaannaa tgtttgacag ngtttaaaat aaaaaanaaa    540
   caaaa                                                                545
<210> 52
   <211> 678
   <212> DNA
   <213> Homo sapien
<220>
   <221> misc_feature
   <222> (1)...(678)
   <223> n = A,T,C or G
<400> 52
   actagtagaa gaactttgcc gcttttgtgc ctctcacagg cgcctaaagt cattgccatg     60
   ggaggaagac gatttggggg gggagggggg gggggcangg tccgtggggc tttccctant    120
   ntatctccat ntccantgnn cnntgtcgcc tcttccctcg tcncattnga anttantccc    180
   tggnccccnn nccctctccn ncctncncct cccccctccg ncncctccnn ctttttntan    240
   ncttccccat ctccntcccc cctnanngtc ccaacnccgn cagcaatnnc ncacttnctc    300
   nctccncncc tccnnccgtt cttctnttct cnacntntnc ncnnntnccn tgccnntnaa    360
   annctctccc cnctgcaanc gattctctcc ctccncnnan ctntccactc cntncttctc    420
   ncncgctcct nttcntcnnc ccacctctcn ccttcgnccc cantacnctc nccncccttn    480
   cgnntcnttn nnntcctcnn accncccncc tcccttcncc cctcttctcc ccggtntntc    540
   tctctcccnc nncncnncct cnncccntcc nngcgnccnt ttccgccccn cnccnccntt    600
   ccttcntcnc cantccatcn cntntnccat nctncctncc nctcacnccc gctncccccn    660
   ntctctttca cacngtcc                                                  678
<210> 53
   <211> 502
   <212> DNA
   <213> Homo sapien
<220>
   <221> misc_feature
   <222> (1)...(502)
   <223> n = A,T,C or G
<400> 53
   tgaagatcct ggtgtcgcca tgggccgccg ccccgcccgt tgttaccggt attgtaagaa      60
   caagccgtac ccaaagtctc gcttctgccg aggtgtccct gatgccaaaa ttcgcatttt     120
   tgacctgggg cggaaaaang caaaantgga tgagtctccg ctttgtggcc acatggtgtc     180
   agatcaatat gagcagctgt cctctgaagc cctgnangct gcccgaattt gtgccaataa     240
   gtacatggta aaaagtngtg gcnaagatgc ttccatatcc gggtgcggnt ccaccccttc     300
   cacgtcatcc gcatcaacaa gatgttgtcc tgtgctgggg ctgacaggct cccaacaggc     360
   atgcgaagtg cctttggaaa acccanggca ctgtggccag ggttcacatt gggccaattn     420
   atcatgttca tccgcaccaa ctgcagaaca angaacntgt naattnaagc cctgcccagg     480
   gncaanttca aatttcccgg cc                                              502
<210> 54
   <211> 494
   <212> DNA
   <213> Homo sapien
<220>
   <221> misc_feature
   <222> (1)...(494)
   <223> n = A,T,C or G
<400> 54
   actagtccaa gaaaaatatg cttaatgtat attacaaagg ctttgtatat gttaacctgt      60
   tttaatgcca aaagtttgct ttgtccacaa tttccttaag acctcttcag aaagggattt     120
   gtttgcctta atgaatactg ttgggaaaaa acacagtata atgagtgaaa agggcagaag     180
   caagaaattt ctacatctta gcgactccaa gaagaatgag tatccacatt tagatggcac     240
   attatgagga ctttaatctt tccttaaaca caaaaatgtt ttcttttttc ttttatccac     300
   atgatttcta agtatatttt tcatgcagga cagtttttca accttgatgt acagtgactg     360
   tgttaaattt ttctttcagt ggcaacctct ataatcttta aaatatggtg agcatcttgt     420
   ctgttttgaa ngggatatga cnatnaatct atcagatggg aaatcctgtt tccaagttag      480
   aaaaaaaaaa aaaa                                                       494
<210> 55
   <211> 606
   <212> DNA
   <213> Homo sapien
<220>
   <221> misc_feature
   <222> (1)...(606)
   <223> n = A,T,C or G
<400> 55
   actagtaaaa agcagcattg ccaaataatc cctaattttc cactaaaaat ataatgaaat      60
   gatgttaagc tttttgaaaa gtttaggtta aacctactgt tgttagatta atgtatttgt     120
   tgcttccctt tatctggaat gtggcattag cttttttatt ttaaccctct ttaattctta   180
   ttcaattcca tgacttaagg ttggagagct aaacactggg atttttggat aacagactga     240
   cagttttgca taattataat cggcattgta catagaaagg atatggctac cttttgttaa     300
   atctgcactt tctaaatatc aaaaaaggga aatgaagtat aaatcaattt ttgtataatc     360
   tgtttgaaac atgantttta tttgcttaat attanggctt tgcccttttc tgttagtctc    420
   ttgggatcct gtgtaaaact gttctcatta aacaccaaac agttaagtcc attctctggt     480
   actagctaca aattccgttt catattctac ntaacaattt aaattaactg aaatatttct     540
   anatggtcta cttctgtcnt ataaaaacna aacttgantt nccaaaaaaa aaaaaaaaaa     600
   aaaaaa                                                                606
<210> 56
   <211> 183
   <212> DNA
   <213> Homo sapien
<400> 56
   actagtatat ttaaacttac aggcttattt gtaatgtaaa ccaccatttt aatgtactgt      60
   aattaacatg gttataatac gtacaatcct tccctcatcc catcacacaa ctttttttgt      120
   gtgtgataaa ctgattttgg tttgcaataa aaccttgaaa aataaaaaaa aaaaaaaaaa      180
   aaa                                                                   183
<210> 57
   <211> 622
   <212> DNA
   <213> Homo sapien
<220>
   <221> misc_feature
   <222> (1)...(622)
   <223> n = A,T,C or G
<400> 57
   actagtcact actgtcttct ccttgtagct aatcaatcaa tattcttccc ttgcctgtgg      60
   gcagtggaga gtgctgctgg gtgtacgctg cacctgccca ctgagttggg gaaagaggat     120
   aatcagtgag cactgttctg ctcagagctc ctgatctacc ccacccccta ggatccagga      180
   ctgggtcaaa gctgcatgaa accaggccct ggcagcaacc tgggaatggc tggaggtggg     240
   agagaacctg acttctcttt ccctctccct cctccaacat tactggaact ctatcctgtt     300
   agggatcttc tgagcttgtt tccctgctgg gtgggacaga agacaaagga gaagggangg     360
   tctacaanaa gcagcccttc tttgtcctct ggggttaatg agcttgacct ananttcatg     420
   gaganaccan aagcctctga tttttaattt ccntnaaatg tttgaagtnt atatntacat     480
   atatatattt ctttnaatnt ttgagtcttt gatatgtctt aaaatccant ccctctgccn     540
   gaaacctgaa ttaaaaccat gaanaaaaat gtttncctta aagatgttan taattaattg     600
   aaacttgaaa aaaaaaaaaa aa                                              622
<210> 58
   <211> 433
   <212> DNA
   <213> Homo sapien
<400> 58
   gaacaaattc tgattggtta tgtaccgtca aaagacttga agaaatttca tgattttgca      60
   gtgtggaagc gttgaaaatt gaaagttact gcttttccac ttgctcatat agtaaaggga     120
   tcctttcagc tgccagtgtt gaataatgta tcatccagag tgatgttatc tgtgacagtc     180
   accagcttta agctgaacca ttttatgaat accaaataaa tagacctctt gtactgaaaa     240
   catatttgtg actttaatcg tgctgcttgg atagaaatat ttttactggt tcttctgaat     300
   tgacagtaaa cctgtccatt atgaatggcc tactgttcta ttatttgttt tgacttgaat     360
   ttatccacca aagacttcat ttgtgtatca tcaataaagt tgtatgtttc aactgaaaaa     420
   aaaaaaaaaa aaa                                                        433
<210> 59
   <211> 649
   <212> DNA
   <213> Homo sapien
<220>
   <221> misc_feature
   <222> (1)...(649)
   <223> n = A,T,C or G
<400> 59
   actagttatt atctgacttt cnggttataa tcattctaat gagtgtgaag tagcctctgg     60
   tgtcatttgg atttgcattt ctctgatgag tgatgctatc aagcaccttt gctggtgctg    120
   ttggccatat gtgtatgttc cctggagaag tgtctgtgct gagccttggc ccacttttta    180
   attaggcgtn tgtcttttta ttactgagtt gtaaganttc tttatatatt ctggattcta   240
   gacccttatc agatacatgg tttgcaaata ttttctccca ttctgtgggt tgtgttttca    300
   ctttatcgat aatgtcctta gacatataat aaatttgtat tttaaaagtg acttgatttg    360
   ggctgtgcaa ggtgggctca cgcttgtaat cccagcactt tgggagactg aggtgggtgg    420
   atcatatgan gangctagga gttcgaggtc agcctggcca gcatagcgaa aacttgtctc    480
   tacnaaaaat acaaaaatta gtcaggcatg gtggtgcacg tctgtaatac cagcttctca    540
   ggangctgan gcacaaggat cacttgaacc ccagaangaa gangttgcag tganctgaag    600
   atcatgccag ggcaacaaaa atgagaactt gtttaaaaaa aaaaaaaaa               649
<210> 60
   <211> 423
   <212> DNA
   <213> Homo sapien
<220>
   <221> misc_feature
   <222> (1)...(423)
   <223> n = A,T,C or G
<400> 60
   actagttcag gccttccagt tcactgacaa acatggggaa gtgtgcccag ctggctggaa     60
   acctggcagt gataccatca agcctgatgt ccaaaagagc aaagaatatt tcnccaagca    120
   gaagtgagcg ctgggctgtt ttagtgccag gctgcggtgg gcagccatga gaacaaaacc    180
   tcttctgtat tttttttttc cattagtana acacaagact cngattcagc cgaattgtgg     240
   tgtcttacaa ggcagggctt tcctacaggg ggtgganaaa acagcctttc ttcccttggt    300
   aggaatggcc tgagttggcg ttgtgggcag gctactggtt tgtatgatgt attagtagag    360
   caacccatta atcttttgta gtttgtatna aacttganct gagaccttaa acaaaaaaaa     420
   aaa                                                                  423
<210> 61
   <211> 423
   <212> DNA
   <213> Homo sapien
<220>
   <221> misc_feature
   <222> (1)...(423)
   <223> n = A,T,C or G
<400> 61
   cgggactgga atgtaaagtg aagttcggag ctctgagcac gggctcttcc cgccgggtcc     60
   tccctcccca gaccccagag ggagaggccc accccgccca gccccgcccc agcccctgct    120
   caggtctgag tatggctggg agtcgggggc cacaggcctc tagctgtgct gctcaagaag    180
   actggatcag ggtanctaca agtggccggg ccttgccttt gggattctac cctgttccta 240
   atttggtgtt ggggtgcggg gtccctggcc cccttttcca cactncctcc ctccngacag 300
   caacctccct tggggcaatt gggcctggnt ctccncccgn tgttgcnacc ctttgttggt 360
   ttaaggnctt taaaaatgtt annttttccc ntgccngggt taaaaaagga aaaaactnaa 420
   aaa 423
<210> 62
   <211> 683
   <212> DNA
   <213> Homo sapien
<220>
   <221> misc_feature
   <222> (1)...(683)
   <223> n = A,T,C or G
<400> 62
   gctggagagg ggtacggact ttcttggagt tgtcccaggt tggaatgaga ctgaactcaa 60
   gaagagaccc taagagactg gggaatggtt cctgccttca ggaaagtgaa agacgcttag 120
   gctgtcaaca cttaaaggaa gtccccttga agcccagagt ggacagacta gacccattga 180
   tggggccact ggccatggtc cgtggacaag acattccngt gggccatggc acaccggggg 240
   ggatcaaaat gtgtacttgt ggggtctcgc cccttgccaa aaccaaacca ntcccactcc 300
   tgtcnttgga ctttcttccc attccctcct ccccaaatgc acttcccctc ctccctctgc 360
   ccctcctgtg tttttggaat tctgtttccc tcaaaattgt taatttttta nttttngacc 420
   atgaacttat gtttggggtc nangttcccc ttnccaatgc atactaatat attaatggtt 480
   atttattttt gaaatatttt ttaatgaact tggaaaaaat tnntggaatt tccttncttc 540
   cnttttnttt ggggggggtg gggggntggg ttaaaatttt tttggaancc cnatnggaaa 600
   ttnttacttg gggcccccct naaaaaantn anttccaatt cttnnatngc ccctnttccn 660
   ctaaaaaaaa ananannaaa aan 683
<210> 63
   <211> 731
   <212> DNA
   <213> Homo sapien
<220>
   <221> misc_feature
   <222> (1)...(731)
   <223> n = A,T,C or G
<400> 63
<210> 64
   <211> 313
   <212> DNA
   <213> Homo sapien
<220>
   <221> misc_feature
   <222> (1)...(313)
   <223> n = A,T,C or G
<400> 64
   actagttgtg caaaccacga ctgaagaaag acgaaaagtg ggaaataact tgcaacgtct 60
   gttagagatg gttgctacac atgttgggtc tgtagagaaa catcttgagg agcagattgc 120
   taaagttgat agagaatatg aagaatgcat gtcagaagat ctctcggaaa atattaaaga 180
   gattagagat aagtatgaga agaaagctac tctaattaag tcttctgaag aatgaagatn 240
   aaatgttgat catgtatata tatccatagt gaataaaatt gtctcagtaa agttgtaaaa 300
   aaaaaaaaaa aaa 313
<210> 65
   <211> 420
   <212> DNA
   <213> Homo sapien
<220>
   <221> misc_feature
   <222> (1)...(420)
   <223> n = A,T,C or G
<400> 65
   actagttccc tggcaggcaa gggcttccaa ctgaggcagt gcatgtgtgg cagagagagg 60
   caggaagctg gcaggggcag cttctgtgtc tagggagggg tgtggctccc tccttccctg 120
   tctgggaggt tggagggaag aatctaggcc ttagcttgcc ctcctgccac ccttcccctt 180
   gtagatactg ccttaacact ccctcctctc tcagctgtgg ctgccaccca agccaggttt 240
   ctccgtgctc actaatttat ttccaggaaa ggtgtgtgga agacatgagc cgtgtacaat 300
   atttgtttta acattttcat tgcaagtatt gaccatcatc cttggttgtg tatcgttgta 360
   acacaaatta atgatattaa aaagcatcca aacaaagccn annnnnaana nnannngaaa 420
<210> 66
   <211> 676
   <212> DNA
   <213> Homo sapien
<220>
   <221> misc_feature
   <222> (1)...(676)
   <223> n = A,T,C or G
<400> 66
   actagtttcc tatgatcatt aaactcattc tcagggttaa gaaaggaatg taaatttctg 60
   cctcaatttg tacttcatca ataagttttt gaagagtgca gatttttagt caggtcttaa 120
   aaataaactc acaaatctgg atgcatttct aaattctgca aatgtttcct ggggtgactt 180
   aacaaggaat aatcccacaa tatacctagc tacctaatat atggagctgg ggctcaaccc 240
   actgttttta aggatttgcg cttacttgtg gctgaggaaa aataagtagt tccgagggaa 300
   gtagttttta aatgtgagct tatagatngg aaacagaata tcaacttaat tatggaaatt 360
   gttagaaacc tgttctcttg ttatctgaat cttgattgca attactattg tactggatag 420
   actccagccc attgcaaagt ctcagatatc ttanctgtgt agttgaattc cttggaaatt     480
   ctttttaaga aaaaattgga gtttnaaaga aataaacccc tttgttaaat gaagcttggc      540
   tttttggtga aaaanaatca tcccgcaggg cttattgttt aaaaanggaa ttttaagcct      600
   ccctggaaaa anttgttaat taaatgggga aaatgntggg naaaaattat ccgttagggt     660
   ttaaagggaa aactta                                                     676
<210> 67
   <211> 620
   <212> DNA
   <213> Homo sapien
<220>
   <221> misc_feature
   <222> (1)...(620)
   <223> n = A,T,C or G
<400> 67
   caccattaaa gctgcttacc aagaacttcc ccagcatttt gacttccttg tttgatagct      60
   gaattgtgag caggtgatag aagagccttt ctagttgaac atacagataa tttgctgaat     120
   acattccatt taatgaaggg gttacatctg ttacgaagct actaagaagg agcaagagca      180
   taggggaaaa aaatctgatc agaacgcatc aaactcacat gtgccccctc tactacaaac      240
   agattgtagt gctgtggtgg tttattccgt tgtgcagaac ttgcaagctg agtcactaaa     300
   cccaaagaga ggaaattata ggttagttaa acattgtaat cccaggaact aagtttaatt     360
   cacttttgaa gtgttttgtt ttttattttt ggtttgtctg atttactttg ggggaaaang     420
   ctaaaaaaaa agggatatca atctctaatt cagtgcccac taaaagttgt ccctaaaaag     480
   tctttactgg aanttatggg actttttaag ctccaggtnt tttggtcctc caaattaacc     540
   ttgcatgggc cccttaaaat tgttgaangg cattcctgcc tctaagtttg gggaaaattc     600
   ccccnttttn aaaatttgga                                                 620
<210> 68
   <211> 551
   <212> DNA
   <213> Homo sapien
<220>
   <221> misc_feature
   <222> (1)...(551)
   <223> n = A,T,C or G
<400> 68
   actagtagct ggtacataat cactgaggag ctatttctta acatgctttt atagaccatg      60
   ctaatgctag accagtattt aagggctaat ctcacacctc cttagctgta agagtctggc     120
   ttagaacaga cctctctgtg caataacttg tggccactgg aaatccctgg gccggcattt     180
   gtattggggt tgcaatgact cccaagggcc aaaagagtta aaggcacgac tgggatttct     240
   tctgagactg tggtgaaact ccttccaagg ctgagggggt cagtangtgc tctgggaggg     300
   actcggcacc actttgatat tcaacaagcc acttgaagcc caattataaa attgttattt     360
   tacagctgat ggaactcaat ttgaaccttc aaaactttgt tagtttatcc tattatattg     420
   ttaaacctaa ttacatttgt ctagcattgg atttggttcc tgtngcatat gtttttttcn     480
   cctatgtgct cccctccccc nnatcttaat ttaaaccnca attttgcnat tcnccnnnnn     540
   nannnannna a                                                          551
<210> 69
   <211> 396
   <212> DNA
   <213> Homo sapien
<220>
   <221> misc_feacure
   <222> (1)...(396)
   <223> n = A,T,C or G
<400> 69
   cagaaatgga aagcagagtt ttcatttctg tttataaacg tctccaaaca aaaatggaaa     60
   gcagagtttt cattaaatcc ttttaccttt tttttttctt ggtaatcccc tcaaataaca    120
   gtatgtggga tattgaatgt taaagggata tttttttcta ttatttttat aattgtacaa    180
   aattaagcaa atgttaaaag ttttatatgc tttattaatg ttttcaaaag gtatnataca    240
   tgtgatacat tttttaagct tcagttgctt gtcttctggt actttctgtt atgggctttt    300
   ggggagccan aaaccaatct acnatctctt tttgtttgcc aggacatgca ataaaattta    360
   aaaaataaat aaaaactatt nagaaattga aaaaaa                              396
<210> 70
   <211> 536
   <212> DNA
   <213> Homo sapien
<220>
   <221> misc_feature
   <222> (1)...(536)
   <223> n = A,T,C or G
<400> 70
   actagtgcaa aagcaaatat aaacatcgaa aaggcgttcc tcacgttagc tgaagatatc     60
   ctttgaaaga cccctgtaaa agagcccaac agtgaaaatg tagatatcag cagtggagga   120
   ggcgtgacag gctggaagag caaatgctgc tgagcattct cctgttccat cagttgccat    180
   ccactacccc gtttctcttc cttgctgcaa aataaaccac tctgtccatt tttaactcta    240
   aacagatatt tttgtttctc atcttaacta tccaagccac ctattttatt tgttctttca    300
   tctgtgactg cttgctgact ttatcataat tttcttcaaa caaaaaaatg tatagaaaaa    360
   tcatgtctgt gacttcattt ttaaatgnta cttgctcagc tcaactgcat ttcagttgtt    420
   ttatagtcca gttcttatca acattnaaac ctatngcaat catttcaaat ctattctgca    480
   aattgtataa gaataaaagt tagaatttaa caattaaaaa aaaaaaaaaa aaaaaa        536
<210> 71
   <211> 865
   <212> DNA
   <213> Homo sapien
<220>
   <221> misc_feature
   <222> (1)...(865)
   <223> n = A,T,C or G
<400> 71
   gacaaagcgt taggagaaga anagaggcag ggaanactnc ccaggcacga tggccncctt     60
   cccaccagca accagcgccc cccaccagcc cccaggcccg gacgacgaag actccatcct    120
   ggattaatct nacctctntc gcctgnccca ttcctacctc ggaggtggag gccggaaagg    180
   tcncaccaag aganaanctg ctgccaacac caaccgcccc agccctggcg ggcacganag    240
   gaaactggtg accaatctgc agaattctna gaggaanaag cnaggggccc cgcgctnaga    300
   cagagctgga tatgangcca gaccatggac nctacncccn ncaatncana cgggactgcg    360
   gaagatggan gacccncgac nngatcaggc cngctnncca nccccccacc cctatgaatt  420
   attcccgctg aangaatctc tganngcttt ccannaaagc gcctccccnc cnaacgnaan    480
   tncaacatng ggattanang ctgggaactg naaggggcaa ancctnnaat atccccagaa 540
   acaanctctc ccnaanaaac tggggcncct catnggtggn accaactatt aactaaaccg 600
   cacgccaagn aantataaaa ggggggcccc tccncggnng accccctttt gtcccttaat 660
   ganggttatc cnccttgcgt accatggtnc ccnnttctgt ntgnatgttt ccnctccccc 720
   ccncctatnt cnagccgaac tcnnatttnc ccgggggtgc natcnantng tncncctttn 780
   ttngttgncc cngccctttc cgncggaacn cgtttccccg ttantaacgg cacccggggn 840
   aagggtgntt ggccccctcc ctccc 865
<210> 72
   <211> 560
   <212> DNA
   <213> Homo sapien
<220>
   <221> misc_feature
   <222> (1)...(560)
   <223> n = A,T,C or G
<400> 72
   cctggacttg tcttggttcc agaacctgac gacccggcga cggcgacgtc tcttttgact 60
   aaaagacagt gtccagtgct ccngcctagg agtctacggg gaccgcctcc cgcgccgcca 120
   ccatgcccaa cttctctggc aactggaaaa tcatccgatc ggaaaacttc gangaattgc 180
   tcnaantgct gggggtgaat gtgatgctna ngaanattgc tgtggctgca gcgtccaagc 240
   cagcagtgga gatcnaacag gagggagaca ctttctacat caaaacctcc accaccgtgc 300
   gcaccacaaa gattaacttc nnngttgggg aggantttga ggancaaact gtggatngga      360
   ngcctgtnaa aacctggtga aatgggagaa tganaataaa atggtctgtg ancanaaact 420
   cctgaaagga gaaggccccc anaactcctg gaccngaaaa actgacccnc cnatngggga 480
   actgatnctt gaaccctgaa cgggcgggat ganccttttt tnttgccncc naangggttc 540
   tttccntttc cccaaaaaaa 560
<210> 73
   <211> 379
   <212> DNA
   <213> Homo sapien
<220>
   <221> misc_feature
   <222> (1)...(379)
   <223> n = A,T,C or G
<400> 73
   ctggggancc ggcggtnngc nccatntcnn gncgcgaagg tggcaataaa aanccnctga 60
   aaccgcncaa naaacatgcc naagatatgg acgaggaaga tngngctttc nngnacaanc 120
   gnanngagga acanaacaaa ctcnangagc tctcaagcta atgccgcggg gaaggggccc 180
   ttggccacnn gtggaattaa gaaatctggc aaanngtann tgttccttgt gcctnangag 240
   ataagngacc ctttatttca tctgtattta aacctctctn ttccctgnca taacttcttt 300
   tnccacgtan agntggaant anttgttgtc ttggactgtt gtncatttta gannaaactt 360
   ttgttcaaaa aaaaaataa 379
<210> 74
   <211> 437
   <212> DNA
   <213> Homo sapien
<220>
   <221> misc_feature
   <222> (1)...(437)
   <223> n = A,T,C or G
<400> 74
   actagttcag actgccacgc caaccccaga aaatacccca catgccagaa aagtgaagtc     60
   ctaggtgttt ccatctatgt ttcaatctgt ccatctacca ggcctcgcga taaaaacaaa    120
   acaaaaaaac gctgccaggt tttanaagca gttctggtct caaaaccatc aggatcctgc    180
   caccagggtt cttttgaaat agtaccacat gtaaaaggga atttggcttt cacttcatct    240
   aatcactgaa ttgtcaggct ttgattgata attgtagaaa taagtagcct tctgttgtgg    300
   gaataagtta taatcagtat tcatctcttt gttttttgtc actcttttct ctctnattgt     360
   gtcatttgta ctgtttgaaa aatatttctt ctataaaatt aaactaacct gccttaaaaa    420
   aaaaaaaaaa aaaaaaa                                                   437
<210> 75
   <211> 579
   <212> DNA
   <213> Homo sapien
<220>
   <221> misc_feature
   <222> (1)...(579)
   <223> n = A,T,C or G
<400> 75
   ctccgtcgcc gccaagatga tgtgcggggc gccctccgcc acgcagccgg ccaccgccga     60
   gacccagcac atcgccgacc aggtgaggtc ccagcttgaa gagaaagaaa acaagaagtt    120
   ccctgtgttt aaggccgtgt cattcaagag ccaggtggtc gcggggacaa actacttcat    180
   caaggtgcac gtcggcgacg aggacttcgt acacctgcga gtgttccaat ctctccctca    240
   tgaaaacaag cccttgacct tatctaacta ccagaccaac aaagccaagc atgatgagct    300
   gacctatttc tgatcctgac tttggacaag gcccttcagc cagaagactg acaaagtcat    360
   cctccgtcta ccagagcgtg cacttgtgat cctaaaataa gcttcatctc cgggctgtgc    420
   ccttggggtg gaaggggcan gatctgcact gcttttgcat ttctcttcct aaatttcatt    480
   gtgttgattc tttccttcca ataggtgatc ttnattactt tcagaatatt ttccaaatna    540
   gatatatttt naaaatcctt aaaaaaaaaa aaaaaaaaa                           579
<210> 76
   <211> 666
   <212> DNA
   <213> Homo sapien
<220>
   <221> misc_feature
   <222> (1)...(666)
   <223> n = A,T,C or G
<400> 76
   gtttatccta tctctccaac cagattgtca gctccttgag ggcaagagcc acagtatatt     60
   tccctgtttc ttccacagtg cctaataata ctgtggaact aggttttaat aattttttaa    120
   ttgatgttgt tatgggcagg atggcaacca gaccattgtc tcagagcagg tgctggctct    180
   ttcctggcta ctccatgttg gctagcctct ggtaacctct tacttattat cttcaggaca    240
   ctcactacag ggaccaggga tgatgcaaca tccttgtctt tttatgacag gatgtttgct    300
   cagcttctcc aacaataaaa agcacgtggt aaaacacttg cggatattct ggactgtttt    360
   taaaaaatat acagtttacc gaaaatcata ttatcttaca atgaaaagga ntttatagat    420
   cagccagtga acaacctttt cccaccatac aaaaattcct tttcccgaan gaaaanggct    480
   ttctcaataa ncctcacttt cttaanatct tacaagatag ccccganatc ttatcgaaac 540
   tcattttagg caaatatgan ttttattgtn cgttacttgt ttcaaaattt ggtattgtga 600
   atatcaatta ccacccccat ctcccatgaa anaaanggga aanggtgaan ttcntaancg 660
   cttaaa 666
<210> 77
   <211> 396
   <212> DNA
   <213> Homo sapien
<220>
   <221> misc_feature
   <222> (1)...(396)
   <223> n = A,T,C or G
<400> 77
   ctgcagcccg ggggatccac taatctacca nggttatttg gcagctaatt ctanatttgg 60
   atcattgccc aaagttgcac ttgctggtct cttgggattt ggccttggaa aggtatcata 120
   catanganta tgccanaata aattccattt ttttgaaaat canctccntg gggctggttt 180
   tggtccacag cataacangc actgcctcct tacctgtgag gaatgcaaaa taaagcatgg 240
   attaagtgag aagggagact ctcagccttc agcttcctaa attctgtgtc tgtgactttc 300
   gaagtttttt aaacctctga atttgtacac atttaaaatt tcaagtgtac tttaaaataa 360
   aatacttcta atgggaacaa aaaaaaaaaa aaaaaa 396
<210> 78
   <211> 793
   <212> DNA
   <213> Homo sapien
<220>
   <221> misc_feature
   <222> (1)...(793)
   <223> n = A,T,C or G
<400> 78
   gcatcctagc cgccgactca cacaaggcag gtgggtgagg aaatccagag ttgccatgga 60
   gaaaattcca gtgtcagcat tcttgctcct tgtggccctc tcctacactc tggccagaga 120
   taccacagtc aaacctggag ccaaaaagga cacaaaggac tctcgaccca aactgcccca 180
   gaccctctcc agaggttggg gtgaccaact catctggact cagacatatg aagaagctct 240
   atataaatcc aagacaagca acaaaccctt gatgattatt catcacttgg atgagtgccc 300
   acacagtcna gctttaaaga aagtgtttgc tgaaaataaa gaaatccaga aattggcaga 360
   gcagtttgtc ctcctcaatc tggtttatga aacaactgac aaacaccttt ctcctgatgg 420
   ccagtatgtc ccaggattat gtttgttgac ccatctctga cagttgaagc cgatatcctg 480
   ggaagatatt cnaaccgtct ctatgcttac aaactgcaga tacgctctgt tgcttgacac 540
   atgaaaaagc tctcaagttg ctnaaaatga attgtaagaa aaaaaatctc cagccttctg 600
   tctgtcggct tgaaaattga aaccagaaaa atgtgaaaaa tggctattgt ggaacanatn 660
   gacacctgat taggttttgg ttatgttcac cactattttt aanaaaanan nttttaaaat 720
   ttggttcaat tntctttttn aaacaatntg tttctacntt gnganctgat ttctaaaaaa 780
   aataatnttt ggc 793
<210> 79
   <211> 456
   <212> DNA
   <213> Homo sapien
<220>
   <221> misc_feature
   <222> (1)...(456)
   <223> n = A,T,C or G
<400> 79
   actagtatgg ggtgggaggc cccacccttc tcccctaggc gctgttcttg ctccaaaggg      60
   ctccgtggag agggactggc agagctgang ccacctgggg ctggggatcc cactcttctt     120
   gcagctgttg agcgcaccta accactggtc atgcccccac ccctgctctc cgcacccgct     180
   tcctcccgac cccangacca ggctacttct cccctcctct tgcctccctc ctgcccctgc   240
   tgcctctgat cgtangaatt gangantgtc ccgccttgtg gctganaatg gacagtggca     300
   ggggctggaa atgggtgtgt gtgtgtgtgt gtgtgtgtgt gtgtgtgtgt gcnccccccc    360
   tgcaagaccg agattgaggg aaancatgtc tgctgggtgt gaccatgttt cctctccata    420
   aantncccct gtgacnctca naaaaaaaaa aaaaaa                              456
<210> 80
   <211> 284
   <212> DNA
   <213> Homo sapien
<220>
   <221> misc_feature
   <222> (1)...(284)
   <223> n = A,T,C or G
<400> 80
<210> 81
   <211> 671
   <212> DNA
   <213> Homo sapien
<220>
   <221> misc_feature
   <222> (1)...(671)
   <223> n = A,T,C or G
<400> 81
   gccaccaaca ttccaagcta ccctgggtac ctttgtgcag tagaagctag tgagcatgtg      60
   agcaagcggt gtgcacacgg agactcatcg ttataattta ctatctgcca agagtagaaa     120
   gaaaggctgg ggatatttgg gttggcttgg ttttgatttt ttgcttgttt gtttgttttg     180
   tactaaaaca gtattatctt ttgaatatcg tagggacata agtatataca tgttatccaa     240
   tcaagatggc tagaatggtg cctttctgag tgtctaaaac ttgacacccc tggtaaatct     300
   ttcaacacac tttcactgcc tgcgtaatga agttttgatt catttttaac cactggaatt     360
   tttcaatgcc gtcattttca gttagatnat tttgcacttt gagattaaaa tgccatgtct     420
   atttgattag tcttattttt ttatttttac aggcttatca gtctcactgt tggctgtcat     480
   tgtgacaaag tcaaataaac ccccnaggac aacacacagt atgggatcac atattgtttg     540
   acattaagct ttggccaaaa aatgttgcat gtgttttacc tcgacttgct aaatcaatan     600
   canaaaggct ggctnataat gttggtggtg aaataattaa tnantaacca aaaaaaaaan     660
   aaaaaaaaaa a                                                          671
<210> 82
   <211> 217
   <212> DNA
   <213> Homo sapien
<220>
   <221> misc_feature
   <222> (1)...(217)
   <223> n = A,T,C or G
<400> 82
   ctgcagatgt ttcttgaatg ctttgtcaaa ttaanaaagt taaagtgcaa taatgtttga      60
   agacaataag tggtggtgta tcttgtttct aataagataa acttttttgt ctttgcttta     120
   tcttattagg gagttgtatg tcagtgtata aaacatactg tgtggtataa caggcttaat     180
   aaattcttta aaaggaaaaa aaaaaaaaaa aaaaaaa                               217
<210> 83
   <211> 460
   <212> DNA
   <213> Homo sapien
<220>
   <221> misc_feature
   <222> (1)...(460)
   <223> n = A,T,C or G
<400> 83
   cgcgagtggg agcaccagga tctcgggctc ggaacgagac tgcacggatt gttttaagaa      60
   aatggcagac aaaccagaca tgggggaaat cgccagcttc gatnaggcca agctgaanaa     120
   aacggagacg caggagaaga acaccctgcc gaccaaagag accattgagc angagaagcg     180
   gagtgaaatt tcctaagatc ctggaggatt tcctaccccc gtccttctcg agaccccagt     240
   cgtgatgtgg aggaagagcc acctgcaaga tggacacgag ccacaagctg cactgtgaac     300
   ctgggcactc cgcgccgatg ccaccggcct gtgggtctct gaagggaccc cccccaatcg     360
   gactgccaaa ttctccggtt tgccccggga tattatacaa nattatttgt atgaataatg     420
   annataaaac acacctcgtg gcancaaana aaaaaaaaaa                           460
<210> 84
   <211> 323
   <212> DNA
   <213> Homo sapien
<220>
   <221> misc_feature
   <222> (1)...(323)
   <223> n = A,T,C or G
<400> 84
   tggtggatct tggctctgtg gagctgctgg gacgggatct aaaagactat tctggaagct      60
   gtggtccaan gcattttgct ggcttaacgg gtcccggaac aaaggacacc agctctctaa     120
   aattgaagtt tacccganat aacaatcttt tgggcagaga tgcctatttt aacaaacncc     180
   gtccctgcgc aacaacnaac aatctctggg aaataccggc catgaacntg ctgtctcaat     240
   cnancatctc tctagctgac cgatcatatc gtcccagatt actacanatc ataataattg     300
   atttcctgta naaaaaaaaa aaa                                             323
<210> 85
   <211> 771
   <212> DNA
   <213> Homo sapien
<220>
   <221> misc_feature
   <222> (1)...(771)
   <223> n = A,T,C or G
<400> 85
   aaactgggta ctcaacactg agcagatctg ttctttgagc taaaaaccat gtgctgtacc      60
   aanagtttgc tcctggctgc tttgatgtca gtgctgctac tccacctctg cggcgaatca     120
   gaagcaagca actttgactg ctgtcttgga tacacagacc gtattcttca tcctaaattt     180
   attgtgggct tcacacggca gctggccaat gaaggctgtg acatcaatgc tatcatcttt     240
   cacacaaaga aaaagttgtc tgtgtgcgca aatccaaaac agacttgggt gaaatatatt     300
   gtgcgtctcc tcagtaaaaa agtcaagaac atgtaaaaac tgtggctttt ctggaatgga     360
   attggacata gcccaagaac agaaagaact tgctggggtt ggaggtttca cttgcacatc     420
   atgganggtt tagtgcttat cttatttgtg cctcctggac ttgtccaatt natgaagtta     480
   atcatattgc atcatanttt gctttgttta acatcacatt naaattaaac tgtattttat     540
   gttatttata gctntaggtt ttctgtgttt aactttttat acnaantttc ctaaactatt     600
   ttggtntant gcaanttaaa aattatattt ggggggggaa taaatattgg antttctgca      660
   gccacaagct ttttttaaaa aaccantaca nccnngttaa atggtnggtc ccnaatggtt      720
   tttgcttttn antagaaaat ttnttagaac natttgaaaa aaaaaaaaaa a               771
<210> 86
   <211> 628
   <212> DNA
   <213> Homo sapien
<220>
   <221> misc_feature
   <222> (1)...(628)
   <223> n = A,T,C or G
<400> 86
   actagtttgc tttacatttt tgaaaagtat tatttttgtc caagtgctta tcaactaaac      60
   cttgtgttag gtaagaatgg aatttattaa gtgaatcagt gtgacccttc ttgtcataag     120
   attatcttaa agctgaagcc aaaatatgct tcaaaagaaa angactttat tgttcattgt     180
   agttcataca ttcaaagcat ctgaactgta gtttctatag caagccaatt acatccataa     240
   gtggagaang aaatagatta atgtcnaagt atgattggtg gagggagcaa ggttgaagat     300
   aatctggggt tgaaattttc tagttttcat tctgtacatt tttagttnga catcagattt     360
   gaaatattaa tgtttacctt tcaatgtgtg gtatcagctg gactcantaa cacccctttc     420
   ttccctnggg gatggggaat ggattattgg aaaatggaaa gaaaaaagta cttaaagcct     480
   tcctttcnca gtttctggct cctaccctac tgatttancc agaataagaa aacattttat     540
   catcntctgc tttattccca ttaatnaant tttgatgaat aaatctgctt ttatgcnnac     600
   ccaaggaatt nagtggnttc ntcnttgt                                        628
<210> 87
   <211> 518
   <212> DNA
   <213> Homo sapien
<220>
   <221> misc_feature
   <222> (1)...(518)
   <223> n = A,T,C or G
<400> 87
   ttttttattt tttttagaga gtagttcagc ttttatttat aaatttattg cctgttttat       60
   tataacaaca ttatactgtt tatggtttaa tacatatggt tcaaaatgta taatacatca      120
   agtagtacag ttttaaaatt ttatgcttaa aacaagtttt gtgtaaaaaa tgcagataca      180
   ttttacatgg caaatcaatt tttaagtcat cctaaaaatt gatttttttt tgaaatttaa      240
   aaacacattt aatttcaatt tctctcttat ataaccttta ttactatagc atggtttcca      300
   ctacagttta acaatgcagc aaaattccca tttcacggta aattgggttt taagcggcaa      360
   ggttaaaatg ctttgaggat cctnaatacc ctttgaactt caaatgaagg ttatggttgt      420
   naatttaacc ctcatgccat aagcagaagc acaagtttag ctgcattttg ctctaaactg      480
   taaaancgag ccccccgttg aaaaagcaaa agggaccc                               518
<210> 88
   <211> 1844
   <212> DNA
   <213> Homo sapien
<400> 98
<210> 89
   <211> 523
   <212> DNA
   <213> Homo sapien
<220>
   <221> misc_feature
   <222> (1)...(523)
   <223> n = A,T,C or G
<400> 89
   tttttttttt tttttttagt caatccacat ttattgatca cttattatgt accaggcact     60
   gggataaaga tgactgttag tcactcacag taaggaagaa aactagcaaa taagacgatt    120
   acaatatgat gtagaaaatg ctaagccaga gatatagaaa ggtcctattg ggtccttctg    180
   tcaccttgtc tttccacatc cctacccttc acaggccttc cctccagctt cctgcccccg    240
   ctccccactg cagatcccct gggattttgc ctagagctaa acgagganat gggccccctg     300
   gccctggcat gacttgaacc caaccacaga ctgggaaagg gagcctttcg anagtggatc     360
   actttgatna gaaaacacat agggaattga agagaaantc cccaaatggc cacccgtgct     420
   ggtgctcaag aaaagtttgc agaatggata aatgaaggat caagggaatt aatanatgaa    480
   taattgaatg gtggctcaat aagaatgact ncnttgaatg acc                      523
<210> 90
   <211> 604
   <212> DNA
   <213> Home sapien
<220>
   <221> misc_feature
   <222> (1)...(604)
   <223> n = A,T,C or G
<400> 90
   ccagtgtggt ggaatgcaaa gattaccccg gaagctttcg agaagctggg attccctgca     60
   gcaaaggaaa tagccaatat gtgtcgtttc tatgaaatga agccagaccg agatgtcaat    120
   ctcacccacc aactaaatcc caaagtcaaa agcttcagcc agtttatctc agagaaccag    180
   gggagccttc aagggcatgt agaaaatcag ctgttcagat aggcctctgc accacacagc    240
   ctctttcctc tctgatcctt ttcctcttta cggcacaaca ttcatgtttg acagaacatg    300
   ctggaatgca attgtttgca acaccgaagg atttcctgcg gtcgcctctt cagtaggaag    360
   cactgcattg gtgataggac acggtaattt gattcacatt taacttgcta gttagtgata    420
   aggggtggta cacctgtttg gtaaaatgag aagcctcgga aacttgggag cttctctcct    480
   accactaatg gggagggcag attattactg ggatttctcc tggggtgaat taatttcaag    540
   ccctaattgc tgaaattccc ctnggcaggc tccagttttc tcaactgcat tgcaaaattc    600
   cccc                                                                 604
<210> 91
   <211> 858
   <212> DNA
   <213> Homo sapien
<220>
   <221> misc_feature
   <222> (1)...(858)
   <223> n = A,T,C or G
<400> 91
   tttttttttt ttttttttta tgattattat tttttttatt gatctttaca tcctcagtgt     60
   tggcagagtt tctgatgctt aataaacatt tgttctgatc agataagtgg aaaaaattgt    120
   catttcctta ttcaagccat gcttttctgt gatattctga tcctagttga acatacagaa    180
   ataaatgtct aaaacagcac ctcgattctc gtctataaca ggactaagtt cactgtgatc 240
   ttaaataagc ttggctaaaa tgggacatga gtggaggtag tcacacttca gcgaagaaag 300
   agaatctcct gtataatctc accaggagat tcaacgaatt ccaccacact ggactagtgg 360
   atcccccggg ctgcaggaat tcgatatcaa gcttatcgat accgtcgacc tcgagggggg 420
   gcccggtacc caattcgccc tatagtgagt cgtattacgc gcgctcactg gccgtcgttt 480
   tacaacgtcg tgactgggaa aaccctggcg ttacccaact taatcgcctt gcagcacatc 540
   cccctttcgc cagctggcgt aatagcgaan agcccgcacc gatcgccctt ncaacagttg 600
   cgcagcctga atggcgaatg ggacgcgccc tgtagcggcg cattaaagcg cggcngggtg 660
   tggnggntcc cccacgtgac cgntacactt ggcagcgcct tacgccggtc nttcgctttc 720
   ttcccttcct ttctcgcacc gttcgccggg tttccccgnn agctnttaat cgggggnctc 780
   cctttanggg tncnaattaa nggnttacng gaccttngan cccaaaaact ttgattaggg 840
   ggaaggtccc cgaagggg 858
<210> 92
   <211> 585
   <212> DNA
   <213> Homo sapien
<220>
   <221> misc_feature
   <222> (1)...(585)
   <223> n = A,T,C or G
<400> 92
   gttgaatctc ctggtgagat tatacaggag attctctttc ttcgctgaag tgtgactacc 60
   tccactcatg tcccatttta gccaagctta tttaagatca cagtgaactt agtcctgtta 120
   tagacgagaa tcgaggtgct gttttagaca tttatttctg tatgttcaac taggatcaga 180
   atatcacaga aaagcatggc ttgaataagg aaatgacaat tttttccact tatctgatca 240
   gaacaaatgt ttattaagca tcagaaactc tgccaacact gaggatgtaa agatcaataa 300
   aaaaaataat aatcatnann naaanannan nngaagggcg gccgccaccg cggtggagct 360
   ccagcttttg ttccctttag tgagggttaa ttgcgcgctt ggcgttaatc atggtcatag 420
   ctgtttcctg tgtgaaattg ttatccggct cacaattccn cncaacatac gagccgggaa 480
   gcntnangtg taaaagcctg gggtgcctaa attgagtgag ctnactcaca ttaattgngt 540
   tgcgctccac ttgcccgctt ttccantccg ggaaacctgt tcgnc 585
<210> 93
   <211> 567
   <212> DNA
   <213> Homo sapien
<220>
   <221> misc_feature
   <222> (1)...(567)
   <223> n = A,T,C or G
<400> 93
   cggcagtgtt gctgtctgcg tgtccacctt ggaatctggc tgaactggct gggaggacca 60
   agactgcggc tggggtgggc anggaaggga accgggggct gctgtgaagg atcttggaac 120
   ttccctgtac ccaccttccc cttgcttcat gtttgtanag gaaccttgtg ccggccaagc 180
   ccagtttcct tgtgtgatac actaatgtat ttgctttttt tgggaaatan anaaaaatca 240
   attaaattgc tantgtttct ttgaannnnn nnnnnnnnnn nnnnnnnggg ggggncgccc 300
   ccncggngga aacnccccct tttgttccct ttaattgaaa ggttaattng cncncntggc 360
   gttaanccnt gggccaaanc tngttncccg tgntgaaatt gttnatcccc tcccaaattc 420
   ccccccnncc ttccaaaccc ggaaancctn annntgttna ancccggggg gttgcctaan 480
   ngnaattnaa ccnaaccccc ntttaaatng nntttgcncn ccacnngccc cnctttccca 540
   nttcggggaa aaccctntcc gtgccca 567
<210> 94
   <211> 620
   <212> DNA
   <213> Homo sapien
<220>
   <221> misc_feature
   <222> (1)...(620)
   <223> n = A,T,C or G
<400> 94
   actagtcaaa aatgctaaaa taatttggga gaaaatattt tttaagtagt gttatagttt 60
   catgtttatc ttttattatg ttttgtgaag ttgtgtcttt tcactaatta cctatactat 120
   gccaatattt ccttatatct atccataaca tttatactac atttgtaana naatatgcac 180
   gtgaaactta acactttata aggtaaaaat gaggtttcca anatttaata atctgatcaa 240
   gttcttgtta tttccaaata gaatggactt ggtctgttaa gggctaagga gaagaggaag 300
   ataaggttaa aagttgttaa tgaccaaaca ttctaaaaga aatgcaaaaa aaaagtttat 360
   tttcaagcct tcgaactatt taaggaaagc aaaatcattt cctaaatgca tatcatttgt 420
   gagaatttct cattaatatc ctgaatcatt catttcacta aggctcatgt tnactccgat 480
   atgtctctaa gaaagtacta tttcatggtc caaacctggt tgccatantt gggtaaaggc 540
   tttcccttaa gtgtgaaant atttaaaatg aaattttcct ctttttaaaa attctttana 600
   agggttaagg gtgttgggga 620
<210> 95
   <211> 470
   <212> DNA
   <213> Homo sapien
<220>
   <221> misc_feature
   <222> (1)...(470)
   <223> n = A,T,C or G
<400> 95
   ctcgaccttc tctgcacagc ggatgaaccc tgagcagctg aagaccagaa aagccactat 60
   nactttntgc ttaattcang agcttacang attcttcaaa gagtgngtcc agcatccttt 120
   gaaacatgag ttcttaccag cagaagcaga cctttacccc accacctcag cttcaacagc 180
   agcaggtgaa acaacccatc cagcctccac ctnaggaaat atttgttccc acaaccaagg 240
   agccatgcca ctcaaaggtt ccacaacctg naaacacaaa nattccagag ccaggctgta 300
   ccaaggtccc tgagccaggg ctgtaccaan gtccctgagc caggttgtac caangtccct 360
   gagccaggat gtaccaaggt ccctgancca ggttgtccaa ggtccctgag ccaggctaca 420
   ccaagggcct gngccaggca gcatcaangt ccctgaccaa ggcttatcaa 470
<210> 96
   <211> 660
   <212> DNA
   <213> Homo sapien
<220>
   <221> misc_feature
   <222> (1)...(660)
   <223> n = A,T,C or G
<400> 96
   tttttttttt tttttttttt ggaattaaaa gcaatttaat gagggcagag caggaaacat    60
   gcatttcttt tcattcgaat cttcagatga accctgagca gccgaagacc agaaaagcca  120
   tgaagacttt ctgcttaatt caggggctta caggattctt cagagtgtgt gtgaacaaaa  180
   gctttatagt acgtattttt aggatacaaa taagagagag actatggctt ggggtgagaa  240
   tgtactgatt acaaggtcta cagacaatta agacacagaa acagatggga agagggtgnc  300
   cagcatctgg nggttggctt ctcaagggct tgtctgtgca ccaaattact tctgcttggn  360
   cttctgctga gctgggcctg gagtgaccgt tgaaggacat ggctctggta cctttgtgta  420
   gcctgncaca ggaactttgg tgtatccttg ctcaggaact ttgatggcac ctggctcagg  480
   aaacttgatg aagccttggt caagggacct tgatgcttgc tggctcaggg accttggngn  540
   ancctgggct canggacctt tgncncaacc ttggcttcaa gggacccttg gnacatcctg  600
   gcnnagggac ccttgggncc aaccctgggc ttnagggacc ctttggntnc nanccttggc  660
<210> 97
   <211> 441
   <212> DNA
   <213> Homo sapien
<220>
   <221> misc_feature
   <222> (1)...(441)
   <223> n = A,T,C or G
<400> 97
   gggaccatac anagtattcc tctcttcaca ccaggaccag ccactgttgc agcatgagtt    60
   cccagcagca gaagcagccc tgcatcccac cccctcagct tcagcagcag caggtgaaac  120
   agccttgcca gcctccacct caggaaccat gcatccccaa aaccaaggag ccctgccacc  180
   ccaaggtgcc tgagccctgc caccccaaag tgcctgagcc ctgccagccc aaggttccag  240
   agccatgcca ccccaaggtg cctgagccct gcccttcaat agtcactcca gcaccagccc  300
   agcagaanac caagcagaag taatgtggtc cacagccatg cccttgagga gccggccacc  360
   agatgctgaa tcccctatcc cattctgtgt atgagtccca tttgccttgc aattagcatt  420
   ctgtctcccc caaaaaaaaa a                                             441
<210> 98
   <211> 600
   <212> DNA
   <213> Homo sapien
<220>
   <221> misc_feature
   <222> (1)...(600)
   <223> n = A,T,C or G
<400> 98
   gtattcctct cttcacacca ggaccagcca ctgttgcagc atgagttccc agcagcagaa    60
   gcagccctgc atcccacccc ctcagcttca gcagcagcag gtgaaacagc cttgccagcc  120
   tccacctcag gaaccatgca tccccaaaac caaggagccc tgccacccca aggtgcctga  180
   gccctgccac cccaaagtgc ctgagccctg ccagcccaag gttccagagc catgccaccc  240
   caaggtgcct gagccctgcc cttcaatagt cactccagca ccagcccagc agaanaccaa  300
   gcagaagtaa tgtggtccac agccatgccc ttgaggagcc ggccaccana tgctgaatcc  360
   cctatcccat tctgtgtatg agtcccattt gccttgcaat tagcattctg tctcccccaa  420
   aaaagaatgt gctatgaagc tttctttcct acacactctg agtctctgaa tgaagctgaa  480
   ggtcttaant acaganctag ttttcagctg ctcagaattc tctgaagaaa agatttaaga  540
   tgaaaggcaa atgattcagc tccttattac cccattaaat tcnctttcaa ttccaaaaaa  600
<210> 99
   <211> 667
   <212> DNA
   <213> Homo sapien
<220>
   <221> misc_feature
   <222> (1)...(667)
   <223> n = A,T,C or G
<400> 99
   actagtgact gagttcctgg caaagaaatt tgacctggac cagttgataa ctcatgtttt 60
   accatttaaa aaaatcagtg aaggatttga gctgctcaat tcaggacaaa gcattcgaac 120
   ggtcctgacg ttttgagatc caaagtggca ggaggtctgt gttgtcatgg tgaactggag 180
   tttctcttgt gagagttccc tcatctgaaa tcatgtatct gtctcacaaa tacaagcata 240
   agtagaagat ttgttgaaga catagaaccc ttataaagaa ttattaacct ttataaacat 300
   ttaaagtctt gtgagcacct gggaattagt ataataacaa tgttnatatt tttgatttac 360
   attttgtaag gctataattg tatcttttaa gaaaacatac cttggatttc tatgttgaaa 420
   tggagatttt taagagtttt aaccagctgc tgcagatata ttactcaaaa cagatatagc 480
   gtataaagat atagtaaatg catctcctag agtaatattc acttaacaca ttggaaacta 540
   ttatttttta gatttgaata tnaatgttat tttttaaaca cttgttatga gttacttggg 600
   attacatttt gaaatcagtt cattccatga tgcanattac tgggattaga ttaagaaaga 660
   cggaaaa 667
<210> 100
   <211> 583
   <212> DNA
   <213> Homo sapien
<220>
   <221> misc_feature
   <222> (1)...(583)
   <223> n = A,T,C or G
<400> 100
   gttttgtttg taagatgatc acagtcatgt tacactgatc taaaggacat atatataacc 60
   ctttaaaaaa aaaatcactg cctcattctt atttcaagat gaatttctat acagactaga 120
   tgtttttctg aagatcaatt agacattttg aaaatgattt aaagtgtttt ccttaatgtt 180
   ctctgaaaac aagtttcttt tgtagtttta accaaaaaag tgcccttttt gtcactggat 240
   tctcctagca ttcatgattt ttttttcata caatgaaatt aaaattgcta aaatcatgga 300
   ctggctttct ggttggattt caggtaagat gtgtttaagg ccagagcttt tctcagtatt 360
   tgattttttt ccccaatatt tgatttttta aaaatataca catnggtgct gcatttatat 420
   ctgctggttt aaaattctgt catatttcac ttctagcctt ttagttatgg caaatcatat 480
   tttactttta cttaaagcat ttggtnattt ggantatctg gttctannct aaaaaaanta 540
   attctatnaa ttgaantttt ggtactcnnc catatttgga tcc 583
<210> 101
   <211> 592
   <212> DNA
   <213> Homo sapien
<220>
   <221> misc_feature
   <222> (1)...(592)
   <223> n = A,T,C or G
<400> 101
   gtggagacgt acaaagagca gccgctcaag acacctggga agaaaaagaa aggcaagccc    60
   gggaaacgca aggagcagga aaagaaaaaa cggcgaactc gctctgcctg gttagactct   120
   ggagtgactg ggagtgggct agaaggggac cacctgtctg acacctccac aacgtcgctg   180
   gagctcgatt cacggaggca ttgaaatttt cagcaganac cttccaagga catattgcag   240
   gattctgtaa tagtgaacat atggaaagta ttagaaatat ttattgtctg taaatactgt   300
   aaatgcattg gaataaaact gtctccccca ttgctctatg aaactgcaca ttggtcattg   360
   tgaatatttt tttttttgcc aaggctaatc caattattat tatcacattt accataattt   420
   attttgtcca ttgatgtatt tattttgtaa atgtatcttg gtgctgctga atttctatat   480
   tttttgtaca taatgcnttt anatatacct atcaagtttg ttgataaatg acncaatgaa   540
   gtgncncnan ttggnggttg aatttaatga atgcctaatt ttattatccc aa           592
<210> 102
   <211> 587
   <212> DNA
   <213> Homo sapien
<220>
   <221> misc_feature
   <222> (1) ... (587)
   <223> n = A,T,C or G
<400> 102
   cgtcctaagc acttagacta catcagggaa gaacacagac cacatccctg tcctcatgcg    60
   gcttatgttt tctggaagaa agtggagacc nagtccttgg ctttagggct ccccggctgg   120
   gggctgtgca ntccggtcag ggcgggaagg gaaatgcacc gctgcatgtg aacttacagc   180
   ccaggcggat gccccttccc ttagcactac ctggcctcct gcatcccctc gcctcatgtt   240
   cctcccacct tcaaanaatg aanaacccca tgggcccagc cccttgccct ggggaaccaa   300
   ggcagccttc caaaactcag gggctgaagc anactattag ggcaggggct gactttgggt   360
   gacactgccc attccctctc agggcagctc angtcacccn ggnctcttga acccagcctg   420
   ttcctttgaa aaagggcaaa actgaaaagg gcttttccta naaaaagaaa aaccagggaa   480
   ctttgccagg gcttcnntnt taccaaaacn ncttctcnng gatttttaat tccccattng   540
   gcctccactt accnggggcn atgccccaaa attaanaatt tcccatc                 587
<210> 103
   <211> 496
   <212> DNA
   <213> Homo sapien
<220>
   <221> misc_feature
   <222> (1)...(496)
   <223> n = A,T,C or G
<400> 103
   anaggactgg ccctacntgc tctctctcgt cctacctatc aatgcccaac atggcagaac    60
   ctgcanccct tggncactgc anatggaaac ctctcagtgt cttgacatca ccctacccnt   120
   gcggtgggtc tccaccacaa ccactttgac tctgtggtcc ctgnanggtg gnttctcctg   180
   actggcagga tggaccttan ccnacatatc cctctgttcc ctctgctnag anaaagaatt   240
   cccttaacat gatataatcc acccatgcaa ntngctactg gcccagctac catttaccat   300
   ttgcctacag aatttcattc agtctacact ttggcattct ctctggcgat agagtgtggc   360
   tgggctgacc gcaaaaggtg ccttacacac tggcccccac cctcaaccgt tgacncatca   420
   gangcttgcc tcctccttct gattnncccc catgttggat atcagggtgc tcnagggatt   480
   ggaaaagaaa caaaac                                                   496
<210> 104
   <211> 575
   <212> DNA
   <213> Homo sapien
<220>
   <221> misc_feature
   <222> (1)...(575)
   <223> n = A,T,C or G
<400> 104
   gcacctgctc tcaatccnnc tctcaccatg atcctccgcc tgcanaaact cctctgccaa      60
   ctatggangt ggtttcnggg gtggctcttg ccaactggga agaagccgtg gtgtctctac     120
   ctgttcaact cngtttgtgt ctgggggatc aactnggggc tatggaagcg gctnaactgt     180
   tgttttggtg gaagggctgg taattggctt tgggaagtng cttatngaag ttggcctngg     240
   gaagttgcta ttgaaagtng ccntggaagt ngntttggtg gggggttttg ctggtggcct     300
   ttgttnaatt tgggtgcttt gtnaatggcg gccccctcnc ctgggcaatg aaaaaaatca     360
   ccnatgcngn aaacctcnac nnaacagcct gggcttccct cacctcgaaa aaagttgctc     420
   cccccccaaa aaaggncaan cccctcaann tggaangttg aaaaaatcct cgaatgggga     480
   ncccnaaaac aaaaancccc ccntttcccn gnaanggggg aaataccncc cccccactta     540
   cnaaaaccct tntaaaaaac cccccgggaa aaaaa                                575
<210> 105
   <211> 619
   <212> DNA
   <213> Homo sapien
<220>
   <221> misc_feature
   <222> (1)...(619)
   <223> n = A,T,C or G
<400> 105
   cactagtagg atagaaacac tgtgtcccga gagtaaggag agaagctact attgattaga      60
   gcctaaccca ggttaactgc aagaagaggc gggatacttt cagctttcca tgtaactgta     120
   tgcataaagc caatgtagtc cagtttctaa gatcatgttc caagctaact gaatcccact     180
   tcaatacaca ctcatgaact cctgatggaa caataacagg cccaagcctg tggtatgatg     240
   tgcacacttg ctagactcan aaaaaatact actctcataa atgggtggga gtattttggt     300
   gacaacctac tttgcttggc tgagtgaagg aatgatattc atatattcat ttattccatg     360
   gacatttagt tagtgctttt tatataccag gcatgatgct gagtgacact cttgtgtata     420
   tttccaaatt tttgtacagt cgctgcacat atttgaaatc atatattaag acttccaaaa     480
   aatgaagtcc ctggtttttc atggcaactt gatcagtaaa ggattcncct ctgtttggta      540
   cttaaaacat ctactatatn gttnanatga aattcctttt ccccncctcc cgaaaaaana     600
   aagtggtggg gaaaaaaaa                                                  619
<210> 106
   <211> 506
   <212> DNA
   <213> Homo sapien
<220>
   <221> misc_feature
   <222> (1)...(506)
   <223> n = A,T,C or G
<400> 106
   cattggtnct ttcatttgct ntggaagtgt nnatctctaa cagtggacaa agttcccngt 60
   gccttaaact ctgtnacact tttgggaant gaaaanttng tantatgata ggttattctg 120
   angtanagat gttctggata ccattanatn tgcccccngt gtcagaggct catattgtgt 180
   tatgtaaatg gtatntcatt cgctactatn antcaattng aaatanggtc tttgggttat 240
   gaatantnng cagcncanct nanangctgt ctgtngtatt cattgtggtc atagcacctc 300
   acancattgt aacctcnatc nagtgagaca nactagnaan ttcctagtga tggctcanga 360
   ttccaaatgg nctcatntcn aatgtttaaa agttanttaa gtgtaagaaa tacagactgg 420
   atgttccacc aactagtacc tgtaatgacn ggcctgtccc aacacatctc ccttttccat 480
   gactgtggta ncccgcatcg gaaaaa 506
<210> 107
   <211> 452
   <212> DNA
   <213> Homo sapien
<220>
   <221> misc_feature
   <222> (1)...(452)
   <223> n = A,T,C or G
<400> 107
   gttgagtctg tactaaacag taagatatct caatgaacca taaattcaac tttgtaaaaa 60
   tcttttgaag catagataat attgtttggt aaatgtttct tttgtttggt aaatgtttct 120
   tttaaagacc ctcctattct ataaaactct gcatgtagag gcttgtttac ctttctctct 180
   ctaaggttta caataggagt ggtgatttga aaaatataaa attatgagat tggttttcct 240
   gtggcataaa ttgcatcact gtatcatttt cttttttaac cggtaagant ttcagtttgt 300
   tggaaagtaa ctgtganaac ccagtttccc gtccatctcc cttagggact acccatagaa 360
   catgaaaagg tccccacnga agcaagaaga taagtctttc atggctgctg gttgcttaaa 420
   ccactttaaa accaagaact tccccttgga aa 452
<210> 108
   <211> 502
   <212> DNA
   <213> Homo sapien
<220>
   <221> misc_feature
   <222> (1)...(502)
   <223> n = A,T,C or G
<400> 108
   atcttcttcc cttaattagt tnttatttat ntattaaatt ttattgcatg tcctggcaaa 60
   caaaaagaga ttgtagattg gcttctggct ccccaaaagc ccataacaga aagtaccaca 120
   agaccncaac tgaagcttaa aaaatctatc acatgtataa tacctttnga agaacattaa 180
   tanagcatat aaaactttta acatntgctt aatgttgtnc aattataaaa ntaatngaaa 240
   aaaatgtccc tttaacatnc aatatcccac atagtgttat ttnaggggat taccnngnaa 300
   naaaaaaagg gtagaaggga tttaatgaaa actctgcttn ccatttctgt ttanaaacgt 360
   ctccagaaca aaaacttntc aantctttca gctaaccgca tttgagctna ggccactcaa 420
   aaactccatt agncccactt tctaanggtc tctanagctt actaancctt ttgacccctt 480
   accctggnta ctcctgccct ca 502
<210> 109
   <211> 1308
   <212> DNA
   <213> Homo sapien
<400> 109
   acccgaggtc tcgctaaaat catcatggat tcacttggcg ccgtcagcac tcgacttggg     60
   tttgatcttt tcaaagagct gaagaaaaca aatgatggca acatcttctt ttcccctgtg    120
   ggcatcttga ctgcaattgg catggtcctc ctggggaccc gaggagccac cgcttcccag    180
   ttggaggagg tgtttcactc tgaaaaagag acgaagagct caagaataaa ggctgaagaa    240
   aaagaggtga ttgagaacac agaagcagta catcaacaat tccaaaagtt tttgactgaa    300
   ataagcaaac tcactaatga ttatgaactg aacataacca acaggctgtt tggagaaaaa    360
   acatacctct tccttcaaaa atacttagat tatgttgaaa aatattatca tgcatctctg    420
   gaacctgttg attttgtaaa tgcagccgat gaaagtcgaa agaagattaa ttcctgggtt    480
   gaaagcaaaa caaatgaaaa aatcaaggac ttgttcccag atggctctat tagtagctct    540
   accaagctgg tgctggtgaa catggtttat tttaaagggc aatgggacag ggagtttaag    600
   aaagaaaata ctaaggaaga gaaattttgg atgaataaga gcacaagtaa atctgtacag    660
   atgatgacac agagccattc ctttagcttc actttcctgg aggacttgca ggccaaaatt    720
   ctagggattc catataaaaa caacgaccta agcatgtttg tgcttctgcc caacgacatc    780
   gatggcctgg agaagataat agataaaata agtcctgaga aattggtaga gtggactagt    840
   ccagggcata tggaagaaag aaaggtgaat ctgcacttgc cccggtttga ggtggaggac     900
   agttacgatc tagaggcggt cctggctgcc atggggatgg gcgatgcctt cagtgagcac    960
   aaagccgact actcgggaat gtcgtcaggc tccgggttgt acgcccagaa gttcctgcac   1020
   agttcctttg tggcagtaac tgaggaaggc accgaggctg cagctgccac tggcataggc   1080
   tttactgtca catccgcccc aggtcatgaa aatgttcact gcaatcatcc cttcctgttc   1140
   ttcatcaggc acaatgaatc caacagcatc ctcttcttcg gcagattttc ttctccttaa   1200
   gatgatcgtt gccatggcat tgctgctttt agcaaaaaac aactaccagt gttactcata   1260
   tgattatgaa aatcgtccat tcttttaaat ggtggctcac ttgcattt                 1308
<210> 110
   <211> 391
   <212> PRT
   <213> Homo sapien
<400> 110
<210> 111
   <211> 1419
   <212> DNA
   <213> Homo sapien
<400> 111
   ggagaactat aaattaagga tcccagctac ttaattgact tatgcttcct agttcgttgc      60
   ccagccacca ccgtctctcc aaaaacccga ggtctcgcta aaatcatcat ggattcactt     120
   ggcgccgtca gcactcgact tgggtttgat cttttcaaag agctgaagaa aacaaatgat     180
   ggcaacatct tcttttcccc tgtgggcatc ttgactgcaa ttggcatggt cctcctgggg     240
   acccgaggag ccaccgcttc ccagttggag gaggtgtttc actctgaaaa agagacgaag     300
   agctcaagaa taaaggctga agaaaaagag gtggtaagaa taaaggctga aggaaaagag     360
   attgagaaca cagaagcagt acatcaacaa ttccaaaagt ttttgactga aataagcaaa      420
   ctcactaatg attatgaact gaacataacc aacaggctgt ttggagaaaa aacatacctc     480
   ttccttcaaa aatacttaga ttatgttgaa aaatattatc atgcatctct ggaacctgtt     540
   gattttgtaa atgcagccga tgaaagtcga aagaagatta attcctgggt tgaaagcaaa     600
   acaaatgaaa aaatcaagga cttgttccca gatggctcta ttagtagctc taccaagctg     660
   gtgctggtga acatggttta ttttaaaggg caatgggaca gggagtttaa gaaagaaaat     720
   actaaggaag agaaattttg gatgaataag agcacaagta aatctgtaca gatgatgaca     780
   cagagccatt cctttagctt cactttcctg gaggacttgc aggccaaaat tctagggatt     840
   ccatataaaa acaacgacct aagcatgttt gtgcttctgc ccaacgacat cgatggcctg     900
   gagaagataa tagataaaat aagtcctgag aaattggtag agtggactag tccagggcat     960
   atggaagaaa gaaaggtgaa tctgcacttg ccccggtttg aggtggagga cagttacgat    1020
   ctagaggcgg tcctggctgc catggggatg ggcgatgcct tcagtgagca caaagccgac    1080
   tactcgggaa tgtcgtcagg ctccgggttg tacgcccaga agttcctgca cagttccttt    1140
   gtggcagtaa ctgaggaagg caccgaggct gcagctgcca ctggcatagg ctttactgtc    1200
   acatccgccc caggtcatga aaatgttcac tgcaatcatc ccttcctgtt cttcatcagg    1260
   cacaatgaat ccaacagcat cctcttcttc ggcagatttt cttctcctta agatgatcgt    1320
   tgccatggca ttgctgcttt tagcaaaaaa caactaccag tgttactcat atgattatga    1380
   aaatcgtcca ttcttttaaa tggtggctca cttgcattt                           1419
<210> 112
   <211> 400
   <212> PRT
   <213> Homo sapien
<400> 112
<210> 113
   <211> 957
   <212> DNA
   <213> Homo sapien
<400> 113
   ctcgaccttc tctgcacagc ggatgaaccc tgagcagctg aagaccagaa aagccactat      60
   gactttctgc ttaattcagg agcttacagg attcttcaaa gagtgtgtcc agcatccttt     120
   gaaacatgag ttcttaccag cagaagcaga cctttacccc accacctcag cttcaacagc     180
   agcaggtgaa acaacccagc cagcctccac ctcaggaaat atttgttccc acaaccaagg     240
   agccatgcca ctcaaaggtt ccacaacctg gaaacacaaa gattccagag ccaggctgta     300
   ccaaggtccc tgagccaggc tgtaccaagg tccctgagcc aggttgtacc aaggtccctg     360
   agccaggatg taccaaggtc cctgagccag gttgtaccaa ggtccctgag ccaggctaca     420
   ccaaggtccc tgagccaggc agcatcaagg tccctgacca aggcttcatc aagttccctg     480
   agccaggtgc catcaaagtt cctgagcaag gatacaccaa agttcctgtg ccaggctaca     540
   caaaggtacc agagccatgt ccttcaacgg tcactccagg cccagctcag cagaagacca     600
   agcagaagta atttggtgca cagacaagcc cttgagaagc caaccaccag atgctggaca     660
   ccctcttccc atctgtttct gtgtcttaat tgtctgtaga ccttgtaatc agtacattcc     720
   caccccaagc catagtctct ctcttatttg tatcctaaaa atacggtact ataaagcttt     780
   tgttcacaca cactctgaag aatcctgtaa gcccctgaat taagcagaaa gtcttcatgg     840
   ctttttcggt cttcggctgc tcagggttca tctgaagatt cgaatgaaaa gaaatgcatg     900
   tttcctgctc tgccctcatt aaattgcttt taattccaaa aaaaaaaaaa aaaaaaa        957
<210> 114
   <211> 161
   <212> PRT
   <213> Homo sapien
<400> 114
   Met Ser Ser Tyr Gln Gln Lys Gln Thr Phe Thr Pro Pro Pro Gln Leu
    1               5                  10                  15
   Gln Gln Gln Gln Val Lys Gln Pro Ser Gln Pro Pro Pro Gln Glu Ile
               20                  25                  30
   Phe Val Pro Thr Thr Lys Glu Pro Cys His Ser Lys Val Pro Gln Pro
           35                  40                  45
   Gly Asn Thr Lys Ile Pro Glu Pro Gly Cys Thr Lys Val Pro Glu Pro
       50                  55                  60
   Gly Cys Thr Lys Val Pro Glu Pro Gly Cys Thr Lys Val Pro Glu Pro
   65                  70                  75                  80
   Gly Cys Thr Lys Val Pro Glu Pro Gly Cys Thr Lys Val Pro Glu Pro
                   85                  90                  95
   Gly Tyr Thr Lys Val Pro Glu Pro Gly Ser Ile Lys Val Pro Asp Gln
               100                 105                 110
   Gly Phe Ile Lys Phe Pro Glu Pro Gly Ala Ile Lys Val Pro Glu Gln
           115                 120                 125
   Gly Tyr Thr Lys Val Pro Val Pro Gly Tyr Thr Lys Val Pro Glu Pro
       130                 135                 140
   Cys Pro Ser Thr Val Thr Pro Gly Pro Ala Gln Gln Lys Thr Lys Gln
   145                 150                 155                 160
   Lys
<210> 115
   <211> 506
   <212> DNA
   <213> Homo sapien
<220>
   <221> misc_feature
   <222> (1) ... (506)
   <223> n = A, T, C or G
<400> 115
   cattggtnct ttcatttgct ntggaagtgt nnatctctaa cagtggacaa agttcccngt      60
   gccttaaact ctgtnacact tttgggaant gaaaanttng tantatgata ggttattctg     120
   angtanagat gttctggata ccattanatn tgcccccngt gtcagaggct catattgtgt     180
   tatgtaaatg gtatntcatt cgctactatn antcaattng aaatanggtc tttgggttat     240
   gaatantnng cagcncanct nanangctgt ctgtngtatt cattgtggtc atagcacctc     300
   acancattgt aacctcnatc nagtgagaca nactagnaan ttcctagtga tggctcanga     360
   ttccaaatgg nctcatntcn aatgtttaaa agttanttaa gtgtaagaaa tacagactgg     420
   atgttccacc aactagtacc tgtaatgacn ggcctgtccc aacacatctc ccttttccat     480
   gactgtggta ncccgcatcg gaaaaa                                          506
<210> 116
   <211> 3079
   <212> DNA
   <213> Homo sapien
<400> 116
   ggatccccgg gtttcctaaa ccccccacag agtcctgccc aggccaaaga gcaaggaaaa      60
   ggtcaaaggg cagaaaaaat gctgagttag gaggagctat ggaaggataa acctggcctt     120
   aaagaggtca aagtggttta tagggggcgc tgagggcttc ccacattctc tggcctaaac     180
   cttgcaggca gatctgccca gtgggctctg ggatagctgt gccttcccta acaaaaaaat     240
   tgtgcacaaa aggatgaaac tctattttcc ctctagcaca taaccaagaa tataaggcta     300
   cagattgcct ttcccagagg gaaaaccctg cagcaacctg ctgcctggaa aagtgtaaga     360
   gcagatcact ggggaatcgt ttgccccccg ctgatggaca gcttccccaa gctccaaggg     420
   caggtgctca gcatgtaccg tactgggatg gttgtcaata ctcctggtcc tgtaagagtc     480
   ccaggacact gccatgccaa tgccccctca gttcctggca tcctttttgg gctgctcaca     540
   gccccagcct ctatggtgaa gacatacttg ctagcagcgt caccaacttg ttgccaagag     600
   atcagtgctc gaaggcaagg ttatttctaa ctgagcagag cctgccagga agaaagcgtt     660
   tgcaccccac accactgtgc aggtgtgacc ggtgagctca cagctgcccc ccaggcatgc     720
   ccagcccact taatcatcac agctcgacag ctctctcgcc cagcccagtt ctggaaggga     780
   taaaaagggg catcaccgtt cctgggtaac agagccacct tctgcgtcct gctgagctct     840
   gttctctcca gcacctccca acccactagt gcctggttct cttgctccac caggaacaag     900
   ccaccatgtc tcgccagtca agtgtgtctt ccggagcggg gggcagtcgt agcttcagca     960
   ccgcctctgc catcaccccg tctgtctccc gcaccagctt cacctccgtg tcccggtccg    1020
   ggggtggcgg tggtggtggc ttcggcaggg tcagccttgc gggtgcttgt ggagtgggtg    1080
   gctatggcag ccggagcctc tacaacctgg ggggctccaa gaggatatcc atcagcacta    1140
   gtggtggcag cttcaggaac cggtttggtg ctggtgctgg aggcggctat ggctttggag    1200
   gtggtgccgg tagtggattt ggtttcggcg gtggagctgg tggtggcttt gggctcggtg    1260
   gcggagctgg ctttggaggt ggcttcggtg gccctggctt tcctgtctgc cctcctggag    1320
   gtatccaaga ggtcactgtc aaccagagtc tcctgactcc cctcaacctg caaatcgacc    1380
   ccagcatcca gagggtgagg accgaggagc gcgagcagat caagaccctc aacaataagt    1440
   ttgcctcctt catcgacaag gtgcggttcc tggagcagca gaacaaggtt ctggaaacaa    1500
   agtggaccct gctgcaggag cagggcacca agactgtgag gcagaacctg gagccgttgt    1560
   tcgagcagta catcaacaac ctcaggaggc agctggacag catcgtgggg gaacggggcc    1620
   gcctggactc agagctgaga aacatgcagg acctggtgga agacttcaag aacaagtatg    1680
   aggatgaaat caacaagcgt accactgctg agaatgagtt tgtgatgctg aagaaggatg    1740
   tagatgctgc ctacatgaac aaggtggagc tggaggccaa ggttgatgca ctgatggatg    1800
   agattaactt catgaagatg ttctttgatg cggagctgtc ccagatgcag acgcatgtct    1860
   ctgacacctc agtggtcctc tccatggaca acaaccgcaa cctggacctg gatagcatca    1920
   tcgctgaggt caaggcccag tatgaggaga ttgccaaccg cagccggaca gaagccgagt    1980
   cctggtatca gaccaagtat gaggagctgc agcagacagc tggccggcat ggcgatgacc    2040
   tccgcaacac caagcatgag atctctgaga tgaaccggat gatccagagg ctgagagccg    2100
   agattgacaa tgtcaagaaa cagtgcgcca atctgcagaa cgccattgcg gatgccgagc    2160
   agcgtgggga gctggccctc aaggatgcca ggaacaagct ggccgagctg gaggaggccc    2220
   tgcagaaggc caagcaggac atggcccggc tgctgcgtga gtaccaggag ctcatgaaca    2280
   ccaagctggc cctggacgtg gagatcgcca cttaccgcaa gctgctggag ggcgaggaat    2340
   gcagactcag tggagaagga gttggaccag tcaacatctc tgttgtcaca agcagtgttt    2400
   cctctggata tggcagtggc agtggctatg gcggtggcct cggtggaggt cttggcggcg    2460
   gcctcggtgg aggtcttgcc ggaggtagca gtggaagcta ctactccagc agcagtgggg    2520
   gtgtcggcct aggtggtggg ctcagtgtgg ggggctctgg cttcagtgca agcagtagcc    2580
   gagggctggg ggtgggcttt ggcagtggcg ggggtagcag ctccagcgtc aaatttgtct    2640
   ccaccacctc ctcctcccgg aagagcttca agagctaaga acctgctgca agtcactgcc    2700
   ttccaagtgc agcaacccag cccatggaga ttgcctcttc taggcagttg ctcaagccat    2760
   gttttatcct tttctggaga gtagtctaga ccaagccaat tgcagaacca cattctttgg    2820
   ttcccaggag agccccattc ccagcccctg gtctcccgtg ccgcagttct atattctgct    2880
   tcaaatcagc cttcaggttt cccacagcat ggcccctgct gacacgagaa cccaaagttt    2940
   tcccaaatct aaatcatcaa aacagaatcc ccaccccaat cccaaatttt gttctggttc    3000
   taactacctc cagaatgtgt tcaataaaat gttttataat ataagctggt gtgcagaatt    3060
   gttttttttt tctacccaa                                                 3079
<210>117
   <211> 6921
   <212> DNA
   <213> Homo sapien
<400> 117
<210> 118
   <211> 946
   <212> DNA
   <213> Homo sapien
<400> 118
<210> 119
   <211> 8948
   <212> DNA
   <213> Homo sapien
<400> 119
<210> 120
   <211> 587
   <212> DNA
   <213> Homo sapien
<220>
   <221> misc_feature
   <222> (1)...(587)
   <223> n = A,T,C or G
<400> 120
   cgtcctaagc acttagacta catcagggaa gaacacagac cacatccctg tcctcatgcg 60
   gcttatgttt tctggaagaa agtggagacc nagtccttgg ctttagggct ccccggctgg 120
   gggctgtgca ntccggtcag ggcgggaagg gaaatgcacc gctgcatgtg aacttacagc 180
   ccaggcggat gccccttccc ttagcactac ctggcctcct gcatcccctc gcctcatgtt 240
   cctcccacct tcaaanaatg aanaacccca tgggcccagc cccttgccct ggggaaccaa 300
   ggcagccttc caaaactcag gggctgaagc anactattag ggcaggggct gactttgggt 360
   gacactgccc attccctctc agggcagctc angtcacccn ggnctcttga acccagcctg 420
   ttcctttgaa aaagggcaaa actgaaaagg gcttttccta naaaaagaaa aaccagggaa 480
   ctttgccagg gcttcnntnt taccaaaacn ncttctcnng gatttttaat tccccattng 540
   gcctccactt accnggggcn atgccccaaa attaanaatt tcccatc 587
<210> 121
   <211> 619
   <212> DNA
   <213> Homo sapien
<220>
   <221> misc_feature
   <222> (1)...(619)
   <223> n = A,T,C or G
<400> 121
   cactagtagg atagaaacac tgtgtcccga gagtaaggag agaagctact attgattaga 60
   gcctaaccca ggttaactgc aagaagaggc gggatacttt cagctttcca tgtaactgta 120
   tgcataaagc caatgtagtc cagtttctaa gatcatgttc caagctaact gaatcccact 180
   tcaatacaca ctcatgaact cctgatggaa caataacagg cccaagcctg tggtatgatg 240
   tgcacacttg ctagactcan aaaaaatact actctcataa atgggtggga gtattttggt 300
   gacaacctac tttgcttggc tgagtgaagg aatgatattc atatattcat ttattccatg 360
   gacatttagt tagtgctttt tatataccag gcatgatgct gagtgacact cttgtgtata 420
   tttccaaatt tttgtacagt cgctgcacat atttgaaatc atatattaag acttccaaaa 480
   aatgaagtcc ctggtttttc atggcaactt gatcagtaaa ggattcncct ctgtttggta 540
   cttaaaacat ctactatatn gttnanatga aattcctttt ccccncctcc cgaaaaaana 600
   aagtggtggg gaaaaaaaa 619
<210> 122
   <211> 1475
   <212> DNA
   <213> Homo sapien
<400> 122
   tccacctgtc cccgcagcgc cggctcgcgc cctcctgccg cagccaccga gccgccgtcc 60
   agcgccccga cctcgccacc atgagagccc tgctggcgcg cctgcttctc tgcgtcctgg 120
   tcgtgagcga ctccaaaggc agcaatgaac ttcatcaagt tccatcgaac tgtgactgtc     180
   taaatggagg aacatgtgtg tccaacaagt acttctccaa cattcactgg tgcaactgcc     240
   caaagaaatt cggagggcag cactgtgaaa tagataagtc aaaaacctgc tatgagggga     300
   atggtcactt ttaccgagga aaggccagca ctgacaccat gggccggccc tgcctgccct     360
   ggaactctgc cactgtcctt cagcaaacgt accatgccca cagatctgat gctcttcagc     420
   tgggcctggg gaaacataat tactgcagga acccagacaa ccggaggcga ccctggtgct     480
   atgtgcaggt gggcctaaag ccgcttgtcc aagagtgcat ggtgcatgac tgcgcagatg     540
   gaaaaaagcc ctcctctcct ccagaagaat taaaatttca gtgtggccaa aagactctga     600
   ggccccgctt taagattatt gggggagaat tcaccaccat cgagaaccag ccctggtttg     660
   cggccatcta caggaggcac cgggggggct ctgtcaccta cgtgtgtgga ggcagcctca     720
   tcagcccttg ctgggtgatc agcgccacac actgcttcat tgattaccca aagaaggagg     780
   actacatcgt ctacctgggt cgctcaaggc ttaactccaa cacgcaaggg gagatgaagt     840
   ttgaggtgga aaacctcatc ctacacaagg actacagcgc tgacacgctt gctcaccaca     900
   acgacattgc cttgctgaag atccgttcca aggagggcag gtgtgcgcag ccatcccgga     960
   ctatacagac catctgcctg ccctcgatgt ataacgatcc ccagtttggc acaagctgtg    1020
   agatcactgg ctttggaaaa gagaattcta ccgactatct ctatccggag cagctgaaga    1080
   tgactgttgt gaagctgatt tcccaccggg agtgtcagca gccccactac tacggctctg    1140
   aagtcaccac caaaatgctg tgtgctgctg acccacagtg gaaaacagat tcctgccagg    1200
   gagactcagg gggacccctc gtctgttccc tccaaggccg catgactttg actggaattg    1260
   tgagctgggg ccgtggatgt gccctgaagg acaagccagg cgtctacacg agagtctcac    1320
   acttcttacc ctggatccgc agtcacacca aggaagagaa tggcctggcc ctctgagggt    1380
   ccccagggag gaaacgggca ccacccgctt tcttgctggt tgtcattttt gcagtagagt    1440
   catctccatc agctgtaaga agagactggg aagat                               1475
<210> 123
   <211> 2294
   <212> DNA
   <213> Homo sapien
<400> 123
   cagcgccggc tcgcgccctc ctgccgcagc caccgagccg ccgtctagcg ccccgacctc      60
   gccaccatga gagccctgct ggcgcgcctg cttctctgcg tcctggtcgt gagcgactcc     120
   aaaggcagca atgaacttca tcaagttcca tcgaactgtg actgtctaaa tggaggaaca     180
   tgtgtgtcca acaagtactt ctccaacatt cactggtgca actgcccaaa gaaattcgga     240
   gggcagcact gtgaaataga taagtcaaaa acctgctatg aggggaatgg tcacttttac     300
   cgaggaaagg ccagcactga caccatgggc cggccctgcc tgccctggaa ctctgccact     360
   gtccttcagc aaacgtacca tgcccacaga tctgatgctc ttcagctggg cctggggaaa     420
   cataattact gcaggaaccc agacaaccgg aggcgaccct ggtgctatgt gcaggtgggc     480
   ctaaagccgc ttgtccaaga gtgcatggtg catgactgcg cagatggaaa aaagccctcc      540
   tctcctccag aagaattaaa atttcagtgt ggccaaaaga ctctgaggcc ccgctttaag     600
   attattgggg gagaattcac caccatcgag aaccagccct ggtttgcggc catctacagg     660
   aggcaccggg ggggctctgt cacctacgtg tgtggaggca gcctcatcag cccttgctgg     720
   gtgatcagcg ccacacactg cttcattgat tacccaaaga aggaggacta catcgtctac     780
   ctgggtcgct caaggcttaa ctccaacacg caaggggaga tgaagtttga ggtggaaaac     840
   ctaatcctac acaaggacta cagcgctgac acgcttgctc accacaacga cattgccttg     900
   ctgaagatcc gttccaagga gggcaggtgt gcgcagccat cccggactat acagaccatc     960
   tgcctgccct cgatgtataa cgatccccag tttggcacaa gctgtgagat cactggcttt    1020
   ggaaaagaga attctaccga ctatctctat ccggagcagc tgaaaatgac tgttgtgaag    1080
   ctgatttccc accgggagtg tcagcagccc cactactacg gctctgaagt caccaccaaa    1140
   atgctgtgtg ctgctgaccc acagtggaaa acagattcct gccagggaga ctcaggggga    1200
   cccctcgtct gttccctcca aggccgcatg actttgactg gaattgtgag ctggggccgt    1260
   ggatgtgccc tgaaggacaa gccaggcgtc tacacgagag tctcacactt cttaccctgg    1320
   atccgcagtc acaccaagga agagaatggc ctggccctct gagggtcccc agggaggaaa    1380
   cgggcaccac ccgctttctt gctggttgct attttgcagt agagtcatct ccatcagctg    1440
   taagaagagc tgggaatata ggctctgcac agatggattt gcctgtgcca ccaccagggc    1500
   gaacgacaat agctttaccc tcaggcatag gcctgggtgc tggctgccca gacccctctg    1560
   gccaggatgg aggggtggtc ctgactcaac atgttactga ccagcaactt gtctttttct    1620
   ggactgaagc ctgcaggagt taaaaagggc agggcatctc ctgtgcatgg gctcgaaggg    1680
   agagccagct cccccgaccg gtgggcattt gtgaggccca tggttgagaa atgaataatt    1740
   tcccaattag gaagtgtaag cagctgaggt ctcttgaggg agcttagcca atgtgggagc    1800
   agcggtttgg ggagcagaga cactaacgac ttcagggcag ggctctgata ttccatgaat    1860
   gtatcaggaa atatatatgt gtgtgtatgt ttgcacactt gtgtgtgggc tgtgagtgta    1920
   agtgtgagta agagctggtg tctgattgtt aagtctaaat atttccttaa actgtgtgga    1980
   ctgtgatgcc acacagagtg gtctttctgg agaggttata ggtcactcct ggggcctctt    2040
   gggtccccca cgtgacagtg cctgggaatg tattattctg cagcatgacc tgtgaccagc    2100
   actgtctcag tttcactttc acatagatgt ccctttcttg gccagttatc ccttcctttt    2160
   agcctagttc atccaatcct cactgggtgg ggtgaggacc actcctgtac actgaatatt    2220
   tatatttcac tatttttatt tatatttttg taattttaaa taaaagtgat caataaaatg    2280
   tgatttttct gatg                                                      2294
<210> 124
   <211> 956
   <212> DNA
   <213> Homo sapien
<400> 124
   gatgagttcc gcaccaagtt tgagacagac caggccctgc gcctgagtgt ggaggccgac      60
   atcaatggcc tgcgcagggt gctggatgag ctgaccctgg ccagagccga cctggagatg     120
   cagattgaga acctcaagga ggagctggcc tacctgaaga agaaccacga ggaggagatg     180
   aacgccctgc gaggccaggt gggtggtgag atcaatgtgg agatggacgc tgccccaggc     240
   gtggacctga gccgcatcct caacgagatg cgtgaccagt atgagaagat ggcagagaag     300
   aaccgcaagg atgccgagga ttggttcttc agcaagacag aggaactgaa ccgcgaggtg     360
   gccaccaaca gtgagctggt gcagagtggc aagagtgaga tctcggagct ccggcgcacc     420
   atgcaggcct tggagataga gctgcagtcc cagctcagca tgaaagcatc cctggagggc     480
   aacctggcgg agacagagaa ccgctactgc gtgcagctgt cccagatcca ggggctgatt     540
   ggcagcgtgg aggagcagct ggcccagctt cgctgcgaga tggagcagca gaaccaggaa     600
   tacaaaatcc tgctggatgt gaagacgcgg ctggagcagg agattgccac ctaccgccgc     660
   ctgctggagg gagaggatgc ccacctgact cagtacaaga aagaaccggt gaccacccgt     720
   caggtgcgta ccattgtgga agaggtccag gatggcaagg tcatctcctc ccgcgagcag     780
   gtccaccaga ccacccgctg aggactcagc taccccggcc ggccacccag gaggcaggga     840
   cgcagccgcc ccatctgccc cacagtctcc ggcctctcca gcctcagccc cctgcttcag     900
   tcccttcccc atgcttcctt gcctgatgac aataaaagct tgttgactca gctatg         956
<210> 125
   <211> 486
   <212> DNA
   <213> Homo sapien
<220>
   <221> misc_feature
   <222> (1)...(486)
   <223> n = A,T,C or G
<400> 125
   aaattatata tagtgnttca gctcccattg tggtgttcat agtcttctag gaacagataa      60
   acttaagtat tcaattcact cttggcattt tttctttaat ataggctttt tagcctattt     120
   ttggaaaact gcttttcttc tgagaacctt attctgaatg tcatcaactt taccaaacct     180
   tctaagtcca gagctaactt agtactgttt aagttactat tgactgaatt ttcttcattt     240
   tctgtttagc cagtgttacc aaggtaagct ggggaatgaa gtataccaac ttctttcaga     300
   gcattttagg acattatggc agctttagaa ggctgtcttg tttctagcca agggagagcc     360
   agcgcaggtt ttggatacta gagaaagtca tttgcttgta ctattgccat tttagaaagc 420
   tctgatgtga attcaaattt tacctctgtt acttaaagcc aacaatttta aggcagtagt 480
   tttact 486
<210> 126
   <211> 3552
   <212> DNA
   <213> Homo sapien
<400> 126
   cggcaggcag gtctcgtctc ggcaccctcc cggcgcccgc gttctcctgg ccctgcccgg 60
   catcccgatg gccgccgctg ggccccggcg ctccgtgcgc ggagccgtct gcctgcatct 120
   gctgctgacc ctcgtgatct tcagtcgtgc tggtgaagcc tgcaaaaagg tgatacttaa 180
   tgtaccttct aaactagagg cagacaaaat aattggcaga gttaatttgg aagagtgctt 240
   caggtctgca gacctcatcc ggtcaagtga tcctgatttc agagttctaa atgatgggtc 300
   agtgtacaca gccagggctg ttgcgctgtc tgataagaaa agatcattta ccatatggct 360
   ttctgacaaa aggaaacaga cacagaaaga ggttactgtg ctgctagaac atcagaagaa 420
   ggtatcgaag acaagacaca ctagagaaac tgttctcagg cgtgccaaga ggagatgggc 480
   acctattcct tgctctatgc aagagaattc tttgggccct ttcccattgt ttcttcaaca 540
   agttgaatct gatgcagcac agaactatac tgtcttctac tcaataagtg gacgtggagt 600
   tgataaagaa cctttaaatt tgttttatat agaaagagac actggaaatc tattttgcac 660
   tcggcctgtg gatcgtgaag aatatgatgt ttttgatttg attgcttatg cgtcaactgc 720
   agatggatat tcagcagatc tgcccctccc actacccatc agggtagagg atgaaaatga 780
   caaccaccct gttttcacag aagcaattta taattttgaa gttttggaaa gtagtagacc 840
   tggtactaca gtgggggtgg tttgtgccac agacagagat gaaccggaca caatgcatac 900
   gcgcctgaaa tacagcattt tgcagcagac accaaggtca cctgggctct tttctgtgca 960
   tcccagcaca ggcgtaatca ccacagtctc tcattatttg gacagagagg ttgtagacaa 1020
   gtactcattg ataatgaaag tacaagacat ggatggccag ttttttggat tgataggcac 1080
   atcaacttgt atcataacag taacagattc aaatgataat gcacccactt tcagacaaaa 1140
   tgcttatgaa gcatttgtag aggaaaatgc attcaatgtg gaaatcttac gaatacctat 1200
   agaagataag gatttaatta acactgccaa ttggagacgt aattttacca ttttaaaggg 1260
   aaatgaaaat ggacatttca aaatcagcac agacaaagca acaaatgaag gtgttctttc 1320
   tgttgtaaag ccactgaatt atgaagaaaa ccgtcaagtg aacctggaaa ttggagtaaa 1380
   caatgaagcg ccatttgcta gagatattcc cagagtgaca gccttgaaca gagccttggt 1440
   tacagttcat gtgagggatc tggatgaggg gcctgaatgc actcctgcag cccaatatgt 1500
   gcggattaaa gaaaacttag cagtggggtc aaagatcaac ggctataagg catatgaccc 1560
   cgaaaataga aatggcaatg gtttaaggta caaaaaattg catgatccta aaggttggat 1620
   caccattgat gaaatttcag ggtcaatcat aacttccaaa atcctggata gggaggttga 1680
   aactcccaaa aatgagttgt ataatattac agtcctggca atagacaaag atgatagatc 1740
   atgtactgga acacttgctg tgaacattga agatgtaaat gataatccac cagaaatact 1800
   tcaagaatat gtagtcattt gcaaaccaaa aatggggtat accgacattt tagctgttga 1860
   tcctgatgaa cctgtccatg gagctccatt ttatttcagt ttgcccaata cttctccaga 1920
   aatcagtaga ctgtggagcc tcaccaaagt taatgataca gctgcccgtc tttcatatca 1980
   gaaaaatgct ggatttcaag aatataccat tcctattact gtaaaagaca gggccggcca 2040
   agctgcaaca aaattattga gagttaatct gtgtgaatgt actcatccaa ctcagtgtcg 2100
   tgcgacttca aggagtacag gagtaatact tggaaaatgg gcaatccttg caatattact 2160
   gggtatagca ctgctctttt ctgtattgct aactttagta tgtggagttt ttggtgcaac 2220
   taaagggaaa cgttttcctg aagatttagc acagcaaaac ttaattatat caaacacaga 2280
   agcacctgga gacgatagag tgtgctctgc caatggattt atgacccaaa ctaccaacaa 2340
   ctctagccaa ggtttttgtg gtactatggg atcaggaatg aaaaatggag ggcaggaaac 2400
   cattgaaatg atgaaaggag gaaaccagac cttggaatcc tgccgggggg ctgggcatca 2460
   tcataccctg gactcctgca ggggaggaca cacggaggtg gacaactgca gatacactta 2520
   ctcggagtgg cacagtttta ctcaaccccg tctcggtgaa aaattgcatc gatgtaatca 2580
   gaatgaagac cgcatgccat cccaagatta tgtcctcact tataactatg agggaagagg 2640
   atctccagct ggttctgtgg gctgctgcag tgaaaagcag gaagaagatg gccttgactt 2700
   tttaaataat ttggaaccca aatttattac attagcagaa gcatgcacaa agagataatg 2760
   tcacagtgct acaattaggt ctttgtcaga cattctggag gtttccaaaa ataatattgt 2820
   aaagttcaat ttcaacatgt atgtatatga tgattttttt ctcaattttg aattatgcta 2880
   ctcaccaatt tatattttta aagcaagttg ttgcttatct tttccaaaaa gtgaaaaatg 2940
   ttaaaacaga caactggtaa atctcaaact ccagcactgg aattaaggtc tctaaagcat 3000
   ctgctctttt ttttttttac agatatttta gtaataaata tgctggataa atattagtcc 3060
   aacaatagct aagttatgct aatatcacat tattatgtat tcactttaag tgatagttta 3120
   aaaaataaac aagaaatatt gagtatcact atgtgaagaa agttttggaa aagaaacaat 3180
   gaagactgaa ttaaattaaa aatgttgcag ctcataaaga attggactca cccctactgc 3240
   actaccaaat tcatttgact ttggaggcaa aatgtgttga agtgccctat gaagtagcaa 3300
   ttttctatag gaatatagtt ggaaataaat gtgtgtgtgt atattattat taatcaatgc 3360
   aatatttaaa tgaaatgaga acaaagagga aaatggtaaa aacttgaaat gaggctgggg 3420
   tatagtttgt cctacaatag aaaaaagaga gagcttccta ggcctgggct cttaaatgct 3480
   gcattataac tgagtctatg aggaaatagt tcctgtccaa tttgtgtaat ttgtttaaaa 3540
   ttgtaaataa at 3552
<210> 127
   <211> 754
   <212> DNA
   <213> Homo sapien
<400> 127
   tttttttttt ttgtcattgt tcattgattt taatgagaaa gctaagagag gaaataagta 60
   gcctttcaaa ggtcacacag aagtaagtga cagatccagg atttatatcc aagcattctg 120
   gctctagtgt ccatgcttct caaccattat gacccaatat tcaaccaaat caatactgaa 180
   ggacacgtga aatgtatccg gtattttact attacaaaca aaaatccaat gaacattctt 240
   gaagacatac acaaaaataa tggttacaat agaagttact ggaattgaaa ttttggttca 300
   acctatatta aaatgtaagg cttttgatat agctaataga tttttgaaat gatcagtctt 360
   aacgtttgta ggggagcaca ctcctgcatg gggaaaagat tcactgtgaa gcacagagca 420
   cctttatggt tggatcatct tgtcattaaa gttcaggcgt tatctatcct gtaagtggca 480
   gaatcaagac tgcaatatcg cctgcttttc tttttaactc atgttttccc ttgactacac 540
   tggtcctcaa agtaaaaccc ctgtgtcagt gtactattca tggaatactc tgcaattata 600
   accaccttct aatactttta atacccaatc aaaatttatt atacatatgt atcatagata 660
   ctcatctgta aagctgtgct tcaaaatagt gatctcttcc caacattaca atatatatta 720
   atgatgtcga acctgcccgg gcggccgctc gaag 754
<210> 128
   <211> 374
   <212> DNA
   <213> Homo sapien
<400> 128
   aggttttgat taaaaaggca aatgatttta ttgttcgata atcttttaaa aaaataagag 60
   gaaggagtaa aattaaagat gaaagatgat ttttatttcc ttgtgacctc tatatccccc 120
   ttcccctgcc cttggtaagt aactcttgat ggagaaagga ttaaagaccc ttatttaacc 180
   aaaaaacaga gccagctaat catttccaaa ggttagtatc tccctgctga cctcttcttt 240
   ggtttaattg aataaaacta tatgttcata tatgtattaa aacaactcag aataacatct 300
   tttcttcctt agttaaggca ttataagggc tatactatca tccataataa ccaaggcaat 360
   aacttaaaaa gctg 374
<210> 129
   <211> 546
   <212> DNA
   <213> Home sapien
<400> 129
<210> 130
   <211> 5156
   <212> DNA
   <213> Homo sapien
<400> 130
<210> 131
   <211> 671
   <212> DNA
   <213> Homo sapien
<400> 131
   aggtctggag ggcccacagc cggatgtggg acaccgggaa aaagtggtca tagcacacat 60
   ttttgcatcc cggttgcagt gtgttgcaga cgaagtcctc ttgctcgtca ccccacactt 120
   cctgggcagc caycacgagg atcatgactc ggaaaataaa gatgactgtg atccacacct 180
   tcccgatgct ggtggagtgt ttgttgacac ccccgatgaa agtgtgcagc gtcccccaat 240
   ccattgcgct ggtttatccc tgagtcctgt ttccaacgac tgccagtgtt tcagacccaa 300
   agaatgaggg caagatccct ctgcgagggt ttcagacctc cttctcctac cccactggag 360
   tgcctagaag ccaatgggtg cacagtgatg atacgaatgt caatctttgc tcggtcagtg 420
   aggatgtcgc ctggaatatt caaattgaat tacagatgca tgaagagggc gtacaagtta 480
   gaatttttct ttcgccatac agaaattgtt tagccagatc ttctgtactt cttttccttc 540
   cctgaccctt cctgctcccc aggaagggag gtcagccccg tttgcaaaac acaggatgcc 600
   cgtgacaccg gagacaggtc ttcttcaccg acaggaagtg ccttctggtg cctgcacgtt 660
   ttaactgcta t 671
<210> 132
   <211> 590
   <222> DNA
   <213> Homo sapien
<400> 132
   ctgaatggaa aagcttatgg ctctgtgatg atattagtga ccagcggaga tgataagctt 60
   cttggcaatt gcttacccac tgtgctcagc agtggttcaa caattcactc cattgccctg 120
   ggttcatctg cagccccaaa tctggaggaa ttatcacgtc ttacaggagg tttaaagttc 180
   tttgttccag atatatcaaa ctccaatagc atgattgatg ctttcagtag aatttccctt 240
   ggaactggag acattttcca gcaacatatt cagcttgaaa gtacaggtga aaatgtcaaa 300
   cctcaccatc aattgaaaaa cacagtgact gtggataata ctgtgggcaa cgacactatg 360
   tttctagtta cgtggcaggc cagtggtcct cctgagatta tattatttga tcctgatgga 420
   cgaaaatact acacaaataa ttttatcacc aatctaactt ttcggacagc tagtctttgg 480
   attccaggaa cagctaagcc tgggcactgg acttacaccc tgaacaatac ccatcattct 540
   ctgcaagccc tgaaagtgac agtgacctct cgcgcctcca actcagacct 590
<210> 133
   <211> 581
   <212> DNA
   <213> Homo sapien
<400> 133
   aggtcctgtc cgggggcact gagaactccc tctggaattc ttggggggtg ttggggagag 60
   actgtgggcc tggagataaa acttgtctcc tctaccacca ccctgtaccc tagcctgcac 120
   ctgtcctcat ctctgcaaag ttcagcttcc ttccccaggt ctctgtgcac tctgtcttgg 180
   atgctctggg gagctcatgg gtggaggagt ctccaccaga gggaggctca ggggactggt 240
   tgggccaggg atgaatattt gagggataaa aattgtgtaa gagccaaaga attggtagta 300
   gggggagaac agagaggagc tgggctatgg gaaatgattt gaataatgga gctgggaata 360
   tggctggata tctggtacta aaaaagggtc tttaagaacc cacttcctaa tctcttcccc 420
   aatccaaacc atagctgtct gtccagtgct ctcttcctgc ctccagctct gccccaggct 480
   cctcctagac tctgtccctg ggctagggca ggggaggagg gagagcaggg ttgggggaga 540
   ggctgaggag agtgtgacat gtggggagag gaccagacct c 581
<210> 134
   <211> 4797
   <212> DNA
   <213> Homo sapien
<220>
   <221> misc_feature
   <222> (1)...(4797)
   <223> n = A,T,C or G
<400> 134
   cctgggacca aagtgctgcc cagagctgag ggtcctggag ccacatgaga aggcttctcc     60
   ctgtgtacct gtgcagcaca gggtagggtg agtccactca gctgtctagg agaggaccca    120
   ggagcagcag agacncgcca agcctttact cataccatat tctgatcctt ttccagcaaa    180
   ttgtggctac taatttgccc cctgaagatc aagatggctc tggggatgac tctgacaact    240
   tctccggctc aggtgcaggt gaggttgtca tgggggcccc ccccacccaa gacggcaaca    300
   ggtcatgcct gggggcagtg gtcaggcagt ctcctgtgtt tactgagcat gtactgagtg    360
   caccctgcct gccctgtctc cacccagctg gctccaaagg gcaatgctga ggagaggaat    420
   ggggtcgtga gctgctgtta aggagagctc atgcttggag gtgaggtgaa ggctgtgagc    480
   tccagaaggc cccagggcgc nctgctgcac gcaggctcat attcactagg aatagcttta    540
   ctcactaaga aacctctgga acccccttca gaaggttatt tgactcctga gcctctattt    600
   tctcatctgc aaaatgggaa taataccttg acctgataag cttgtggagc tgtaaggcag    660
   cacagagcca gctggggtgt agctcttcca tccaagctcc cttccttact tcccctttcc    720
   tgtggggact gggggagaga agtccctgag ctggaggtgg tcagggaagc ttcacagagg    780
   aggtggctct tgagtggacc tcaggaagag gggtgagaga gctaaggaag gaggctgagg    840
   tcatccctgg ggaagtgacc tagcggaggc ctgagagctg caaggtagga tatctgttgt    900
   tggaagtgtc tgttgttgga agtgggggcc tttttttcag ggagggtggg gccagagaag    960
   tgtgtgccct gggataagta ggataaccac agtagttatg cccctaaggg atgcccaccc   1020
   cacccctgtg gtcacagaaa agctttccca ggtggcctag gcacctgtct cgtggctcca   1080
   gagacaggct gcacctgaca cacacaatgg aaggacagct ctccttgtcc attttccaag   1140
   gagcttagcc tcagctgcct tgtccaggta ctagcctccc tcatagcctg agcttggcca   1200
   gcccaggtgc tctggagcct cccccgaccc acccaacaca ctctgcttct ggtcctcccc   1260
   accccccacc tccccaacac actctgcttc tggtcctgca ggtgctttgc aagatatcac   1320
   cttgtcacag cagaccccct ccacttggaa ggacacgcag ctcctgacgg ctattcccac   1380
   gtctccagaa cccaccggcc tggaggctac agctgcctcc acctccaccc tgccggctgg   1440
   agaggggccc aaggagggag aggctgtagt cctgccagaa gtggagcctg gcctcaccgc   1500
   ccgggagcag gaggccaccc cccgacccag ggagaccaca cagctcccga ccactcatca   1560
   ggcctcaacg accacagcca ccacggccca ggagcccgcc acctcccacc cccacaggga   1620
   catgcagcct ggccaccatg agacctcaac ccctgcagga cccagccaag ctgaccttca   1680
   cactccccac acagaggatg gaggtccttc tgccaccgag agggctgctg aggatggagc   1740
   ctccagtcag ctcccagcag cagagggctc tggggagcag gtgagtggcc tctgcattcc   1800
   ttgggaaatt gagtgggttg gtcctaatgc ctggcacttg gcaggcccta cacctgtgcc   1860
   ctgcgcgatc tcgtattcct caccaggaag acagggcaca ggggccgcct tcccctaccc   1920
   ccagggcctc gcagagcagg acagactaac tatgagatca gagcagaagc acccttaaag   1980
   atcacccaag agagggctcc caaactcaca atccaaactt gcagccctcg tcgaagagtg   2040
   aacgttatac cagtcatttt atttatagct tcgtggattt acgcttacac taaatagtct   2100
   gctattcata caaaatgtgt gctttgtatc actttttgtg atatccatgc catggtccag   2160
   ccagggtccg gagttgatgt ggcaagaagg cctggctttc gggccctgtg cgatcctggt   2220
   ttgggtgcat ctgagtgggt ggtggcaaag atcagggagg caggagctgc ttctgggtct   2280
   gtagtggagc tggttgctgc tgctggcggt gacctggcca acccaatctg cccctgccct   2340
   cccacaggac ttcacctttg aaacctcggg ggagaatacg gctgtagtgg ccgtggagcc   2400
   tgaccgccgg aaccagtccc cagtggatca gggggccacg ggggcctcac agggcctcct    2460
   ggacaggaaa gaggtgctgg gaggtgagtt ttctttcagg ggggtagttt ggggtgaatt   2520
   gctgctgtgg ggtcagggtg gggctgacca cagccaaggc cactgctttg ggagggtctg   2580
   cacgagagcc caaggagccg ctgagctgag ctggccccgt ctacctgccc taggggtcat   2640
   tgccggaggc ctcgtggggc tcatctttgc tgtgtgcctg gtgggtttca tgctgtaccg   2700
   catgaagaag aaggacgaag gcagctactc cttggaggag ccgaaacaag ccaacggcgg   2760
   ggcctaccag aagcccacca aacaggagga attctatgcc tgacgcggga gccatgcgcc   2820
   ccctccgccc tgccactcac taggccccca cttgcctctt ccttgaagaa ctgcaggccc   2880
   tggcctcccc tgccaccagg ccacctcccc agcattccag cccctctggt cgctcctgcc   2940
   cacggagtcg tgggtgtgct gggagctcca ctctgcttct ctgacttctg cctggagact   3000
   tagggcacca ggggtttctc gcataggacc tttccaccac agccagcacc tggcatcgca   3060
   ccattctgac tcggtttctc caaactgaag cagcctctcc ccaggtccag ctctggaggg    3120
   gagggggatc cgactgcttt ggacctaaat ggcctcatgt ggctggaaga tcctgcgggt    3180
   ggggcttggg gctcacacac ctgtagcact tactggtagg accaagcatc ttgggggggt    3240
   ggccgctgag tggcagggga caggagtcac tttgtttcgt ggggaggtct aatctagata    3300
   tcgacttgtt tttgcacatg tttcctctag ttctttgttc atagcccagt agaccttgtt    3360
   acttctgagg taagttaagt aagttgattc ggtatccccc catcttgctt ccctaatcta    3420
   tggtcgggag acagcatcag ggttaagaag actttttttt ttttttttaa actaggagaa    3480
   ccaaatctgg aagccaaaat gtaggcttag tttgtgtgtt gtctcttgag tttgtcgctc    3540
   atgtgtgcaa cagggtatgg actatctgtc tggtggcccc gttctggtgg tctgttggca    3600
   ggctggccag tccaggctgc cgtggggccg ccgcctcttt caagcagtcg tgcctgtgtc    3660
   catgcgctca gggccatgct gaggcctggg ccgctgccac gttggagaag cccgtgtgag    3720
   aagtgaatgc tgggactcag ccttcagaca gagaggactg tagggagggc ggcaggggcc    3780
   tggagatcct cctgcaggct cacgcccgtc ctcctgtggc gccgtctcca ggggctgctt    3840
   cctcctggaa attgacgagg ggtgtcttgg gcagagctgg ctctgagcgc ctccatccaa    3900
   ggccaggttc tccgttagct cctgtggccc caccctgggc cctgggctgg aatcaggaat    3960
   atttttcaaa gagtgatagt cttttgcttt tggcaaaact ctacttaatc caatgggttt    4020
   ttccctgtac agtagatttt ccaaatgtaa taaactttaa tataaagtag tctgtgaatg    4080
   ccactgcctt cgcttcttgc ctctgtgctg tgtgtgacgt gactggactt ttctgcaaac    4140
   accaacatgt tgggaaactt ggctcgaatc tctgtgcctt cgtctttccc atggggaggg    4200
   attctggttc cagggtccct ctgtgtattt gcttttttgt tttggctgaa attctcctgg    4260
   aggtcggtag gttcagccaa ggttttataa ggctgatgtc aatttctgtg ttgccaagct    4320
   ccaagcccat cttctaaatg gcaaaggaag gtggatggcc ccagcacagc ttgacctgag    4380
   gctgtggtca cagcggaggt gtggagccga ggcctacccc ncagacacct tggacatcct    4440
   cctcccaccc ggctgcagag gccaganncc agcccagggt cctgcactta cttgcttatt    4500
   tgacaacgtt tcagcgactc cgttggccac tccgagagtg ggccagtctg tggatcagag    4560
   atgcaccacc aagccaaggg aacctgtgtc cggtattcga tactgcgact ttctgcctgg    4620
   agtgtatgac tgcacatgac tcgggggtgg ggaaaggggt cggctgacca tgctcatctg    4680
   ctggtccgtg ggacggtncc caagccagag gtgggttcat ttgtgtaacg acaataaacg    4740
   gtacttgtca tttcgggcaa cggctgctgt ggtggtggtt gagtctcttc ttggctt       4797
<210> 135
   <211> 2956
   <212> DNA
   <213> Homo sapien
<400> 135
<210> 136
   <211> 356
   <212> DNA
   <213> Homo sapien
<400> 136
   ggtggagcca aatgaagaaa atgaagatga aagagacaga cacctcagtt tttctggatc 60
   aggcattgat gatgatgaag attttatctc cagcaccatt tcaaccacac cacgggcttt 120
   tgaccacaca aaacagaacc aggactggac tcagtggaac ccaagccatt caaatccgga 180
   agtgctactt cagacaacca caaggatgac tgatgtagac agaaatggca ccactgctta 240
   tgaaggaaac tggaacccag aagcacaccc tcccctcatt caccatgagc atcatgagga 300
   agaagagacc ccacattcta caagcacaat ccaggcaact cctagtagta caacgg 356
<210> 137
   <211> 356
   <212> DNA
   <213> Homo sapien
<220>
   <221> misc_feature
   <222> (1)...(356)
   <223> n = A,T,C or G
<400> 137
   gcaggtggag aagacatttt attgttcctg gggtctctgg aggcccattg gtggggctgg 60
   gtcactggct gcccccggaa cagggcgctg ctccatggct ctgcttgtgg tagtctgtgg 120
   ctatgtctcc cagcaaggac agaaactcag aaaaatcaat cttcttatcc tcattcttgt 180
   cctttttctc aaagacatcg gcgaggtaat ttgtgccctt tttacctcgg cccgcgacca 240
   cgctaaggcc aaanttccag acanayggcc gggccggtnc nataggggan cccaacttgg 300
   ggacccaaac tctggcgcgg aaacacangg gcataagctt gnttcctgtg gggaaa 356
<210> 138
   <211> 353
   <212> DNA
   <213> Homo sapien
<400> 138
   aggtccagtc ctccacttgg cctgatgaga gtggggagtg gcaagggacg tttctcctgc 60
   aatagacact tagatttctc tcttgtggga agaaaccacc tgtccatcca ctgactcttc 120
   tacattgatg tggaaattgc tgctgctacc accacctcct gaagaggctt ccctgatgcc 180
   aatgccagcc atcttggcat cctggccctc gagcaggctg cggtaagtag cgatctcctg 240
   ctccagccgt gtctttatgt caagcagcat cttgtactcc tggttctgag cctccatctc 300
   gcatcggagc tcactcagac ctcgsccgsg mssmcgctam gccgaattcc agc 353
<210> 139
   <211> 371
   <212> DNA
   <213> Homo sapien
<400> 139
   agcgtggtcg cggccgaggt ccatccgaag caagattgca gatggcagtg tgaagagaga 60
   agacatattc tacacttcaa agctttggtg caattcccat cgaccagagt tggtccgacc 120
   agccttggaa aggtcactga aaaatcttca attggattat gttgacctct accttattca 180
   ttttccagtg tctgtaaagc caggtgagga agtgatccca aaagatgaaa atggaaaaat 240
   actatttgac acagtggatc tctgtgccac gtgggaggcc gtggagaagt gtaaagatgc 300
   aggattggac ctgcccgggc ggccgctcga aagccgaatt ccagcacact ggcggccgtt 360
   actagtggat c 371
<210> 140
   <211> 370
   <212> DNA
   <213> Homo sapien
<400> 140
   tagcgtggtc gcggccgagg tccatctccc tttgggaact agggggctgc tggtgggaaa 60
   tgggagccag ggcagatgtt gcattccttt gtgtccctgt aaatgtggga ctacaagaag 120
   aggagctgcc tgagtggtac tttctcttcc tggtaatcct ctggcccagc ctcatggcag 180
   aatagaggta tttttaggct atttttgtaa tatggcttct ggtcaaaatc cctgtgtagc 240
   tgaattccca agccctgcat tgtacagccc cccactcccc tcaccaccta ataaaggaat 300
   agttaacact caaaaaaaaa aaaaaacctg cccgggcggc cgctcgaaag ccgaattcca 360
   gcacactggc 370
<210> 141
   <211> 371
   <212> DNA
   <213> Homo sapien
<400> 141
   tagcgtggtc gcggccgagg tcctctgtgc tgcctgtcac agcccgatgg taccagcgca 60
   gggtgtaggc agtgcaggag ccctcatcca gtggcaggga acaggggtca tcactatccc 120
   aaggagcttc agggtcctgg tactcctcca cagaatactc ggagtattca gagtactcat 180
   catcctcagg gggtacccgc tcttcctcct ctgcatgaga gacgcggagc acaggcacag 240
   catggagctg ggagccggca gtgtctgcag cataactagg gaggggtcgt gatccagatg 300
   cgatgaactg gccctggcag gcacagtgct gactcatctc ttggcgacct gcccgggcgg 360
   ccgctcgaag c 371
<210> 142
   <211> 343
   <212> DNA
   <213> Homo sapien
<400> 142
   gcgttttgag gccaatggtg taaaaggaaa tatcttcaca taaaaactag atggaagcat 60
   tgtcagaaac ctctttgtga tgtttgcttt caactcacag agttgaacat tccttttcat 120
   agagcagttt tgaaacactc ttttgtagaa tttgcaagcg gatgattgga tcgctatgag 180
   gtcttcattg gaaacgggat acctttacat aaaaactaga cagtagcatt ctcagaaatt 240
   tctttgggat gtgggcattc aacccacaga ggagaacttc atttgataga gcagttttga 300
   aacacccttt ttgtagaatc tacaggtgga catttagagt gct 343
<210> 143
   <211> 354
   <212> DNA
   <213> Homo sapien
<400> 143
   aggtctgatg gcagaaaaac tcagactgtc tgcaacttta cagatggtgc attggttcag      60
   catcaggagt gggatgggaa ggaaagcaca ataacaagaa aattgaaaga tgggaaatta     120
   gtggtggagt gtgtcatgaa caatgtcacc tgtactcgga tctatgaaaa agtagaataa     180
   aaattccatc atcactttgg acaggagtta attaagagaa tgaccaagct cagttcaatg     240
   agcaaatctc catactgttt ctttcttttt tttttcatta ctgtgttcaa ttatctttat     300
   cataaacatt ttacatgcag ctatttcaaa gtgtgttgga ttaattagga tcat           354
<210> 144
   <211> 353
   <212> DNA
   <213> Homo sapien
<400> 144
   ggtcaaggac ctgggggacc cccaggtcca gcagccacat gattctgcag cagacaggga      60
   cctagagcac atctggatct cagccccacc cctggcaacc tgcctgccta gagaactccc     120
   aagatgacag actaagtagg attctgccat ttagaataat tctggtatcc tgggcgttgc     180
   gttaagttgc ttaactttca ttctgtctta cgatagtctt cagaggtggg aacagatgaa     240
   gaaaccatgc cccagagaag gttaagtgac ttcctcttta tggagccagt gttccaacct     300
   aggtttgcct gataccagac ctgtggcccc acctcccatg caggtctctg tgg            353
<210> 145
   <211> 371
   <212> DNA
   <213> Homo sapien
<400> 145
   caggtctgtc ataaactggt ctggagtttc tgacgactcc ttgttcacca aatgcaccat      60
   ttcctgagac ttgctggcct ctccgttgag tccacttggc tttctgtcct ccacagctcc     120
   attgccactg ttgatcacta gctttttctt ctgcccacac cttcttcgac tgttgactgc     180
   aatgcaaact gcaagaatca aagccaaggc caagagggat gccaagatga tcagccattc     240
   tggaatttgg ggtgtcctta taggaccaga ggttgtgttt gctccacctt cttgactccc     300
   atgtgagacc tcggccgcga ccacgctaag ccgaattcca gcacactggc ggcccgttac     360
   tagtggatcc g                                                          371
<210> 146
   <211> 355
   <212> DNA
   <213> Homo sapien
<400> 146
   ggtcctccgt cctcttccca gaggtgtcgg ggcttggccc cagcctccat cttcgtctct 60
   caggatggcg agtagcagcg gctccaaggc tgaattcatt gtcggaggga aatataaact 120
   ggtacggaag atcgggtctg gctccttcgg ggacatctat ttggcgatca acatcaccaa 180
   cggcgaggaa gtggcagtga agctagaatc tcagaaggcc aggcatcccc agttgctgta 240
   cgagagcaag ctctataaga ttcttcaagg tggggttggc atcccccaca tacggtggta 300
   tggtcaggaa aaagactaca atgtactagt catggatctt ctgggaccta gcctc 355
<210> 147
   <211> 355
   <212> DNA
   <213> Homo sapien
<400> 147
<2T0> 148
   <211> 369
   <212> DNA
   <213> Homo sapien
<400> 148
<210> 149
   <211> 620
   <212> DNA
   <213> Homo sapien
<220>
   <221> misc_feature
   <222> (1)...(620)
   <223> n = A,T,C or G
<400> 149 <210> 150
   <211> 371
   <212> DNA
   <213> Homo sapien
<400> 150 <210> 151
   <211> 4655
   <212> DNA
   <213> Homo sapien
<400> 151
<210> 152
   <211> 586
   <212> PRT
   <213> Homo sapien
<400> 152
   Met Leu Tyr Leu Glu Asn Asn Ala Gln Thr Gln Phe Ser Glu Pro Gln
   1               5                   10                  15
   Tyr Thr Asn Leu Gly Leu Leu Asn Ser Met Asp Gln Gln Ile Gln Asn
               20                  25                  30
   Gly Ser Ser Ser Thr Ser Pro Tyr Asn Thr Asp His Ala Gln Asn Ser
           35                  40                  45
   Val Thr Ala Pro Ser Pro Tyr Ala Gln Pro Ser Ser Thr Phe Asp Ala
       50                  55                  60
   Leu Ser Pro Ser Pro Ala Ile Pro Ser Asn Thr Asp Tyr Pro Gly Pro
   65                  70                  75                  80
   His Ser Phe Asp Val Ser Phe Gln Gln Ser Ser Thr Ala Lys Ser Ala
                   85                  90                  95
   Thr Trp Thr Tyr Ser Thr Glu Leu Lys Lys Leu Tyr Cys Gln Ile Ala
               100                 105                 110
   Lys Thr Cys Pro Ile Gln Ile Lys Val Met Thr Pro Pro Pro Gln Gly
           115                 120                 125
   Ala Val Ile Arg Ala Met Pro Val Tyr Lys Lys Ala Glu His Val Thr
       130                 135                 140
   Glu Val Val Lys Arg Cys Pro Asn His Glu Leu Ser Arg Glu Phe Asn
   145                 150                 155                 160
   Glu Gly Gln Ile Ala Pro Ser Ser His Leu Ile Arg Val Glu Gly Asn
                   165                 170                 175
   Ser His Ala Gln Tyr Val Glu Asp Pro Ile Thr Gly Arg Gln Ser Val
               180                 185                 190
   Leu Val Pro Tyr Glu Pro Pro Gln Val Gly Thr Glu Phe Thr Thr Val
           195                 200                 205
   Leu Tyr Asn Phe Met Cys Asn Ser Ser Cys Val Gly Gly Met Asn Arg
       210                 215                 220
   Arg Pro Ile Leu Ile Ile Val Thr Leu Glu Thr Arg Asp Gly Gln Val
   225                 230                 235                 240
   Leu Gly Arg Arg Cys Phe Glu Ala Arg Ile Cys Ala Cys Pro Gly Arg
                   245                 250                 255
   Asp Arg Lys Ala Asp Glu Asp Ser Ile Arg Lys Gln Gln Val Ser Asp
               260                 265                 270
   Ser Thr Lys Asn Gly Asp Gly Thr Lys Arg Pro Phe Arg Gln Asn Thr
           275                 280                 285
   His Gly Ile Gln Met Thr Ser Ile Lys Lys Arg Arg Ser Pro Asp Asp
       290                 295                 300
   Glu Leu Val Tyr Leu Pro Val Arg Gly Arg Glu Thr Tyr Glu Met Leu
   305                 310                 315                 320
   Val Lys Ile Lys Glu Ser Leu Glu Leu Met Gln Tyr Leu Leu Gln His
                   325                 330                 335
   Thr Ile Glu Thr Tyr Arg Gln Gln Gln Gln Gln Gln His Gln His Leu
               340                 345                 350
   Leu Gln Lys Gln Thr Ser Ile Gln Ser Pro Ser Ser Tyr Gly Asn Ser
           355                 360                 365
   Ser Pro Pro Leu Asn Lys Met Asn Ser Met Asn Lys Leu Pro Ser Val
       370                 375                 380
   Ser Gln Leu Ile Asn Pro Gln Gln Arg Asn Ala Leu Thr Pro Thr Thr
   385                 390                 395                 400
   Ile Pro Asp Gly Met Gly Ala Asn Ile Pro Met Met Gly Thr His Met
                   405                 410                 415
   Pro Met Ala Gly Asp Met Asn Gly Leu Ser Pro Thr Gln Ala Leu Pro
               420                 425                 430
   Pro Pro Leu Ser Met Pro Ser Thr Ser His Cys Thr Pro Pro Pro Pro
           435                 440                 445
   Tyr Pro Thr Asp Cys Ser Ile Val Ser Phe Leu Ala Arg Leu Gly Cys
       450                 455                 460
   Ser Ser Cys Leu Asp Tyr Phe Thr Thr Gln Gly Leu Thr Thr Ile Tyr
   465                 470                 475                 480
   Gln Ile Glu His Tyr Ser Met Asp Asp Leu Ala Ser Leu Lys Ile Pro
                   485                 490                 495
   Glu Gln Phe Arg His Ala Ile Trp Lys Gly Ile Leu Asp His Arg Gln
               500                 505                 510
   Leu His Glu Phe Ser Ser Pro Ser His Leu Leu Arg Thr Pro Ser Ser
           515                 520                 525
   Ala Ser Thr Val Ser Val Gly Ser Ser Glu Thr Arg Gly Glu Arg Val
       530                 535                 540
   Ile Asp Ala Val Arg Phe Thr Leu Arg Gln Thr Ile Ser Phe Pro Pro
   545                 550                 555                 560
   Arg Asp Glu Trp Asn Asp Phe Asn Phe Asp Met Asp Ala Arg Arg Asn
                   565                 570                 575
   Lys Gln Gln Arg Ile Lys Glu Glu Gly Glu
               580                 585
<210> 153
   <211> 2007
   <212> DNA
   <213> Homo sapien
<400> 153
   gaattcgtcg ctgctccagg gaaagttctg ttactccact gactctctct tttcctgatt      60
   acatggccag caagaaagta attacagtgt ttggagcaac aggagctcaa ggtggctctg     120
   tggccagggc aattttggag agcaaaaaat ttgcagtgag agcagtgacc agggatgtga    180
   cttgaccaaa tgccctggag ctccagcgcc ttggagctga ggtggtcaaa ggtgacctga     240
   atgataaagc atcggtggac agtgccttaa aaggtgtcta tggggccttc ttggtgacca     300
   acttctggga ccctctcaac caagataagg aagtgtgtcg gggaagctcg gtggcagact      360
   ccgccaagca cctgggtctg aagcacgtgg tgtacagcgg cctggagaac gtcaagcgac     420
   tgacggatgg caagctggag gtgccgcact ttgacagcaa gggcgaggtg gaggagtact     480
   tctggtccat tggcatcccc atgaccagtg tccgcgtggc ggcctacttt gaaaactttc     540
   tcgcggcgtg gcggcccgtg aaagcctctg atggagatta ctacaccttg gctgtaccga     600
   tgggagatgt accaatggat ggtatctctg ttgctgatat tggagcagcc gtctctagca     660
   tttttaattc tccagaggaa tttttaggca aggccgtggg gctcagtgca gaagcactaa     720
   caatacagca atatgctgat gttttgtcca aggctttggg gaaagaagtc cgagatgcaa     780
   agattacccc ggaagctttc gagaagctgg gattccctgc agcaaaggaa atagccaata     840
   tgtgtcgttt ctatgaaatg aagccagacc gagatgtcaa tctcacccac caactaaatc     900
   ccaaagtcaa aagcttcagc cagtttatct cagagaacca gggagccttc aagggcatgt     960
   agaaaatcag ctgttcagat aggcctctgc accacacagc ctctttcctc tctgatcctt    1020
   ttcctcttta cggcacaaca ttcatgttga cagaacatgc tggaatgcaa ttgtttgcaa    1080
   caccgaagga tttcctgcgg tcgcctcttc agtaggaagc actgcattgg tgataggaca    1140
   cggtaatttg attcacattt aacttgctag ttagtgataa gggtggtaca actgtttggt    1200
   aaaatgagaa gcctcggaac ttggagcttc tctcctacca ctaatgggag ggcagattat    1260
   actgggattt ctcctgggtg agtaatttca agccctaatg ctgaaattcc cctaggcagc    1320
   tccagttttc tcaactgcat tgcaaaattc ccagtgaact tttaagtact tttaacttaa 1380
   aaaaatgaac atctttgtag agaattttct ggggaacatg gtgttcaatg aacaagcaca 1440
   agcattggaa atgctaaaat tcagttttgc ctcaagattg gaagtttatt ttctgaccca 1500
   ttcatgaagt catctattga gccaccattc aattattcat ctattaattc cttgatcctt 1560
   catttatcca ttctgcaaac ttttcttgag caccagcacg ggtggccatt tgtggacttc 1620
   tcttcattcc tatgtgtttt cttatcaaag tgatccactc tcgaaaggct cctttccagt 1680
   ctgtggttgg gttcaagtca tgccagggcc agggggccca tctcctcgtt tagctctagg 1740
   caaaatccag gggatctgca gtggggagcg ggggcaggaa gctggaggga aggcctgtga 1800
   agggtaggga tgtggaaaga caaggtgaca gaaggaccca ataggacctt tctatatctc 1860
   tggcttagca ttttctacat catattgtaa tcgtcttatt tgctagtttt cttccttact 1920
   gtgagtgact aacagtcatc tttatcccag tgcctggtac ataataagtg atcaataaat 1980
   gttgattgac taaaaaaaaa aaaaaaa 2007
<210> 154
   <211> 2148
   <212> DNA
   <213> Homo sapien
<400> 154
   gaattcgtcg ctgctccagg gaaagttctg ttactccact gactctctct tttcctgata 60
   acatggccag caagaaagta attacagtgt ttggagcaac aggagctcaa ggtggctctg 120
   tggccagggc aattttggag agcaaaaaat ttgcagtgag agcagtgacc agggatgtga 180
   cttgaccaaa tgccctggag ctccagcgcc ttggagctga ggtggtcaaa ggtgacctga 240
   atgataaagc atcggtggac agtgccttaa aaggggaagc tggtggcaga ctccgccaag 300
   cacctgggtc tgaagcacgt ggtgtacagc ggcctggaga acgtcaagcg actgacggat 360
   ggcaagctgg aggtgccgca ctttgacagc aagggcgagg tggaggagta cttctggtcc 420
   attggcatcc ccatgaccag tgtccgcgtg gcggcctact ttgaaaactt tctcgcggcg 480
   tggcggcccg tgaaagcctc tgatggagat tactacacct tggctgtacc gatgggagat 540
   gtaccaatgg atggtatctc tgttgctgat attggagcag ccgtctctag catttttaat 600
   tctccagagg aatttttagg caaggccgtg gggctcagtg cagaagcact aacaatacag 660
   caatatgctg atgttttgtc caaggctttg gggaaagaag tccgagatgc aaagactatc 720
   tgtgctatag atgaccagaa aacagtggaa gaaggtttca tggaagacgt gggcttgagt 780
   tggtccttga gggaacatga ccatgtatag acagaggagg catcaagaag gctggcctgg 840
   ctaattctgg aataaacacg acaaaccaga ggcagtacgg gaaggaggca aattctggct 900
   ctgcctctat ccttgattac cccggaagct ttcgagaagc tgggattccc tgcagcaaag 960
   gaaatagcca atatgtgtcg tttctatgaa atgaagccag accgagatgt caatctcacc 1020
   caccaactaa atcccaaagt caaaagcttc agccatttta tctcagagaa ccagggagcc 1080
   ttcaagggca tgtagaaaat cagctgttca gataggcctc tgcaccacac agcctctttc 1140
   ctctctgatc cttttcctct ttacggcaca acattcatgt tgacagaaca tgctggaatg 1200
   caattgtttg caacaccgaa ggatttcctg cggtcgcctc ttcagtagga agcactgcat 1260
   tggtgatagg acacggtaat ttgattcaca tttaacttgc tagttagtga taagggtggt 1320
   acaactgttt ggtaaaatga gaagcctcgg aacttggagc ttctctccta ccactaatgg 1380
   gagggcagat tatactggga tttctcctgg gtgagtaatt tcaagcccta atgctgaaat 1440
   tcccctaggc agctccagtt ttctcaactg cattgcaaaa ttcccagtga acttttaagt 1500
   acttttaact taaaaaaatg aacatctttg tagagaattt tctggggaac atggtgttca 1560
   atgaacaagc acaagcattg gaaatgctaa aattcagttt tgcctcaaga ttggaagttt 1620
   attttctgac tcattcatga agtcatctat tgagccacca ttcaattatt catctattaa 1680
   ttccttgatc cttcatttat ccattctgca aacttttctt gagcaccagc acgggtggcc 1740
   atttgtggac ttctcttcat tcctatgtgt tttcttatca aagtgatcca ctctcgaaag 1800
   gctcctttcc agtctgtggt tgggttcaag tcatgccagg gccagggggc ccatctcctc 1860
   gtttagctct aggcaaaatc caggggatct gcagtgggga gcgggggcag gaagctggag 1920
   ggaaggcctg tgaagggtag ggatgtggaa agacaaggtg acagaaggac ccaataggac 1980
   ctttctatat ctctggctta gcatttctca catcatattg taatcgtctt atttgctagt 2040
   tttcttcctt actgtgagtg actaacagtc atctttatcc cagtgcctgg tacataataa 2100
   gtgatcaata aatgttgatt gactaaatga aaaaaaaaaa aaaaaaaa 2148
<210> 155
   <211> 153
   <212> PRT
   <213> Homo sapien
<400> 155
   Met Thr Ser Val Arg Val Ala Ala Tyr Phe Glu Asn Phe Leu Ala Ala
   1 5 10 15
   Trp Arg Pro Val Lys Ala Ser Asp Gly Asp Tyr Tyr Thr Leu Ala Val
   20 25 30
   Pro Met Gly Asp Val Pro Met Asp Gly Ile Ser Val Ala Asp Ile Gly
   35 40 45
   Ala Ala Val Ser Ser Ile Phe Asn Ser Pro Glu Glu Phe Leu Gly Lys
   50 55 60
   Ala Val Gly Leu Ser Ala Glu Ala Leu Thr Ile Gln Gln Tyr Ala Asp
   65 70 75 80
   Val Leu Ser Lys Ala Leu Gly Lys Glu Val Arg Asp Ala Lys Ile Thr
   85 90 95
   Pro Glu Ala Phe Glu Lys Leu Gly Phe Pro Ala Ala Lys Glu Ile Ala
   100 105 110
   Asn Met Cys Arg Phe Tyr Glu Met Lys Pro Asp Arg Asp Val Asn Leu
   115 120 125
   Thr His Gln Leu Asn Pro Lys Val Lys Ser Phe Ser Gln Phe Ile Ser
   130 135 140
   Glu Asn Gln Gly Ala Phe Lys Gly Met
   145 150
<210> 156
   <211> 128
   <212> PRT
   <213> Homo sapien
<400> 156
   Met Thr Ser Val Arg Val Ala Ala Tyr Phe Glu Asn Phe Leu Ala Ala
   1 5 10 15
   Trp Arg Pro Val Lys Ala Ser Asp Gly Asp Tyr Tyr Thr Leu Ala Val
   20 25 30
   Pro Met Gly Asp Val Pro Met Asp Gly Ile Ser Val Ala Asp Ile Gly
   35 40 45
   Ala Ala Val Ser Ser Ile Phe Asn Ser Pro Glu Glu Phe Leu Gly Lys
   50 55 60
   Ala Val Gly Leu Ser Ala Glu Ala Leu Thr Ile Gln Gln Tyr Ala Asp
   65 70 75 80
   Val Leu Ser Lys Ala Leu Gly Lys Glu Val Arg Asp Ala Lys Thr Ile
   85 90 95
   Cys Ala Ile Asp Asp Gln Lys Thr Val Glu Glu Gly Phe Met Glu Asp
   100 105 110
   Val Gly Leu Ser Trp Ser Leu Arg Glu His Asp His Val Ala Gly Ala
   115 120 125
<210> 157
   <211> 424
   <212> DNA
   <213> Homo sapien
<220>
   <221> misc_feature
   <222> (1)...(424)
   <223> n = A,T,C or G
<400> 157
<210> 158
   <211> 2099
   <212> DNA
   <213> Homo sapien
<400> 158
   ccgcggttaa aaggcgcagc aggtgggagc cggggccttc acccgaaacc cgacgagagc      60
   ccgacagccg gcggcgcccg agcccgacct gcctgcccag ccggagcgaa gggcgccgcc    120
   ccgcgcagag cccgcgccag ggccgccggc cgcagagcag ttaaaacgtg caggcaccag    180
   aaggcacttc ctgtcggtga agaagacctg tctccggtgt cacgggcatc ctgtgttttg    240
   caaacggggc tgacctccct tcctggggag caggaagggt cagggaagga aaagaagtac    300
   agaagatctg gctaaacaat ttctgtatgg cgaaagaaaa attctaactt gtacgccctc    360
   ttcatgcatc tttaattcaa tttgaatatt ccaggcgaca tccccactga ccgagcaaag    420
   attgacattc gtatcatcac tgtgcaccat tggcttctag gcactccagt ggggtaggag    480
   aaggaggtct gaaaccctcg cagagggatc ttgccctcat tctttgggtc tgaaacactg    540
   gcagtcgttg gaaacaggac tcagggataa accagcgcaa tggattgggg gacgctgcac    600
   actttcatcg ggggtgtcaa caaacactcc accagcatcg ggaaggtgtg gatcacagtc    660
   atctttattt tccgagtcat gatcctcgtg gtggctgccc aggaagtgtg gggtgacgag    720
   caagaggact tcgtctgcaa cacactgcaa ccgggatgca aaaatgtgtg ctatgaccac    780
   tttttcccgg tgtcccacat ccggctgtgg gccctccagc tgatcttcgt ctccacccca    840
   gcgctgctgg tggccatgca tgtggcctac tacaggcacg aaaccactcg caagttcagg    900
   cgaggagaga agaggaatga tttcaaagac atagaggaca ttaaaaagca gaaggttcgg    960
   atagaggggt cgctgtggtg gacgtacacc agcagcatct ttttccgaat catctttgaa   1020
   gcagccttta tgtatgtgtt ttacttcctt tacaatgggt accacctgcc ctgggtgttg   1080
   aaatgtggga ttgacccctg ccccaacctt gttgactgct ttatttctag gccaacagag   1140
   aagaccgtgt ttaccatttt tatgatttct gcgtctgtga tttgcatgct gcttaacgtg   1200
   gcagagttgt gctacctgct gctgaaagtg tgttttagga gatcaaagag agcacagacg   1260
   caaaaaaatc accccaatca tgccctaaag gagagtaagc agaatgaaat gaatgagctg   1320
   atttcagata gtggtcaaaa tgcaatcaca ggttcccaag ctaaacattt caaggtaaaa   1380
   tgtagctgcg tcataaggag acttctgtct tctccagaag gcaataccaa cctgaaagtt   1440
   ccttctgtag cctgaagagt ttgtaaatga ctttcataat aaatagacac ttgagttaac   1500
   tttttgtagg atacttgctc cattcataca caacgtaatc aaatatgtgg tccatctctg   1560
   aaaacaagag actgcttgac aaaggagcat tgcagtcact ttgacaggtt ccttttaagt   1620
   ggactctctg acaaagtggg tactttctga aaatttatat aactgttgtt gataaggaac   1680
   atttatccag gaattgatac gtttattagg aaaagatatt tttataggct tggatgtttt   1740
   tagttctgac tttgaattta tataaagtat ttttataatg actggtcttc cttacctgga   1800
   aaaacatgcg atgttagttt tagaattaca ccacaagtat ctaaatttgg aacttacaaa   1860
   gggtctatct tgtaaatatt gttttgcatt gtccgttggc aaatttgtga actgtcatga   1920
   tacgcttaag gtggaaagtg ttcattgcac aatatatttt tactgctttc tgaatgtaga   1980
   cggaacagtg tggaagcaga aggctttttt aactcatccg tttgccaatc attgcaaaca   2040
   actgaaatgt ggatgtgatt gcctcaataa agctcgtccc cattgcttaa aaaaaaaaa    2099
<210> 159
   <211> 291
   <212> PRT
   <213> Homo sapien
<400> 159
   Met Asp Trp Gly Thr Leu His Thr Phe Ile Gly Gly Val Asn Lys His
    1               5                  10                  15
   Ser Thr Ser Ile Gly Lys Val Trp Ile Thr Val Ile Phe Ile Phe Arg
               20                  25                  30
   Val Met Ile Leu Val Val Ala Ala Gln Glu Val Trp Gly Asp Glu Gln
           35                  40                  45
   Glu Asp Phe Val Cys Asn Thr Leu Gln Pro Gly Cys Lys Asn Val Cys
       50                  55                  60
   Tyr Asp His Phe Phe Pro Val Ser His Ile Arg Leu Trp Ala Leu Gln
   65                  70                  75                  80
   Leu Ile Phe Val Ser Thr Pro Ala Leu Leu Val Ala Met His Val Ala
                   85                  90                  95
   Tyr Tyr Arg His Glu Thr Thr Arg Lys Phe Arg Arg Gly Glu Lys Arg
               100                 105                 110
   Asn Asp Phe Lys Asp Ile Glu Asp Ile Lys Lys Gln Lys Val Arg Ile
           115                 120                 125
   Glu Gly Ser Leu Trp Trp Thr Tyr Thr Ser Ser Ile Phe Phe Arg Ile
       130                 135                 140
   Ile Phe Glu Ala Ala Phe Met Tyr Val Phe Tyr Phe Leu Tyr Asn Gly
   145                 150                 155                 160
   Tyr His Leu Pro Trp Val Leu Lys Cys Gly Ile Asp Pro Cys Pro Asn
                   165                 170                 175
   Leu Val Asp Cys Phe Ile Ser Arg Pro Thr Glu Lys Thr Val Phe Thr
               180                 185                 190
   Ile Phe Met Ile Ser Ala Ser Val Ile Cys Met Leu Leu Asn Val Ala
           195                 200                 205
   Glu Leu Cys Tyr Leu Leu Leu Lys Val Cys Phe Arg Arg Ser Lys Arg
       210                 215                 220
   Ala Gln Thr Gln Lys Asn His Pro Asn His Ala Leu Lys Glu Ser Lys
   225                 230                 235                 240
   Gln Asn Glu Met Asn Glu Leu Ile Ser Asp Ser Gly Gln Asn Ala Ile
                   245                 250                 255
   Thr Gly Ser Gln Ala Lys His Phe Lys Val Lys Cys Ser Cys Val Ile
               260                 265                 270
   Arg Arg Leu Leu Ser Ser Pro Glu Gly Asn Thr Asn Leu Lys Val Pro
           275                 280                 285
   Ser Val Ala
       290
<210> 160
   <211> 3951
   <212> DNA
   <213> Homo sapien
<400> 160
   tctgcatcca tattgaaaac ctgacacaat gtatgcagca ggctcagtgt gagtgaactg      60
   gaggcttctc tacaacatga cccaaaggag cattgcaggt cctatttgca acctgaagtt    120
   tgtgactctc ctggttgcct taagttcaga actcccattc ctgggagctg gagtacagct    180
   tcaagacaat gggtataatg gattgctcat tgcaattaat cctcaggtac ctgagaatca    240
   gaacctcatc tcaaacatta aggaaatgat aactgaagct tcattttacc tatttaatgc    300
   taccaagaga agagtatttt tcagaaatat aaagatttta atacctgcca catggaaagc    360
   taataataac agcaaaataa aacaagaatc atatgaaaag gcaaatgtca tagtgactga    420
   ctggtatggg gcacatggag atgatccata caccctacaa tacagagggt gtggaaaaga    480
   gggaaaatac attcatttca cacctaattt cctactgaat gataacttaa cagctggcta    540
   cggatcacga ggccgagtgt ttgtccatga atgggcccac ctccgttggg gtgtgttcga    600
   tgagtataac aatgacaaac ctttctacat aaatgggcaa aatcaaatta aagtgacaag    660
   gtgttcatct gacatcacag gcatttttgt gtgtgaaaaa ggtccttgcc cccaagaaaa    720
   ctgtattatt agtaagcttt ttaaagaagg atgcaccttt atctacaata gcacccaaaa    780
   tgcaactgca tcaataatgt tcatgcaaag tttatcttct gtggttgaat tttgtaatgc    840
   aagtacccac aaccaagaag caccaaacct acagaaccag atgtgcagcc tcagaagtgc    900
   atgggatgta atcacagact ctgctgactt tcaccacagc tttcccatga acgggactga    960
   gcttccacct cctcccacat tctcgcttgt agaggctggt gacaaagtgg tctgtttagt   1020
   gctggatgtg tccagcaaga tggcagaggc tgacagactc cttcaactac aacaagccgc   1080
   agaattttat ttgatgcaga ttgttgaaat tcataccttc gtgggcattg ccagtttcga    1140
   cagcaaagga gagatcagag cccagctaca ccaaattaac agcaatgatg atcgaaagtt   1200
   gctggtttca tatctgccca ccactgtatc agctaaaaca gacatcagca tttgttcagg   1260
   gcttaagaaa ggatttgagg tggttgaaaa actgaatgga aaagcttatg gctctgtgat   1320
   gatattagtg accagcggag atgataagct tcttggcaat tgcttaccca ctgtgctcag   1380
   cagtggttca acaattcact ccattgccct gggttcatct gcagccccaa atctggagga   1440
   attatcacgt cttacaggag gtttaaagtt ctttgttcca gatatatcaa actccaatag   1500
   catgattgat gctttcagta gaatttcctc tggaactgga gacattttcc agcaacatat   1560
   tcagcttgaa agtacaggtg aaaatgtcaa acctcaccat caattgaaaa acacagtgac   1620
   tgtggataat actgtgggca acgacactat gtttctagtt acgtggcagg ccagtggtcc   1680
   tcctgagatt atattatttg atcctgatgg acgaaaatac tacacaaata attttatcac   1740
   caatctaact tttcggacag ctagtctttg gattccagga acagctaagc ctgggcactg   1800
   gacttacacc ctgaacaata cccatcattc tctgcaagcc ctgaaagtga cagtgacctc   1860
   tcgcgcctcc aactcagctg tgcccccagc cactgtggaa gcctttgtgg aaagagacag   1920
   cctccatttt cctcatcctg tgatgattta tgccaatgtg aaacagggat tttatcccat   1980
   tcttaatgcc actgtcactg ccacagttga gccagagact ggagatcctg ttacgctgag   2040
   actccttgat gatggagcag gtgctgatgt tataaaaaat gatggaattt actcgaggta   2100
   ttttttctcc tttgctgcaa atggtagata tagcttgaaa gtgcatgtca atcactctcc   2160
   cagcataagc accccagccc actctattcc agggagtcat gctatgtatg taccaggtta   2220
   cacagcaaac ggtaatattc agatgaatgc tccaaggaaa tcagtaggca gaaatgagga   2280
   ggagcgaaag tggggcttta gccgagtcag ctcaggaggc tccttttcag tgctgggagt   2340
   tccagctggc ccccaccctg atgtgtttcc accatgcaaa attattgacc tggaagctgt   2400
   aaaagtagaa gaggaattga ccctatcttg gacagcacct ggagaagact ttgatcaggg   2460
   ccaggctaca agctatgaaa taagaatgag taaaagtcta cagaatatcc aagatgactt   2520
   taacaatgct attttagtaa atacatcaaa gcgaaatcct cagcaagctg gcatcaggga   2580
   gatatttacg ttctcacccc aaatttccac gaatggacct gaacatcagc caaatggaga   2640
   aacacatgaa agccacagaa tttatgttgc aatacgagca atggatagga actccttaca   2700
   gtctgctgta tctaacattg cccaggcgcc tctgtttatt ccccccaatt ctgatcctgt   2760
   acctgccaga gattatctta tattgaaagg agttttaaca gcaatgggtt tgataggaat   2820
   catttgcctt attatagttg tgacacatca tactttaagc aggaaaaaga gagcagacaa   2880
   gaaagagaat ggaacaaaat tattataaat aaatatccaa agtgtcttcc ttcttagata   2940
   taagacccat ggccttcgac tacaaaaaca tactaacaaa gtcaaattaa catcaaaact   3000
   gtattaaaat gcattgagtt tttgtacaat acagataaga tttttacatg gtagatcaac   3060
   aaattctttt tgggggtaga ttagaaaacc cttacacttt ggctatgaac aaataataaa   3120
   aattattctt taaagtaatg tctttaaagg caaagggaag ggtaaagtcg gaccagtgtc   3180
   aaggaaagtt tgttttattg aggtggaaaa atagccccaa gcagagaaaa ggagggtagg   3240
   tctgcattat aactgtctgt gtgaagcaat catttagtta ctttgattaa tttttctttt   3300
   ctccttatct gtgcagaaca ggttgcttgt ttacaactga agatcatgct atatttcata   3360
   tatgaagccc ctaatgcaaa gctctttacc tcttgctatt ttgttatata tattacagat     3420
   gaaatctcac tgctaatgct cagagatctt ttttcactgt aagaggtaac ctttaacaat     3480
   atgggtatta cctttgtctc ttcataccgg ttttatgaca aaggtctatt gaatttattt     3540
   gtttgtaagt ttctactccc atcaaagcag ctttttaagt tattgccttg gttattatgg     3600
   atgatagtta tagcccttat aatgccttaa ctaaggaaga aaagatgtta ttctgagttt     3660
   gttttaatac atatatgaac atatagtttt attcaattaa accaaagaag aggtcagcag     3720
   ggagatacta acctttggaa atgattagct ggctctgttt tttggttaaa taagagtctt     3780
   taatcctttc tccatcaaga gttacttacc aagggcaggg gaagggggat atagaggtcc     3840
   caaggaaata aaaatcatct ttcatcttta attttactcc ttcctcttat ttttttaaaa     3900
   gattatcgaa caataaaatc atttgccttt ttaattaaaa acataaaaaa a              3951
<210> 161
   <211> 943
   <212> PRT
   <213> Homo sapien
<400> 161
<210> 162
   <211> 493
   <212> DNA
   <213> Homo sapien
<400> 162
<210> 163
   <211> 1128
   <212> DNA
   <213> Homo sapien
<400> 163
   gccacctggc cctcctgatc gacgacacac gcacttgaaa cttgttctca gggtgtgtgg 60
   aatcaacttt ccggaagcaa ccagcccacc agaggaggtc ccgagcgcga gcggagacga 120
   tgcagcggag actggttcag cagtggagcg tcgcggtgtt cctgctgagc tacgcggtgc 180
   cctcctgcgg gcgctcggtg gagggtctca gccgccgcct caaaagagct gtgtctgaac 240
   atcagctcct ccatgacaag gggaagtcca tccaagattt acggcgacga ttcttccttc 300
   accatctgat cgcagaaatc cacacagctg aaatcagagc tacctcggag gtgtccccta 360
   actccaagcc ctctcccaac acaaagaacc accccgtccg atttgggtct gatgatgagg 420
   gcagatacct aactcaggaa actaacaagg tggagacgta caaagagcag ccgctcaaga 480
   cacctgggaa gaaaaagaaa ggcaagcccg ggaaacgcaa ggagcaggaa aagaaaaaac 540
   ggcgaactcg ctctgcctgg ttagactctg gagtgactgg gagtgggcta gaaggggacc 600
   acctgtctga cacctccaca acgtcgctgg agctcgattc acggaggcat tgaaattttc 660
   agcagagacc ttccaaggac atattgcagg attctgtaat agtgaacata tggaaagtat 720
   tagaaatatt tattgtctgt aaatactgta aatgcattgg aataaaactg tctcccccat 780
   tgctctatga aactgcacat tggtcattgt gaatattttt ttttttgcca aggctaatcc 840
   aattattatt atcacattta ccataattta ttttgtccat tgatgtattt attttgtaaa 900
   tgtatcttgg tgctgctgaa tttctatatt ttttgtaaca taatgcactt tagatataca 960
   tatcaagtat gttgataaat gacacaatga agtgtctcta ttttgtggtt gattttaatg 1020
   aatgcctaaa tataattatc caaattgatt ttcctttgtg catgtaaaaa taacagtatt 1080
   ttaaatttgt aaagaatgtc taataaaata taatctaatt acatcatg 1128
<210> 164
   <211> 1310
   <212> DNA
   <213> Homo sapien
<400> 164
   gggcctggtt cgcaaagaag ctgacttcag agggggaaac tttcttcttt taggaggcgg 60
   ttagccctgt tccacgaacc caggagaact gctggccaga ttaattagac attgctatgg 120
   gagacgtgta aacacactac ttatcattga tgcatatata aaaccatttt attttcgcta 180
   ttatttcaga ggaagcgcct ctgatttgtt tcttttttcc ctttttgctc tttctggctg 240
   tgtggtttgg agaaagcaca gttggagtag ccggttgcta aataagtccc gagcgcgagc 300
   ggagacgatg cagcggagac tggttcagca gtggagcgtc gcggtgttcc tgctgagcta 360
   cgcggtgccc tcctgcgggc gctcggtgga gggtctcagc cgccgcctca aaagagctgt 420
   gtctgaacat cagctcctcc atgacaaggg gaagtccatc caagatttac ggcgacgatt 480
   cttccttcac catctgatcg cagaaatcca cacagctgaa atcagagcta cctcggaggt 540
   gtcccctaac tccaagccct ctcccaacac aaagaaccac cccgtccgat ttgggtctga 600
   tgatgagggc agatacctaa ctcaggaaac taacaaggtg gagacgtaca aagagcagcc 660
   gctcaagaca cctgggaaga aaaagaaagg caagcccggg aaacgcaagg agcaggaaaa 720
   gaaaaaacgg cgaactcgct ctgcctggtt agactctgga gtgactggga gtgggctaga 780
   aggggaccac ctgtctgaca cctccacaac gtcgctggag ctcgattcac ggaggcattg 840
   aaattttcag cagagacctt ccaaggacat attgcaggat tctgtaatag tgaacatatg 900
   gaaagtatta gaaatattta ttgtctgtaa atactgtaaa tgcattggaa taaaactgtc 960
   tcccccattg ctctatgaaa ctgcacattg gtcattgtga atattttttt ttttgccaag 1020
   gctaatccaa ttattattat cacatttacc ataatttatt ttgtccattg atgtatttat 1080
   tttgtaaatg tatcttggtg ctgctgaatt tctatatttt ttgtaacata atgcacttta 1140
   gatatacata tcaagtatgt tgataaatga cacaatgaag tgtctctatt ttgtggttga 1200
   ttttaatgaa tgcctaaata taattatcca aattgatttt cctttgtgcc cgtaaaaata 1260
   acagtatttt aaatttgtaa agaatgtcta ataaaatata atctaattac 1310
<210> 165
   <211> 177
   <212> PRT
   <213> Homo sapien
<400> 165
<210> 166
   <211> 177
   <212> PRT
   <213> Homo sapien
<400> 165
<210> 167
   <211> 3362
   <212> DNA
   <213> Homo sapien
<400> 167
   cacaatgtat gcagcaggct cagtgtgagt gaactggagg cttctctaca acatgaccca 60
   aaggagcatt gcaggtccta tttgcaacct gaagtttgtg actctcctgg ttgccttaag 120
   ttcagaactc ccattcctgg gagctggagt acagcttcaa gacaatgggt ataatggatt 180
   gctcattgca attaatcctc aggtacctga gaatcagaac ctcatctcaa acattaagga 240
   aatgataact gaagcttcat tttacctatt taatgctacc aagagaagag tatttttcag 300
   aaatataaag attttaatac ctgccacatg gaaagctaat aataacagca aaataaaaca 360
   agaatcatat gaaaaggcaa atgtcatagt gactgactgg tatggggcac atggagatga 420
   tccatacacc ctacaataca gagggtgtgg aaaagaggga aaatacattc atttcacacc 480
   taatttccta ctgaatgata acttaacagc tggctacgga tcacgaggcc gagtgtttgt 540
   ccatgaatgg gcccacctcc gttggggtgt gttcgatgag tataacaatg acaaaccttt 600
   ctacataaat gggcaaaatc aaattaaagt gacaaggtgt tcatctgaca tcacaggcat 660
   ttttgtgtgt gaaaaaggtc cttgccccca agaaaactgt attattagta agctttttaa 720
   agaaggatgc acctttatct acaatagcac ccaaaatgca actgcatcaa taatgttcat 780
   gcaaagttta tcttctgtgg ttgaattttg taatgcaagt acccacaacc aagaagcacc 840
   aaacctacag aaccagatgt gcagcctcag aagtgcatgg gatgtaatca cagactctgc 900
   tgactttcac cacagctttc ccatgaacgg gactgagctt ccacctcctc ccacattctc 960
   gcttgtagag gctggtgaca aagtggtctg tttagtgctg gatgtgtcca gcaagatggc 1020
   agaggctgac agactccttc aactacaaca agccgcagaa ttttatttga tgcagattgt 1080
   tgaaattcat accttcgtgg gcattgccag tttcgacagc aaaggagaga tcagagccca 1140
   gctacaccaa attaacagca atgatgatcg aaagttgctg gtttcatatc tgcccaccac 1200
   tgtatcagct aaaacagaca tcagcatttg ttcagggctt aagaaaggat ttgaggtggt 1260
   tgaaaaactg aatggaaaag cttatggctc tgtgatgata ttagtgacca gcggagatga 1320
   taagcttctt ggcaattgct tacccactgt gctcagcagt ggttcaacaa ctcactccat 1380
   tgccctgggt tcatctgcag ccccaaatct ggaggaatta tcacgtctta caggaggttt 1440
   aaagttcttt gttccagata tatcaaactc caatagcatg attgatgctt tcagtagaat 1500
   ttcctctgga actggagaca ttttccagca acatattcag cttgaaagta caggtgaaaa 1560
   tgtcaaacct caccatcaat tgaaaaacac agtgactgtg gataatactg tgggcaacga 1620
   cactatgttt ctagttacgt ggcaggccag tggtcctcct gagattatat tatttgatcc 1680
   tgatggacga aaatactaca caaataattt tatcaccaat ctaacttttc ggacagctag 1740
   tctttggatt ccaggaacag ctaagcctgg gcactggact tacaccctga tgtgtttcca 1800
   ccatgcaaaa ttattgacct ggaagctgta aaagtagaag aggaattgac cctatcttgg 1860
   acagcacctg gagaagactt tgatcagggc caggctacaa gctatgaaat aagaatgagt 1920
   aaaagtctac agaatatcca agatgacttt aacaatgcta ttttagtaaa tacatcaaag 1980
   cgaaatcctc agcaagctgg catcagggag atatttacgt tctcacccca aatttccacg 2040
   aatggacctg aacatcagcc aaatggagaa acacatgaaa gccacagaat ttatgttgca 2100
   atacgagcaa tggataggaa ctccttacag tctgctgtat ctaacattgc ccaggcgcct 2160
   ctgtttattc cccccaattc tgatcctgta cctgccagag attatcttat attgaaagga 2220
   gttttaacag caatgggttt gataggaatc atttgcctta ttatagttgt gacacatcat 2280
   actttaagca ggaaaaagag agcagacaag aaagagaatg gaacaaaatt attataaata 2340
   aatatccaaa gtgtcttcct tcttagatat aagacccatg gccttcgact acaaaaacat 2400
   actaacaaag tcaaattaac atcaaaactg tattaaaatg cattgagttt ttgtacaata 2460
   cagataagat ttttacatgg tagatcaaca aattcttttt gggggtagat tagaaaaccc 2520
   ttacactttg gctatgaaca aataataaaa attattcttt aaagtaatgt ctttaaaggc 2580
   aaagggaagg gtaaagtcgg accagtgtca aggaaagttt gttttattga ggtggaaaaa 2640
   tagccccaag cagagaaaag gagggtaggt ctgcattata actgtctgtg tgaagcaatc 2700
   atttagttac tttgattaat ttttcttttc tccttatctg tgcagaacag gttgcttgtt 2760
   tacaactgaa gatcatgcta tatttcatat atgaagcccc taatgcaaag ctctttacct 2820
   cttgctattt tgttatatat attacagatg aaatctcact gctaatgctc agagatcttt 2880
   tttcactgta agaggtaacc tttaacaata tgggtattac ctttgtctct tcataccggt 2940
   tttatgacaa aggtctattg aatttatttg tttgtaagtt tctactccca tcaaagcagc 3000
   tttctaagtt attgccttgg ttattatgga tgatagttat agcccttata atgccttaac 3060
   taaggaagaa aagatgttat tctgagtttg ttttaataca tatatgaaca tatagtttta 3120
   ttcaattaaa ccaaagaaga ggtcagcagg gagatactaa cctttggaaa tgattagctg 3180
   gctctgtttt ttggttaaat aagagtcttt aatcctttct ccatcaagag ttacttacca 3240
   agggcagggg aagggggata tagaggtcac aaggaaataa aaatcatctt tcatctttaa 3300
   ttttactcct tcctcttatt tttttaaaag attatcgaac aataaaatca tttgcctttt 3360
   tt 3362
<210> 168
   <211> 2784
   <212> DNA
   <213> Homo sapien
<400> 168
   tctgcatcca tattgaaaac ctgacacaat gtatgcagca ggctcagtgt gagtgaactg 60
   gaggcttctc tacaacatga cccaaaggag cattgcaggt cctatttgca acctgaagtt 120
   tgtgactctc ctggttgcct taagttcaga actcccattc ctgggagctg gagtacagct 180
   tcaagacaat gggtataatg gattgctcat tgcaattaat cctcaggtac ctgagaatca 240
   gaacctcatc tcaaacatta aggaaatgat aactgaagct tcattttacc tatttaatgc 300
   taccaagaga agagtatttt tcagaaatat aaagatttta atacctgcca catggaaagc 360
   taataataac agcaaaataa aacaagaatc atatgaaaag gcaaatgtca tagtgactga 420
   ctggtatggg gcacatggag atgatccata caccctacaa tacagagggt gtggaaaaga 480
   gggaaaatac attcatttca cacctaattt cctactgaat gataacttaa cagctggcta 540
   cggatcacga ggccgagtgt ttgtccatga atgggcccac ctccgttggg gtgtgttcga 600
   tgagtataac aatgacaaac ctttctacat aaatgggcaa aatcaaatta aagtgacaag 660
   gtgttcatct gacatcacag gcatttttgt gtgtgaaaaa ggtccttgcc cccaagaaaa 720
   ctgtattatt agtaagcttt ttaaagaagg atgcaccttt atctacaata gcacccaaaa 780
   tgcaactgca tcaataatgt tcatgcaaag tttatcttct gtggttgaat tttgtaatgc 840
   aagtacccac aaccaagaag caccaaacct acagaaccag atgtgcagcc tcagaagtgc 900
   atgggatgta atcacagact ctgctgactt tcaccacagc tttcccatga acgggactga 960
   gcttccacct cctcccacat tctcgcttgt agaggctggt gacaaagtgg tctgtttagt 1020
   gctggatgtg tccagcaaga tggcagaggc tgacagactc cttcaactac aacaagccgc 1080
   agaattttat ttgatgcaga ttgttgaaat tcataccttc gtgggcattg ccagtttcga 1140
   cagcaaagga gagatcagag cccagctaca ccaaattaac agcaatgatg atcgaaagtt 1200
   gctggtttca tatctgccca ccactgtatc agctaaaaca gacatcagca tttgttcagg 1260
   gcttaagaaa ggatttgagg tggttgaaaa actgaatgga aaagcttatg gctctgtgat 1320
   gatattagtg accagcggag atgataagct tcttggcaat tgcttaccca ctgtgctcag 1380
   cagtggttca acaattcact ccattgccct gggttcatct gcagccccaa atctggagga 1440
   attatcacgt cttacaggag gtttaaagtt ctttgttcca gatatatcaa actccaatag 1500
   catgattgat gctttcagta gaatttcctc tggaactgga gacattttcc agcaacatat 1560
   tcagcttgaa agtacaggtg aaaatgtcaa acctcaccat caattgaaaa acacagtgac 1620
   tgtggataat actgtgggca acgacactat gtttctagtt acgtggcagg ccagtggtcc 1680
   tcctgagatt atattatttg atcctgatgg acgaaaatac tacacaaata attttatcac 1740
   caatctaact tttcggacag ctagtctttg gattccagga acagctaagc ctgggcactg 1800
   gacttacacc ctgaacaata cccatcattc tctgcaagcc ctgaaagtga cagtgacctc 1860
   tcgcgcctcc aactcagctg tgcccccagc cactgtggaa gcctttgtgg aaagagacag 1920
   cctccatttt cctcatcctg tgatgattta tgccaatgtg aaacagggat tttatcccat 1980
   tcttaatgcc actgtcactg ccacagttga gccagagact ggagatcctg ttacgctgag 2040
   actccttgat gatggagcag gtgctgatgt tataaaaaat gatggaattt actcgaggta 2100
   ttttttctcc tttgctgcaa atggtagata tagcttgaaa gtgcatgtca atcactctcc 2160
   cagcataagc accccagccc actctattcc agggagtcat gctatgtatg taccaggtta 2220
   cacagcaaac ggtaatattc agatgaatgc tccaaggaaa tcagtaggca gaaatgagga 2280
   ggagcgaaag tggggcttta gccgagtcag ctcaggaggc tccttttcag tgctgggagt 2340
   tccagctggc ccccaccctg atgtgtttcc accatgcaaa attattgacc tggaagctgt 2400
   aaatagaaga ggaattgacc ctatcttgga cagcacctgg agaagacttt gatcagggcc 2460
   aggctacaag ctatgaaata agaatgagta aaagtctaca gaatatccaa gatgacttta 2520
   acaatgctat tttagtaaat acatcaaagc gaaatcctca gcaagctggc atcagggaga 2580
   tatttacgtt ctcaccccaa atttccacga atggacctga acatcagcca aatggagaaa 2640
   cacatgaaag ccacagaatt tatgttgcaa tacgagcaat ggataggaac tccttacagt 2700
   ctgctgtatc taacattgcc caggcgcctc tgtttattcc ccccaattct gatcctgtac 2760
   ctgccagaga ttatcttata ttga 2784
<210> 169
   <211> 592
   <212> PRT
   <213> Homo sapien
<400> 169
   Met Thr Gln Arg Ser Ile Ala Gly Pro Ile Cys Asn Leu Lys Phe Val
   1               5                   10                  15
   Thr Leu Leu Val Ala Leu Ser Ser Glu Leu Pro Phe Leu Gly Ala Gly
               20                  25                  30
   Val Gln Leu Gln Asp Asn Gly Tyr Asn Gly Leu Leu Ile Ala Ile Asn
           35                  40                  45
   Pro Gln Val Pro Glu Asn Gln Asn Leu Ile Ser Asn Ile Lys Glu Met
       50                  55                  60
   Ile Thr Glu Ala Ser Phe Tyr Leu Phe Asn Ala Thr Lys Arg Arg Val
   65                  70                  75                  80
   Phe Phe Arg Asn Ile Lys Ile Leu Ile Pro Ala Thr Trp Lys Ala Asn
                   85                  90                  95
   Asn Asn Ser Lys Ile Lys Gln Glu Ser Tyr Glu Lys Ala Asn Val Ile
               100                 105                 110
   Val Thr Asp Trp Tyr Gly Ala His Gly Asp Asp Pro Tyr Thr Leu Gln
           115                 120                 125
   Tyr Arg Gly Cys Gly Lys Glu Gly Lys Tyr Ile His Phe Thr Pro Asn
       130                 135                 140
   Phe Leu Leu Asn Asp Asn Leu Thr Ala Gly Tyr Gly Ser Arg Gly Arg
   145                 150                 155                 160
   Val Phe Val His Glu Trp Ala His Leu Arg Trp Gly Val Phe Asp Glu
                   165                 170                 175
   Tyr Asn Asn Asp Lys Pro Phe Tyr Ile Asn Gly Gln Asn Gln Ile Lys
               180                 185                 190
   Val Thr Arg Cys Ser Ser Asp Ile Thr Gly Ile Phe Val Cys Glu Lys
           195                 200                 205
   Gly Pro Cys Pro Gln Glu Asn Cys Ile Ile Ser Lys Leu Phe Lys Glu
       210                 215                 220
   Gly Cys Thr Phe Ile Tyr Asn Ser Thr Gln Asn Ala Thr Ala Ser Ile
   225                 230                 235                 240
   Met Phe Met Gln Ser Leu Ser Ser Val Val Glu Phe Cys Asn Ala Ser
                   245                 250                 255
   Thr His Asn Gln Glu Ala Pro Asn Leu Gln Asn Gln Met Cys Ser Leu
               260                 265                 270
   Arg Ser Ala Trp Asp Val Ile Thr Asp Ser Ala Asp Phe His His Ser
           275                 280                 285
   Phe Pro Met Asn Gly Thr Glu Leu Pro Pro Pro Pro Thr Phe Ser Leu
       290                 295                 300
   Val Glu Ala Gly Asp Lys Val Val Cys Leu Val Leu Asp Val Ser Ser
   305                 310                 315                 320
   Lys Met Ala Glu Ala Asp Arg Leu Leu Gln Leu Gln Gln Ala Ala Glu
                   325                 330                 335
   Phe Tyr Leu Met Gln Ile Val Glu Ile His Thr Phe Val Gly Ile Ala
               340                 345                 350
   Ser Phe Asp Ser Lys Gly Glu Ile Arg Ala Gln Leu His Gln Ile Asn
           355                 360                 365
   Ser Asn Asp Asp Arg Lys Leu Leu Val Ser Tyr Leu Pro Thr Thr Val
           370                     375                     380
   Ser Ala Lys Thr Asp Ile Ser Ile Cys Ser Gly Leu Lys Lys Gly Phe
   385                 390                 395                 400
   Glu Val Val Glu Lys Leu Asn Gly Lys Ala Tyr Gly Ser Val Met Ile
                   405                 410                 415
   Leu Val Thr Ser Gly Asp Asp Lys Leu Leu Gly Asn Cys Leu Pro Thr
               420                 425                 430
   Val Leu Ser Ser Gly Ser Thr Ile His Ser Ile Ala Leu Gly Ser Ser
           435                 440                 445
   Ala Ala Pro Asn Leu Glu Glu Leu Ser Arg Leu Thr Gly Gly Leu Lys
       450                 455                 460
   Phe Phe Val Pro Asp Ile Ser Asn Ser Asn Ser Met Ile Asp Ala Phe
   465                 470                 475                 480
   Ser Arg Ile Ser Ser Gly Thr Gly Asp Ile Phe Gln Gln His Ile Gln
                   485                 490                 495
   Leu Glu Ser Thr Gly Glu Asn Val Lys Pro His His Gln Leu Lys Asn
               500                 505                 510
   Thr Val Thr Val Asp Asn Thr Val Gly Asn Asp Thr Met Phe Leu Val
           515                 520                 525
   Thr Trp Gln Ala Ser Gly Pro Pro Glu Ile Ile Leu Phe Asp Pro Asp
       530                 535                 540
   Gly Arg Lys Tyr Tyr Thr Asn Asn Phe Ile Thr Asn Leu Thr Phe Arg
   545                 550                 555                 560
   Thr Ala Ser Leu Trp Ile Pro Gly Thr Ala Lys Pro Gly His Trp Thr
                   565                 570                 575
   Tyr Thr Leu Met Cys Phe His His Ala Lys Leu Leu Thr Trp Lys Leu
               580                 585                 590
<210> 170
   <211> 791
   <212> PRT
   <213> Homo sapien
<400> 170
   Met Thr Gln Arg Ser Ile Ala Gly Pro Ile Cys Asn Leu Lys Phe Val
    1               5                  10                  15
   Thr Leu Leu Val Ala Leu Ser Ser Glu Leu Pro Phe Leu Gly Ala Gly
               20                  25                  30
   Val Gln Leu Gln Asp Asn Gly Tyr Asn Gly Leu Leu Ile Ala Ile Asn
           35                  40                  45
   Pro Gln Val Pro Glu Asn Gln Asn Leu Ile Ser Asn Ile Lys Glu Met
       50                  55                  60
   Ile Thr Glu Ala Ser Phe Tyr Leu Phe Asn Ala Thr Lys Arg Arg Val
   65                  70                  75                  80
   Phe Phe Arg Asn Ile Lys Ile Leu Ile Pro Ala Thr Trp Lys Ala Asn
                   85                  90                  95
   Asn Asn Ser Lys Ile Lys Gln Glu Ser Tyr Glu Lys Ala Asn Val Ile
               100                 105                 110
   Val Thr Asp Trp Tyr Gly Ala His Gly Asp Asp Pro Tyr Thr Leu Gln
           115                 120                 125
   Tyr Arg Gly Cys Gly Lys Glu Gly Lys Tyr Ile His Phe Thr Pro Asn
       130                 135                 140
   Phe Leu Leu Asn Asp Asn Leu Thr Ala Gly Tyr Gly Ser Arg Gly Arg
   145                 150                 155                 160
   Val Phe Val His Glu Trp Ala His Leu Arg Trp Gly Val Phe Asp Glu
                   165                 170                 175
   Tyr Asn Asn Asp Lys Pro Phe Tyr Ile Asn Gly Gln Asn Gln Ile Lys
               180                 185                 190
   Val Thr Arg Cys Ser Ser Asp Ile Thr Gly Ile Phe Val Cys Glu Lys
           195                 200                 205
   Gly Pro Cys Pro Gln Glu Asn Cys Ile Ile Ser Lys Leu Phe Lys Glu
       210                 215                 220
   Gly Cys Thr Phe Ile Tyr Asn Ser Thr Gln Asn Ala Thr Ala Ser Ile
   225                 230                 235                 240
   Met Phe Met Gln Ser Leu Ser Ser Val Val Glu Phe Cys Asn Ala Ser
                   245                 250                 255
   Thr His Asn Gln Glu Ala Pro Asn Leu Gln Asn Gln Met Cys Ser Leu
               260                 265                 270
   Arg Ser Ala Trp Asp Val Ile Thr Asp Ser Ala Asp Phe His His Ser
           275                 280                 285
   Phe Pro Met Asn Gly Thr Glu Leu Pro Pro Pro Pro Thr Phe Ser Leu
       290                 295                 300
   Val Glu Ala Gly Asp Lys Val Val Cys Leu Val Leu Asp Val Ser Ser
   305                 310                 315                 320
   Lys Met Ala Glu Ala Asp Arg Leu Leu Gln Leu Gln Gln Ala Ala Glu
                   325                 330                 335
   Phe Tyr Leu Met Gln Ile Val Glu Ile His Thr Phe Val Gly Ile Ala
               340                 345                 350
   Ser Phe Asp Ser Lys Gly Glu Ile Arg Ala Gln Leu His Gln Ile Asn
           355                 360                 365
   Ser Asn Asp Asp Arg Lys Leu Leu Val Ser Tyr Leu Pro Thr Thr Val
       370                 375                 380
   Ser Ala Lys Thr Asp Ile Ser Ile Cys Ser Gly Leu Lys Lys Gly Phe
   385                 390                 395                 400
   Glu Val Val Glu Lys Leu Asn Gly Lys Ala Tyr Gly Ser Val Met Ile
                   405                 410                 415
   Leu Val Thr Ser Gly Asp Asp Lys Leu Leu Gly Asn Cys Leu Pro Thr
               420                 425                 430
   Val Leu Ser Ser Gly Ser Thr Ile His Ser Ile Ala Leu Gly Ser Ser
           435                 440                 445
   Ala Ala Pro Asn Leu Glu Glu Leu Ser Arg Leu Thr Gly Gly Leu Lys
       450                 455                 460
   Phe Phe Val Pro Asp Ile Ser Asn Ser Asn Ser Met Ile Asp Ala Phe
   465                 470                 475                 480
   Ser Arg Ile Ser Ser Gly Thr Gly Asp Ile Phe Gln Gln His Ile Gln
                   485                 490                 495
   Leu Glu Ser Thr Gly Glu Asn Val Lys Pro His His Gln Leu Lys Asn
               500                 505                 510
   Thr Val Thr Val Asp Asn Thr Val Gly Asn Asp Thr Met Phe Leu Val
           515                 520                 525
   Thr Trp Gln Ala Ser Gly Pro Pro Glu Ile Ile Leu Phe Asp Pro Asp
       530                 535                 540
   Gly Arg Lys Tyr Tyr Thr Asn Asn Phe Ile Thr Asn Leu Thr Phe Arg
   545                 550                 555                 560
   Thr Ala Ser Leu Trp Ile Pro Gly Thr Ala Lys Pro Gly His Trp Thr
                   565                 570                 575
   Tyr Thr Leu Asn Asn Thr His His Ser Leu Gln Ala Leu Lys Val Thr
               580                 585                 590
   Val Thr Ser Arg Ala Ser Asn Ser Ala Val Pro Pro Ala Thr Val Glu
           595                 600                 605
   Ala Phe Val Glu Arg Asp Ser Leu His Phe Pro His Pro Val Met Ile
       610                 615                 620
   Tyr Ala Asn Val Lys Gln Gly Phe Tyr Pro Ile Leu Asn Ala Thr Val
   625                 630                 635                 640
   Thr Ala Thr Val Glu Pro Glu Thr Gly Asp Pro Val Thr Leu Arg Leu
                   645                 650                 655
   Leu Asp Asp Gly Ala Gly Ala Asp Val Ile Lys Asn Asp Gly Ile Tyr
               660                 665                 670
   Ser Arg Tyr Phe Phe Ser Phe Ala Ala Asn Gly Arg Tyr Ser Leu Lys
           675                 680                 685
   Val His Val Asn His Ser Pro Ser Ile Ser Thr Pro Ala His Ser Ile
       690                 695                 700
   Pro Gly Ser His Ala Met Tyr Val Pro Gly Tyr Thr Ala Asn Gly Asn
   705                 710                 715                 720
   Ile Gln Met Asn Ala Pro Arg Lys Ser Val Gly Arg Asn Glu Glu Glu
                   725                 730                 735
   Arg Lys Trp Gly Phe Ser Arg Val Ser Ser Gly Gly Ser Phe Ser Val
               740                 745                 750
   Leu Gly Val Pro Ala Gly Pro His Pro Asp Val Phe Pro Pro Cys Lys
           755                 760                 765
   Ile Ile Asp Leu Glu Ala Val Asn Arg Arg Gly Ile Asp Pro Ile Leu
       770                 775                 780
   Asp Ser Thr Trp Arg Arg Leu
   785                 790
<210> 171
   <211> 1491
   <212> DNA
   <213> Homo sapien
<400> 171
   cctcctgcca gccaagtgaa gacatgctta cttccccttc accttccttc atgatgtggg     60
   aagagtgctg caacccagcc ctagccaacg ccgcatgaga gggagtgtgc cgagggcttc    120
   tgagaaggtt tctctcacat ctagaaagaa gcgcttaaga tgtggcagcc cctcttcttc    180
   aagtggctct tgtcctgttg ccctgggagt tctcaaattg ctgcagcagc ctccacccag    240
   cctgaggatg acatcaatac acagaggaag aagagtcagg aaaagatgag agaagttaca    300
   gactctcctg ggcgaccccg agagcttacc attcctcaga cttcttcaca tggtgctaac    360
   agatttgttc ctaaaagtaa agctctagag gccgtcaaat tggcaataga agccgggttc    420
   caccatattg attctgcaca tgtttacaat aatgaggagc aggttggact ggccatccga    480
   agcaagattg cagatggcag tgtgaagaga gaagacatat tctacacttc aaagctttgg    540
   agcaattccc atcgaccaga gttggtccga ccagccttgg aaaggtcact gaaaaatctt    600
   caattggact atgttgacct ctatcttatt cattttccag tgtctgtaaa gccaggtgag    660
   gaagtgatcc caaaagatga aaatggaaaa atactatttg acacagtgga tctctgtgcc    720
   acatgggagg ccatggagaa gtgtaaagat gcaggattgg ccaagtccat cggggtgtcc    780
   aacttcaacc acaggctgct ggagatgatc ctcaacaagc cagggctcaa gtacaagcct    840
   gtctgcaacc aggtggaatg tcatccttac ttcaaccaga gaaaactgct ggatttctgc    900
   aagtcaaaag acattgttct ggttgcctat agtgctctgg gatcccatcg agaagaacca    960
   tgggtggacc cgaactcccc ggtgctcttg gaggacccag tcctttgtgc cttggcaaaa   1020
   aagcacaagc gaaccccagc cctgattgcc ctgcgctacc agctgcagcg tggggttgtg   1080
   gtcctggcca agagctacaa tgagcagcgc atcagacaga acgtgcaggt gtttgaattc   1140
   cagttgactt cagaggagat gaaagccata gatggcctaa acagaaatgt gcgatatttg   1200
   acccttgata tttttgctgg cccccctaat tatccatttt ctgatgaata ttaacatgga   1260
   gggcattgca tgaggtctgc cagaaggccc tgcgtgtgga tggtgacaca gaggatggct   1320
   ctatgctggt gactggacac atcgcctctg gttaaatctc tcctgcttgg cgacttcagt   1380
   aagctacagc taagcccatc ggccggaaaa gaaagacaat aattttgttt ttcatttcga   1440
   aaaaattaaa tgctctctcc taaagattct tcacctaaaa aaaaaaaaaa a 1491
<210> 172
   <211> 364
   <212> PRT
   <213> Homo sapien
<400> 172
   Met Trp Gln Pro Leu Phe Phe Lys Trp Leu Leu Ser Cys Cys Pro Gly
    1               5                  10                  15
   Ser Ser Gln Ile Ala Ala Ala Ala Ser Thr Gln Pro Glu Asp Asp Ile
               20                  25                  30
   Asn Thr Gln Arg Lys Lys Ser Gln Glu Lys Met Arg Glu Val Thr Asp
           35                  40                  45
   Ser Pro Gly Arg Pro Arg Glu Leu Thr Ile Pro Gln Thr Ser Ser His
       50                  55                  60
   Gly Ala Asn Arg Phe Val Pro Lys Ser Lys Ala Leu Glu Ala Val Lys
   65                  70                  75                  80
   Leu Ala Ile Glu Ala Gly Phe His His Ile Asp Ser Ala His Val Tyr
                   85                  90                  95
   Asn Asn Glu Glu Gln Val Gly Leu Ala Ile Arg Ser Lys Ile Ala Asp
               100                 105                 110
   Gly Ser Val Lys Arg Glu Asp Ile Phe Tyr Thr Ser Lys Leu Trp Ser
           115                 120                 125
   Asn Ser His Arg Pro Glu Leu Val Arg Pro Ala Leu Glu Arg Ser Leu
       130                 135                 140
   Lys Asn Leu Gln Leu Asp Tyr Val Asp Leu Tyr Leu Ile His Phe Pro
   145                 150                 155                 160
   Val Ser Val Lys Pro Gly Glu Glu Val Ile Pro Lys Asp Glu Asn Gly
                   165                 170                 175
   Lys Ile Leu Phe Asp Thr Val Asn Leu Cys Ala Thr Trp Glu Ala Met
               180                 185                 190
   Glu Lys Cys Lys Asp Ala Gly Leu Ala Lys Ser Ile Gly Val Ser Asn
           195                 200                 205
   Phe Asn His Arg Leu Leu Glu Met Ile Leu Asn Lys Pro Gly Leu Lys
       210                 215                 220
   Tyr Lys Pro Val Cys Asn Gln Val Glu Cys His Pro Tyr Phe Asn Gln
   225                 230                 235                 240
   Arg Lys Leu Leu Asp Phe Cys Lys Ser Lys Asp Ile Val Leu Val Ala
                   245                 250                 255
   Tyr Ser Ala Leu Gly Ser His Arg Glu Glu Pro Trp Val Asp Pro Asn
               260                 265                 270
   Ser Pro Val Leu Leu Glu Asp Pro Val Leu Cys Ala Leu Ala Lys Lys
           275                 280                 285
   His Lys Arg Thr Pro Ala Leu Ile Ala Leu Arg Tyr Gln Leu Gln Arg
       290                 295                 300
   Gly Val Val Val Leu Ala Lys Ser Tyr Asn Glu Gln Arg Ile Arg Gln
   305                 310                 315                 320
   Asn Val Gln Val Phe Glu Phe Gln Leu Thr Ser Glu Glu Met Lys Ala
                   325                 330                 335
   Ile Asp Gly Leu Asn Arg Asn Val Arg Tyr Leu Thr Leu Asp Ile Phe
               340                 345                 350
   Ala Gly Pro Pro Asn Tyr Pro Phe Ser Asp Glu Tyr
           355                 360